(19) Europäisches Patentamt
European Patent Office
Office européen des brevets

(11) EP 3 805 400 A1

(12) **EUROPEAN PATENT APPLICATION**
published in accordance with Art. 153(4) EPC

(43) Date of publication:
**14.04.2021 Bulletin 2021/15**

(21) Application number: **19814965.0**

(22) Date of filing: **03.06.2019**

(51) Int Cl.:
***C12P 21/08*** *(2006.01)* ***C07K 16/00*** *(2006.01)*
***C07K 16/28*** *(2006.01)* ***C12N 15/09*** *(2006.01)*
***C12N 15/10*** *(2006.01)*

(86) International application number:
**PCT/JP2019/021984**

(87) International publication number:
**WO 2019/235426 (12.12.2019 Gazette 2019/50)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **04.06.2018 JP 2018107051**

(71) Applicant: **CHUGAI SEIYAKU KABUSHIKI KAISHA**
**Tokyo 115-8543 (JP)**

(72) Inventors:
- **TERANISHI, Yuri**
  **Synapse, 138623 (SG)**
- **SAVORY, Nasa**
  **Gotemba-shi, Shizuoka 412-8513 (JP)**
- **KATO, Kazuki**
  **Gotemba-shi, Shizuoka 412-8513 (JP)**
- **SUZUKI, Takashi**
  **Gotemba-shi, Shizuoka 412-8513 (JP)**
- **HIRONIWA, Naoka**
  **Synapse, 138623 (SG)**

(74) Representative: **Vossius & Partner**
**Patentanwälte Rechtsanwälte mbB**
**Siebertstrasse 3**
**81675 München (DE)**

# (54) ANTIGEN-BINDING MOLECULE SHOWING CHANGED HALF-LIFE IN CYTOPLASM

(57) In a non-limiting embodiment, the present invention relates to antigen-binding molecules containing an altered TRIM21-binding domain and having an altered cytosolic half-life; pharmaceutical compositions containing such an antigen-binding molecule; methods for using such an antigen-binding molecule; methods for increasing or decreasing the cytosolic half-life of an antigen-binding molecule containing a TRIM21-binding domain; and methods for producing an antigen-binding molecule containing an altered TRIM21-binding domain and having an increased or decreased cytosolic half-life. The present invention also relates to substitutions at specific positions in a TRIM21-binding domain that increase or decrease the cytosolic half-life of an antigen-binding molecule containing a TRIM21-binding domain.

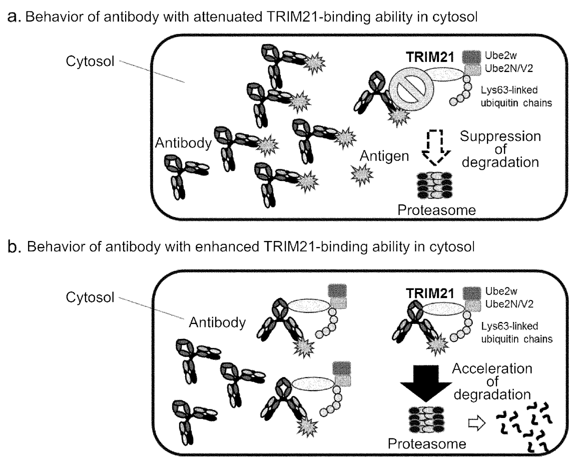


FIG. 1

EP 3 805 400 A1

SEQUENCE LISTING

<110> CHUGAI SEIYAKU KABUSHIKI KAISHA

<120> ANTIGEN-BINDING MOLECULE HAVING ALTERED HALF-LIFE IN CYTOPLASM

<130> C1-A1810P

<150> JP 2018-107051
<151> 2018-06-04

<160> 17

<170> PatentIn version 3.5

<210> 1
<211> 475
<212> PRT
<213> Homo sapiens

<400> 1

```
Met Ala Ser Ala Ala Arg Leu Thr Met Met Trp Glu Glu Val Thr Cys
1               5                   10                  15

Pro Ile Cys Leu Asp Pro Phe Val Glu Pro Val Ser Ile Glu Cys Gly
            20                  25                  30

His Ser Phe Cys Gln Glu Cys Ile Ser Gln Val Gly Lys Gly Gly Gly
        35                  40                  45

Ser Val Cys Pro Val Cys Arg Gln Arg Phe Leu Leu Lys Asn Leu Arg
    50                  55                  60

Pro Asn Arg Gln Leu Ala Asn Met Val Asn Asn Leu Lys Glu Ile Ser
65                  70                  75                  80

Gln Glu Ala Arg Glu Gly Thr Gln Gly Glu Arg Cys Ala Val His Gly
                85                  90                  95

Glu Arg Leu His Leu Phe Cys Glu Lys Asp Gly Lys Ala Leu Cys Trp
            100                 105                 110

Val Cys Ala Gln Ser Arg Lys His Arg Asp His Ala Met Val Pro Leu
        115                 120                 125

Glu Glu Ala Ala Gln Glu Tyr Gln Glu Lys Leu Gln Val Ala Leu Gly
    130                 135                 140

Glu Leu Arg Arg Lys Gln Glu Leu Ala Glu Lys Leu Glu Val Glu Ile
145                 150                 155                 160

Ala Ile Lys Arg Ala Asp Trp Lys Lys Thr Val Glu Thr Gln Lys Ser
```

165 170 175

Arg Ile His Ala Glu Phe Val Gln Gln Lys Asn Phe Leu Val Glu Glu
180 185 190

Glu Gln Arg Gln Leu Gln Glu Leu Glu Lys Asp Glu Arg Glu Gln Leu
195 200 205

Arg Ile Leu Gly Glu Lys Glu Ala Lys Leu Ala Gln Gln Ser Gln Ala
210 215 220

Leu Gln Glu Leu Ile Ser Glu Leu Asp Arg Arg Cys His Ser Ser Ala
225 230 235 240

Leu Glu Leu Leu Gln Glu Val Ile Ile Val Leu Glu Arg Ser Glu Ser
245 250 255

Trp Asn Leu Lys Asp Leu Asp Ile Thr Ser Pro Glu Leu Arg Ser Val
260 265 270

Cys His Val Pro Gly Leu Lys Lys Met Leu Arg Thr Cys Ala Val His
275 280 285

Ile Thr Leu Asp Pro Asp Thr Ala Asn Pro Trp Leu Ile Leu Ser Glu
290 295 300

Asp Arg Arg Gln Val Arg Leu Gly Asp Thr Gln Gln Ser Ile Pro Gly
305 310 315 320

Asn Glu Glu Arg Phe Asp Ser Tyr Pro Met Val Leu Gly Ala Gln His
325 330 335

Phe His Ser Gly Lys His Tyr Trp Glu Val Asp Val Thr Gly Lys Glu
340 345 350

Ala Trp Asp Leu Gly Val Cys Arg Asp Ser Val Arg Arg Lys Gly His
355 360 365

Phe Leu Leu Ser Ser Lys Ser Gly Phe Trp Thr Ile Trp Leu Trp Asn
370 375 380

Lys Gln Lys Tyr Glu Ala Gly Thr Tyr Pro Gln Thr Pro Leu His Leu
385 390 395 400

Gln Val Pro Pro Cys Gln Val Gly Ile Phe Leu Asp Tyr Glu Ala Gly
405 410 415

Met Val Ser Phe Tyr Asn Ile Thr Asp His Gly Ser Leu Ile Tyr Ser
420 425 430

Phe Ser Glu Cys Ala Phe Thr Gly Pro Leu Arg Pro Phe Phe Ser Pro
435 440 445

Gly Phe Asn Asp Gly Gly Lys Asn Thr Ala Pro Leu Thr Leu Cys Pro
450 455 460

Leu Asn Ile Gly Ser Gln Gly Ser Thr Asp Tyr
465 470 475

<210> 2
<211> 1946
<212> DNA
<213> Homo sapiens

<400> 2
gcttctgagc ggaaactgaa agtgaaatag ggagctggct accagcgttg agtcccctgt 60

aaagccaaac cccctaaagg tctccacact gctgtttaac ggcacacttg acaatggctt 120

cagcagcacg cttgacaatg atgtgggagg aggtcacatg ccctatctgc ctggacccct 180

tcgtggagcc tgtgagcatc gagtgtggcc acagcttctg ccaggaatgc atctctcagg 240

ttgggaaagg tgggggcagc gtctgtcctg tgtgccggca gcgctttctg ctcaagaatc 300

tccggcccaa tcgacagcta gccaacatgg tgaacaacct taaagaaatc agccaggagg 360

ccagagaggg cacacagggg gaacggtgtg cagtgcatgg agagagactt cacctgttct 420

gtgagaaaga tgggaaggcc ctttgctggg tatgtgccca gtctcggaaa caccgtgacc 480

acgccatggt ccctcttgag gaggctgcac aggagtacca ggagaagctc caggtggcat 540

taggggaact gagaagaaag caggagttgg ctgagaagtt ggaagtggaa attgcaataa 600

agagagcaga ctggaagaaa acagtggaaa cacagaaatc taggattcac gcagagtttg 660

tgcagcaaaa aaacttcctg gttgaagaag aacagaggca gctgcaggag ctggagaagg 720

atgagaggga gcagctgaga atcctggggg agaaagaggc caagctggcc cagcagagcc 780

aggccctaca ggagctcatc tcagagctag atcgaaggtg ccacagctca gcactggaac 840

tgctgcagga ggtgataatt gtcctggaaa ggagtgagtc ctggaacctg aaggacctgg 900

atattacctc tccagaactc aggagtgtgt gccatgtgcc agggctgaag aagatgctga 960

ggacatgtgc agtccacatc actctggatc cagacacagc caatccgtgg ctgatacttt 1020

cagaagatcg gagacaagtg aggcttggag acacccagca gagcatacct ggaaatgaag 1080

agagatttga tagttatcct atggtcctgg gtgcccagca ctttcactct ggaaaacatt 1140

actgggaggt agatgtgaca ggaaaggagg cctgggacct gggtgtctgc agagactctg 1200

tgcgcaggaa ggggcacttt ttgcttagtt ccaagagtgg cttctggaca atttggttgt 1260

ggaacaaaca aaaatatgag gctggcacct acccccagac tcccctccac cttcaggtgc 1320

ctccatgcca agttgggatt ttcctggact atgaggctgg catggtctcc ttctacaaca 1380

tcactgacca tggctccctc atctactcct tctctgaatg tgcctttaca ggacctctgc 1440

ggcccttctt cagtcctggt ttcaatgatg gaggaaaaaa cacagcccct ctaaccctct 1500

gtccactgaa tattggatca caaggatcca ctgactattg atggctttct ctggacactg 1560

ccactctccc cattggcacc gcttctcagc cacaaaccct gcctcttttc cccatgaact 1620

ctgaaccacc tttgtctctg cagaggcatc cggatcccag caagcgagct ttagcaggga 1680

agtcacttca ccatcaacat tcctgcccca gatggctttg tgattccctc cagtgaagca 1740

gcctccttat atttggccca aactcatctt gatcaaccaa aaacatgttt ctgccttctt 1800

tatgggactt aagttttttt tttctcctct ccatctctag gatgtcgtct ttggtgagat 1860

ctctattata tcttgtatgg tttgcaaaag ggcttcctaa aaataaaaaa taaaatttaa 1920

aaaactgtga aaaaaaaaaa aaaaaa 1946

<210> 3
<211> 470
<212> PRT
<213> Mus musculus

<400> 3

Met Ser Pro Ser Thr Thr Ser Lys Met Ser Leu Glu Lys Met Trp Glu
1 5 10 15

Glu Val Thr Cys Ser Ile Cys Leu Asp Pro Met Val Glu Pro Met Ser
20 25 30

Ile Glu Cys Gly His Cys Phe Cys Lys Glu Cys Ile Phe Glu Val Gly
35 40 45

Lys Asn Gly Gly Ser Ser Cys Pro Glu Cys Arg Gln Gln Phe Leu Leu
50 55 60

Arg Asn Leu Arg Pro Asn Arg His Ile Ala Asn Met Val Glu Asn Leu
65 70 75 80

Lys Gln Ile Ala Gln Asn Thr Lys Lys Ser Thr Gln Glu Thr His Cys
85 90 95

Met Lys His Gly Glu Lys Leu His Leu Phe Cys Glu Glu Asp Gly Gln
100 105 110

Ala Leu Cys Trp Val Cys Ala Gln Ser Gly Lys His Arg Asp His Thr
115 120 125

Arg Val Pro Ile Glu Glu Ala Ala Lys Val Tyr Gln Glu Lys Ile His
    130                 135                 140

Val Ala Leu Glu Lys Leu Arg Lys Gly Lys Glu Leu Ala Glu Lys Met
145                 150                 155                 160

Glu Met Asp Leu Thr Met Gln Arg Thr Asp Trp Lys Arg Asn Ile Asp
                165                 170                 175

Thr Gln Lys Ser Arg Ile His Ala Glu Phe Ala Leu Gln Asn Ser Leu
            180                 185                 190

Leu Ala Gln Glu Glu Gln Arg Gln Leu Gln Arg Leu Glu Lys Asp Gln
        195                 200                 205

Arg Glu Tyr Leu Arg Leu Leu Gly Lys Lys Glu Ala Glu Leu Ala Glu
    210                 215                 220

Lys Asn Gln Ala Leu Gln Glu Leu Ile Ser Glu Leu Glu Arg Arg Ile
225                 230                 235                 240

Arg Gly Ser Glu Leu Glu Leu Leu Gln Glu Val Arg Ile Ile Leu Glu
                245                 250                 255

Arg Ser Gly Ser Trp Asn Leu Asp Thr Leu Asp Ile Asp Ala Pro Asp
            260                 265                 270

Leu Thr Ser Thr Cys Pro Val Pro Gly Arg Lys Lys Met Leu Arg Thr
        275                 280                 285

Cys Trp Val His Ile Thr Leu Asp Arg Asn Thr Ala Asn Ser Trp Leu
    290                 295                 300

Ile Ile Ser Lys Asp Arg Arg Gln Val Arg Met Gly Asp Thr His Gln
305                 310                 315                 320

Asn Val Ser Asp Asn Lys Glu Arg Phe Ser Asn Tyr Pro Met Val Leu
                325                 330                 335

Gly Ala Gln Arg Phe Ser Ser Gly Lys Met Tyr Trp Glu Val Asp Val
            340                 345                 350

Thr Gln Lys Glu Ala Trp Asp Leu Gly Val Cys Arg Asp Ser Val Gln
        355                 360                 365

Arg Lys Gly Gln Phe Ser Leu Ser Pro Glu Asn Gly Phe Trp Thr Ile

370 375 380

Trp Leu Trp Gln Asp Ser Tyr Glu Ala Gly Thr Ser Pro Gln Thr Thr
385 390 395 400

Leu His Ile Gln Val Pro Pro Cys Gln Ile Gly Ile Phe Val Asp Tyr
405 410 415

Glu Ala Gly Val Val Ser Phe Tyr Asn Ile Thr Asp His Gly Ser Leu
420 425 430

Ile Tyr Thr Phe Ser Glu Cys Val Phe Ala Gly Pro Leu Arg Pro Phe
435 440 445

Phe Asn Val Gly Phe Asn Tyr Ser Gly Gly Asn Ala Ala Pro Leu Lys
450 455 460

Leu Cys Pro Leu Lys Met
465 470

<210> 4
<211> 1413
<212> DNA
<213> Mus musculus

<400> 4
atgtcaccct ctacaacctc aaaaatgtct ctggaaaaga tgtgggagga ggtcacctgt 60

tctatctgcc tggatcccat ggtggagcct atgagtatcg aatgtggcca ttgcttttgc 120

aaggaatgca tttttgaagt tgggaagaat gggggcagtt catgtcccga gtgccggcaa 180

cagtttctgc tccgaaacct caggcccaat agacatatag ccaacatggt ggaaaacctt 240

aaacagatag cccagaatac caagaagagt acccaggaaa cgcactgcat gaagcatgga 300

gagaagcttc acctattctg tgaggaagat gggcaggccc tttgctgggt gtgtgcccag 360

tctgggaaac accgggacca caccagggtc cctattgaag aggctgctaa ggtataccag 420

gagaagatcc acgtggcttt agaaaaactg agaaagggga aagagttggc cgagaagatg 480

gaaatggatc tcacgatgca aagaacagac tggaagagga acattgacac ccagaagtcg 540

aggattcacg cagagttcgc acttcagaat agcttgctgg ctcaggagga gcagaggcag 600

ctgcagaggc tggagaagga tcaaagggag tacctgagac tcctggggaa gaaggaggct 660

gagctggctg agaagaacca ggccctgcag gagctgatct cagagctgga gaggaggatt 720

cgtggttcag agctggagct actgcaggag gtgaggatca tcctggaaag gagtggatcc 780

tggaacctgg acacgttaga tattgacgcc ccagacctaa caagcacatg ccctgtgcca 840

gggcggaaga agatgctgag gacgtgttgg gttcatatta ctctggatcg caacaccgcc 900

```
aactcatggc tcatcatctc aaaggatcgg agacaagtga ggatgggaga cacccatcag      960

aacgtgtctg acaataagga gaggtttagt aattacccca tggtgctagg tgcccagaga     1020

ttctcctctg ggaagatgta ctgggaggta gatgtgactc aaaaggaggc ctgggatctg     1080

ggggtttgca gagattctgt tcagaggaaa gggcagtttt cactcagtcc cgagaatggc     1140

ttctggacca tttggttatg gcaagacagc tatgaggctg gtaccagtcc tcagaccacc     1200

ctccacattc aagtacctcc atgccaaatt gggatctttg tggactatga ggctggcgtt     1260

gtctccttct acaacataac tgaccatggc tccctcattt acaccttctc ggagtgtgtt     1320

tttgctggac ctctgcgacc tttcttcaat gttggtttca attatagtgg gggaaatgca     1380

gcgcctctaa agctctgtcc actaaagatg tag                                  1413


<210>  5
<211>  199
<212>  PRT
<213>  Artificial Sequence

<220>
<223>  Artificially synthesized sequence

<400>  5

Met Gly Ser Ser His His His His His His Ser Ser Gly Leu Val Pro
1               5                   10                  15

Arg Gly Ser His Met His Ile Thr Leu Asp Pro Asp Thr Ala Asn Pro
            20                  25                  30

Trp Leu Ile Leu Ser Glu Asp Arg Arg Gln Val Arg Leu Gly Asp Thr
        35                  40                  45

Gln Gln Ser Ile Pro Gly Asn Glu Glu Arg Phe Asp Ser Tyr Pro Met
    50                  55                  60

Val Leu Gly Ala Gln His Phe His Ser Gly Lys His Tyr Trp Glu Val
65                  70                  75                  80

Asp Val Thr Gly Lys Glu Ala Trp Asp Leu Gly Val Cys Arg Asp Ser
                85                  90                  95

Val Arg Arg Lys Gly His Phe Leu Leu Ser Ser Lys Ser Gly Phe Trp
            100                 105                 110

Thr Ile Trp Leu Trp Asn Lys Gln Lys Tyr Glu Ala Gly Thr Tyr Pro
        115                 120                 125

Gln Thr Pro Leu His Leu Gln Val Pro Pro Cys Gln Val Gly Ile Phe
    130                 135                 140
```

Leu Asp Tyr Glu Ala Gly Met Val Ser Phe Tyr Asn Ile Thr Asp His
145                 150                 155                 160

Gly Ser Leu Ile Tyr Ser Phe Ser Glu Cys Ala Phe Thr Gly Pro Leu
                165                 170                 175

Arg Pro Phe Phe Ser Pro Gly Phe Asn Asp Gly Gly Lys Asn Thr Ala
            180                 185                 190

Pro Leu Thr Leu Cys Pro Leu
        195

<210>  6
<211>  200
<212>  PRT
<213>  Artificial Sequence

<220>
<223>  Artificially synthesized sequence

<400>  6

Met Gly Ser Ser His His His His His His Ser Ser Gly Leu Val Pro
1               5                   10                  15

Arg Gly Ser His Met His Ile Thr Leu Asp Arg Asn Thr Ala Asn Ser
            20                  25                  30

Trp Leu Ile Ile Ser Lys Asp Arg Arg Gln Val Arg Met Gly Asp Thr
        35                  40                  45

His Gln Asn Val Ser Asp Asn Lys Glu Arg Phe Ser Asn Tyr Pro Met
    50                  55                  60

Val Leu Gly Ala Gln Arg Phe Ser Ser Gly Lys Met Tyr Trp Glu Val
65                  70                  75                  80

Asp Val Thr Gln Lys Glu Ala Trp Asp Leu Gly Val Cys Arg Asp Ser
                85                  90                  95

Val Gln Arg Lys Gly Gln Phe Ser Leu Ser Pro Glu Asn Gly Phe Trp
            100                 105                 110

Thr Ile Trp Leu Trp Gln Asp Ser Tyr Glu Ala Gly Thr Ser Pro Gln
        115                 120                 125

Thr Thr Leu His Ile Gln Val Pro Pro Cys Gln Ile Gly Ile Phe Val
    130                 135                 140

```
Asp Tyr Glu Ala Gly Val Val Ser Phe Tyr Asn Ile Thr Asp His Gly
145                 150                 155                 160

Ser Leu Ile Tyr Thr Phe Ser Glu Cys Val Phe Ala Gly Pro Leu Arg
                165                 170                 175

Pro Phe Phe Asn Val Gly Phe Asn Tyr Ser Gly Gly Asn Ala Ala Pro
            180                 185                 190

Leu Lys Leu Cys Pro Leu Lys Met
        195                 200


<210>  7
<211>  119
<212>  PRT
<213>  Artificial Sequence

<220>
<223>  Artificially synthesized sequence

<400>  7

Gln Val Gln Leu Gln Glu Ser Gly Pro Gly Leu Val Arg Pro Ser Gln
1               5                   10                  15

Thr Leu Ser Leu Thr Cys Thr Val Ser Gly Tyr Ser Ile Thr Ser Asp
            20                  25                  30

His Ala Trp Ser Trp Val Arg Gln Pro Pro Gly Arg Gly Leu Glu Trp
        35                  40                  45

Ile Gly Tyr Ile Ser Tyr Ser Gly Ile Thr Thr Tyr Asn Pro Ser Leu
    50                  55                  60

Lys Ser Arg Val Thr Met Leu Arg Asp Thr Ser Lys Asn Gln Phe Ser
65                  70                  75                  80

Leu Arg Leu Ser Ser Val Thr Ala Ala Asp Thr Ala Val Tyr Tyr Cys
                85                  90                  95

Ala Arg Ser Leu Ala Arg Thr Thr Ala Met Asp Tyr Trp Gly Gln Gly
            100                 105                 110

Ser Leu Val Thr Val Ser Ser
        115


<210>  8
<211>  328
<212>  PRT
<213>  Artificial Sequence
```

<220>
<223> Artificially synthesized sequence

<400> 8

Ala Ser Thr Lys Gly Pro Ser Val Phe Pro Leu Ala Pro Ser Ser Lys
1 5 10 15

Ser Thr Ser Gly Gly Thr Ala Ala Leu Gly Cys Leu Val Lys Asp Tyr
20 25 30

Phe Pro Glu Pro Val Thr Val Ser Trp Asn Ser Gly Ala Leu Thr Ser
35 40 45

Gly Val His Thr Phe Pro Ala Val Leu Gln Ser Ser Gly Leu Tyr Ser
50 55 60

Leu Ser Ser Val Val Thr Val Pro Ser Ser Ser Leu Gly Thr Gln Thr
65 70 75 80

Tyr Ile Cys Asn Val Asn His Lys Pro Ser Asn Thr Lys Val Asp Lys
85 90 95

Lys Val Glu Pro Lys Ser Cys Asp Lys Thr His Thr Cys Pro Pro Cys
100 105 110

Pro Ala Pro Glu Leu Leu Gly Gly Pro Ser Val Phe Leu Phe Pro Pro
115 120 125

Lys Pro Lys Asp Thr Leu Met Ile Ser Arg Thr Pro Glu Val Thr Cys
130 135 140

Val Val Val Asp Val Ser His Glu Asp Pro Glu Val Lys Phe Asn Trp
145 150 155 160

Tyr Val Asp Gly Val Glu Val His Asn Ala Lys Thr Lys Pro Arg Glu
165 170 175

Glu Gln Tyr Asn Ser Thr Tyr Arg Val Val Ser Val Leu Thr Val Leu
180 185 190

His Gln Asp Trp Leu Asn Gly Lys Glu Tyr Lys Cys Lys Val Ser Asn
195 200 205

Lys Ala Leu Pro Ala Pro Ile Glu Lys Thr Ile Ser Lys Ala Lys Gly
210 215 220

Gln Pro Arg Glu Pro Gln Val Tyr Thr Leu Pro Pro Ser Arg Asp Glu

225 230 235 240

Leu Thr Lys Asn Gln Val Ser Leu Thr Cys Leu Val Lys Gly Phe Tyr
245 250 255

Pro Ser Asp Ile Ala Val Glu Trp Glu Ser Asn Gly Gln Pro Glu Asn
260 265 270

Asn Tyr Lys Thr Thr Pro Pro Val Leu Asp Ser Asp Gly Ser Phe Phe
275 280 285

Leu Tyr Ser Lys Leu Thr Val Asp Lys Ser Arg Trp Gln Gln Gly Asn
290 295 300

Val Phe Ser Cys Ser Val Met His Glu Ala Leu His Asn His Tyr Thr
305 310 315 320

Gln Lys Ser Leu Ser Leu Ser Pro
325

<210> 9
<211> 107
<212> PRT
<213> Artificial Sequence

<220>
<223> Artificially synthesized sequence

<400> 9

Asp Ile Gln Met Thr Gln Ser Pro Ser Ser Leu Ser Ala Ser Val Gly
1 5 10 15

Asp Arg Val Thr Ile Thr Cys Arg Ala Ser Gln Asp Ile Ser Ser Tyr
20 25 30

Leu Asn Trp Tyr Gln Gln Lys Pro Gly Lys Ala Pro Lys Leu Leu Ile
35 40 45

Tyr Tyr Thr Ser Arg Leu His Ser Gly Val Pro Ser Arg Phe Ser Gly
50 55 60

Ser Gly Ser Gly Thr Asp Phe Thr Phe Thr Ile Ser Ser Leu Gln Pro
65 70 75 80

Glu Asp Ile Ala Thr Tyr Tyr Cys Gln Gln Gly Asn Thr Leu Pro Tyr
85 90 95

Thr Phe Gly Gln Gly Thr Lys Val Glu Ile Lys
100 105

<210> 10
<211> 107
<212> PRT
<213> Artificial Sequence

<220>
<223> Artificially synthesized sequence

<400> 10

Arg Thr Val Ala Ala Pro Ser Val Phe Ile Phe Pro Pro Ser Asp Glu
1               5                   10                  15

Gln Leu Lys Ser Gly Thr Ala Ser Val Val Cys Leu Leu Asn Asn Phe
            20                  25                  30

Tyr Pro Arg Glu Ala Lys Val Gln Trp Lys Val Asp Asn Ala Leu Gln
        35                  40                  45

Ser Gly Asn Ser Gln Glu Ser Val Thr Glu Gln Asp Ser Lys Asp Ser
    50                  55                  60

Thr Tyr Ser Leu Ser Ser Thr Leu Thr Leu Ser Lys Ala Asp Tyr Glu
65                  70                  75                  80

Lys His Lys Val Tyr Ala Cys Glu Val Thr His Gln Gly Leu Ser Ser
                85                  90                  95

Pro Val Thr Lys Ser Phe Asn Arg Gly Glu Cys
            100                 105

<210> 11
<211> 120
<212> PRT
<213> Artificial Sequence

<220>
<223> Artificially synthesized sequence

<400> 11

Glu Val Gln Leu Val Glu Ser Gly Gly Gly Leu Val Gln Pro Gly Gly
1               5                   10                  15

Ser Leu Arg Leu Ser Cys Ala Ala Ser Gly Tyr Thr Phe Thr Ser Tyr
            20                  25                  30

Val Met His Trp Val Arg Gln Ala Pro Gly Lys Gly Leu Glu Trp Val
        35                  40                  45

Ser Ala Ile Asn Pro Tyr Asn Asp Gly Asn Tyr Tyr Ala Asp Ser Val

```
    50                  55                  60

Lys Gly Arg Phe Thr Ile Ser Arg Asp Asn Ser Arg Lys Thr Leu Tyr
65                  70                  75                  80

Leu Gln Met Asn Ser Leu Arg Ala Glu Asp Thr Ala Val Tyr Tyr Cys
                85                  90                  95

Ala Arg Gly Ala Tyr Lys Arg Gly Tyr Ala Met Asp Tyr Trp Gly Gln
            100                 105                 110

Gly Thr Thr Val Thr Val Ser Ser
        115                 120


<210>  12
<211>  348
<212>  PRT
<213>  Artificial Sequence

<220>
<223>  Artificially synthesized sequence

<400>  12

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Val Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140
```

```
Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
    210                 215                 220

Gln Pro Arg Glu Pro Gln Val Tyr Thr Leu Pro Pro Ser Arg Glu Glu
225                 230                 235                 240

Met Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro Gly Gly Gly Gly Ser Gly Leu Asn
                325                 330                 335

Asp Ile Phe Glu Ala Gln Lys Ile Glu Trp His Glu
            340                 345
```

<210> 13
<211> 113
<212> PRT
<213> Artificial Sequence

<220>
<223> Artificially synthesized sequence

<400> 13

Asp Leu Val Met Thr Gln Ser Pro Ser Ser Leu Ser Ala Ser Val Gly
1               5                   10                  15

Asp Arg Val Thr Ile Thr Cys Lys Ser Ser Gln Ser Leu Phe Asn Ser
            20                  25                  30

Arg Thr Arg Lys Asn Tyr Leu Ala Trp Tyr Gln Gln Lys Pro Gly Lys
        35                  40                  45

Ala Pro Lys Leu Leu Ile Tyr Trp Ala Ser Thr Arg Glu Ser Gly Val
    50                  55                  60

Pro Ser Arg Phe Ser Gly Ser Gly Ser Gly Thr Asp Phe Thr Leu Thr
65                  70                  75                  80

Ile Ser Ser Leu Gln Pro Glu Asp Phe Ala Thr Tyr Tyr Cys Gln Gln
                85                  90                  95

Tyr Tyr Tyr His Met Tyr Thr Phe Gly Gln Gly Thr Lys Val Glu Ile
            100                 105                 110

Lys

<210>  14
<211>  345
<212>  PRT
<213>  Artificial Sequence

<220>
<223>  Artificially synthesized sequence

<400>  14

Ala Ser Thr Lys Gly Pro Ser Val Phe Pro Leu Ala Pro Cys Ser Arg
1               5                   10                  15

Ser Thr Ser Glu Ser Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Thr Cys Asn Val Asp His Lys Pro Ser Asn Thr Lys Val Asp Lys
85 90 95

Arg Val Glu Ser Lys Tyr Gly Pro Pro Cys Pro Pro Cys Pro Ala Pro
100 105 110

Glu Phe Arg Arg Gly Pro Ser Val Phe Leu Phe Pro Pro Lys Pro Lys
115 120 125

Asp Thr Leu Met Ile Ser Arg Thr Pro Glu Val Thr Cys Val Val Val
130 135 140

Asp Val Ser Gln Glu Asp Pro Glu Val Gln Phe Asn Trp Tyr Val Asp
145 150 155 160

Gly Val Glu Val His Asn Ala Lys Thr Lys Pro Arg Glu Glu Gln Tyr
165 170 175

Asn Ser Thr Tyr Arg Val Val Ser Val Leu Thr Val Leu His Gln Asp
180 185 190

Trp Leu Asn Gly Lys Glu Tyr Lys Cys Lys Val Ser Asn Lys Gly Leu
195 200 205

Pro Ser Ser Ile Glu Lys Thr Ile Ser Lys Ala Lys Gly Gln Pro Arg
210 215 220

Glu Pro Gln Val Tyr Thr Leu Pro Pro Ser Gln Lys Glu Met Thr Lys
225 230 235 240

Asn Gln Val Ser Leu Thr Cys Leu Val Lys Gly Phe Tyr Pro Ser Asp
245 250 255

Ile Ala Val Glu Trp Glu Ser Asn Gly Gln Pro Glu Asn Asn Tyr Lys
260 265 270

Thr Thr Pro Pro Val Leu Asp Ser Asp Gly Ser Phe Phe Leu Tyr Ser
275 280 285

Arg Leu Thr Val Asp Lys Ser Arg Trp Gln Glu Gly Asn Val Phe Ser
290 295 300

Cys Ser Val Met His Glu Ala Leu His Asn His Tyr Thr Gln Lys Ser
305 310 315 320

Leu Ser Leu Ser Pro Gly Gly Gly Gly Ser Gly Leu Asn Asp Ile Phe
325 330 335

Glu Ala Gln Lys Ile Glu Trp His Glu
340 345

<210> 15
<211> 321
<212> PRT
<213> Artificial Sequence

<220>
<223> Artificially synthesized sequence

<400> 15

Met Lys Asp Asn Thr Val Pro Leu Lys Leu Ile Ala Leu Leu Ala Asn
1 5 10 15

Gly Glu Phe His Ser Gly Glu Gln Leu Gly Glu Thr Leu Gly Met Ser
20 25 30

Arg Ala Ala Ile Asn Lys His Ile Gln Thr Leu Arg Asp Trp Gly Val
35 40 45

Asp Val Phe Thr Val Pro Gly Lys Gly Tyr Ser Leu Pro Glu Pro Ile
50 55 60

Gln Leu Leu Asn Ala Lys Gln Ile Leu Gly Gln Leu Asp Gly Gly Ser
65 70 75 80

Val Ala Val Leu Pro Val Ile Asp Ser Thr Asn Gln Tyr Leu Leu Asp
85 90 95

Arg Ile Gly Glu Leu Lys Ser Gly Asp Ala Cys Ile Ala Glu Tyr Gln
100 105 110

Gln Ala Gly Arg Gly Arg Arg Gly Arg Lys Trp Phe Ser Pro Phe Gly
115 120 125

Ala Asn Leu Tyr Leu Ser Met Phe Trp Arg Leu Glu Gln Gly Pro Ala
130 135 140

Ala Ala Ile Gly Leu Ser Leu Val Ile Gly Ile Val Met Ala Glu Val
145 150 155 160

Leu Arg Lys Leu Gly Ala Asp Lys Val Arg Val Lys Trp Pro Asn Asp
165 170 175

Leu Tyr Leu Gln Asp Arg Lys Leu Ala Gly Ile Leu Val Glu Leu Thr
180 185 190

```
Gly Lys Thr Gly Asp Ala Ala Gln Ile Val Ile Gly Ala Gly Ile Asn
        195                 200                 205

Met Ala Met Arg Arg Val Glu Glu Ser Val Val Asn Gln Gly Trp Ile
    210                 215                 220

Thr Leu Gln Glu Ala Gly Ile Asn Leu Asp Arg Asn Thr Leu Ala Ala
225                 230                 235                 240

Met Leu Ile Arg Glu Leu Arg Ala Ala Leu Glu Leu Phe Glu Gln Glu
                245                 250                 255

Gly Leu Ala Pro Tyr Leu Ser Arg Trp Glu Lys Leu Asp Asn Phe Ile
            260                 265                 270

Asn Arg Pro Val Lys Leu Ile Ile Gly Asp Lys Glu Ile Phe Gly Ile
        275                 280                 285

Ser Arg Gly Ile Asp Lys Gln Gly Ala Leu Leu Leu Glu Gln Asp Gly
    290                 295                 300

Ile Ile Lys Pro Trp Met Gly Gly Glu Ile Ser Leu Arg Ser Ala Glu
305                 310                 315                 320

Lys

<210>  16
<211>  328
<212>  PRT
<213>  Artificial Sequence

<220>
<223>  Artificially synthesized sequence

<400>  16

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80
```

```
Tyr Ile Cys Asn Val Asn His Lys Pro Ser Asn Thr Lys Val Asp Lys
                85                  90                  95

Lys Val Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
    210                 215                 220

Gln Pro Arg Glu Pro Gln Val Tyr Thr Leu Pro Pro Ser Arg Glu Glu
225                 230                 235                 240

Met Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro
                325
```

<210> 17
<211> 127
<212> PRT
<213> Artificial Sequence

<220>
<223> Artificially synthesized sequence

<400> 17

```
Arg Thr Val Ala Ala Pro Ser Val Phe Ile Phe Pro Pro Ser Asp Glu
1               5                   10                  15

Gln Leu Lys Ser Gly Thr Ala Ser Val Val Cys Leu Leu Asn Asn Phe
            20                  25                  30

Tyr Pro Arg Glu Ala Lys Val Gln Trp Lys Val Asp Asn Ala Leu Gln
        35                  40                  45

Ser Gly Asn Ser Gln Glu Ser Val Thr Glu Gln Asp Ser Lys Asp Ser
    50                  55                  60

Thr Tyr Ser Leu Ser Ser Thr Leu Thr Leu Ser Lys Ala Asp Tyr Glu
65                  70                  75                  80

Lys His Lys Val Tyr Ala Cys Glu Val Thr His Gln Gly Leu Ser Ser
                85                  90                  95

Pro Val Thr Lys Ser Phe Asn Arg Gly Glu Cys Gly Gly Gly Gly Ser
            100                 105                 110

Gly Leu Asn Asp Ile Phe Glu Ala Gln Lys Ile Glu Trp His Glu
        115                 120                 125
```

## Description

[Technical Field]

The present disclosure relates to antigen-binding molecules comprising an altered TRIM21-binding domain and having an altered cytosolic half-life; pharmaceutical compositions comprising such an antigen-binding molecule; methods for using such an antigen-binding molecule; methods for increasing or decreasing the cytosolic half-life of an antigen-binding molecule comprising a TRIM21-binding domain; and methods for producing an antigen-binding molecule comprising an altered TRIM21-binding domain and having an increased or decreased cytosolic half-life.

[Background Art]

Antibodies are proteins which specifically bind to an antigen with high affinity. Various molecules from low-molecular-weight compounds to proteins are known to be antigens. Since the technique for producing monoclonal antibodies was developed, antibody modification techniques have advanced, making it easy to obtain antibodies that recognize a particular molecule.

Antibodies are receiving attention as pharmaceuticals because they are highly stable in plasma and have less side effects. Not only do antibodies bind to an antigen and exhibit agonistic and antagonistic effects, but they also induce cytotoxic activity mediated by effector cells (also referred to as effector functions) including ADCC (Antibody Dependent Cytotoxicity), ADCP (Antibody Dependent Cell Phagocytosis), and CDC (Complement Dependent Cytotoxicity). Taking advantage of these antibody functions, pharmaceuticals for cancer, immune diseases, chronic diseases, infections, etc. have been developed (Nat. Rev. Drug Discov. 2018 Mar; 17(3): 197-223 (NPL 1)).

Meanwhile, since a full-length IgG molecule, which has a large molecular weight of approximately 150 kDa, generally does not show cell permeability, the antibody pharmaceuticals only target antigens on the cell membrane or extracellular antigens. This led to development of technologies such as intrabodies, which are antibodies or antibody fragments expressed intracellularly; antibody-CPP complexes, which are produced by fusion to a cell-penetrating peptide (CPP); and protein transfection methods; and these have been reported as technologies for allowing antibodies to act on intracellular antigens (MAbs. 2011 Jan-Feb; 3(1):3-16 (NPL 2)). Furthermore, for example, anti-DNA autoantibodies identified in systemic lupus erythematosus (SLE) patients and the MRL-mpj/lpr lupus mouse model have been reported to show a cytosol-penetrating ability (Sci. Rep. 2015 Jul 9; 5:12022 (NPL 3); and Mol. Immunol. 2015 Oct; 67(2 Pt B):377-87 (NPL 4)).

When an antibody is taken into cells by endocytosis, it normally binds to the neonatal Fc receptor (FcRn) at its Fc region, allowing itself to be recycled into the plasma so that it avoids lysosomal degradation. On the other hand, when an antibody enters the cytosol, it binds to tripartite motif 21 (TRIM21), which is a cytosolic Fc receptor. TRIM21 is an E3 ubiquitin ligase. It has been reported that when an antiviral antibody binds to an antigen and enters the cytosol, TRIM21 binds to the Fc region of the antibody, and autopolyubiquitination of TRIM21 causes the virus-antibody complex bound to TRIM21 to be guided to the proteasome and degraded (Trends Immunol. 2017 Dec; 38(12):916-926. (NPL 5)). An anti-human adenovirus type 5 (AdV5) antibody with TRIM21-binding affinity weakened by about 100-fold can reportedly still neutralize AdV5 while being able to prevent TRIM21-dependent activation of NF-kB signals (J. Immunol. 2016 Apr 15;196(8):3452-3459 (NPL 6)).

[Citation List]

[Non-Patent Literature]

[NPL 1] Carter PJ, Lazar GA. Nat Rev Drug Discov. 2018 Mar;17(3):197-223.

[NPL 2] Marschall AL, Frenzel A, Schirrmann T, Schungel M, Dubel S. MAbs. 2011 Jan-Feb;3(1):3-16.

[NPL 3] Weisbart RH, Chan G, Jordaan G, Noble PW, Liu Y, Glazer PM, Nishimura RN, Hansen JE. Sci Rep. 2015 Jul 9;5:12022.

[NPL 4] Im SR, Im SW, Chung HY, Pravinsagar P, Jang YJ. Mol Immunol. 2015 Oct;67(2 Pt B):377-87.

[NPL 5] Rhodes DA, Isenberg DA. Trends Immunol. 2017 Dec;38(12):916-926.

[NPL 6] Foss S, Watkinson RE, Grevys A, McAdam MB, Bern M, Hoydahl LS, Dalhus B, Michaelsen TE, Sandlie I, James LC, Andersen JT. J Immunol. 2016 Apr 15;196(8):3452-3459

[Summary of Invention]

[Technical Problem]

The present invention was achieved in view of the above circumstances. In a non-limiting embodiment, an objective of the present invention is to provide: antigen-binding molecules comprising an altered TRIM21-binding domain and having an altered cytosolic half-life; pharmaceutical compositions comprising such an antigen-binding molecule; methods for using such an antigen-binding molecule; methods for increasing or decreasing the cytosolic half-life of an antigen-binding molecule comprising a TRIM21-binding domain; and methods for producing an antigen-binding molecule comprising an altered TRIM21-binding domain and having an increased or decreased cytosolic half-life.

[Means for Solving the Problems]

In a non-limiting embodiment, the present inventors conducted dedicated research and discovered that an antigen-binding molecule having an altered TRIM21-binding domain with a decreased or increased binding affinity for TRIM21 has an increased or decreased cytosolic half-life compared to the parent antigen-binding molecule. The present inventors also discovered that the cytosolic half-life of an antigen-binding molecule having a TRIM21-binding domain is increased or decreased by substitution at a specific position in the TRIM21-binding domain.

The present disclosure is based on these findings, and specifically includes the embodiments exemplified below:

[1] a method for producing an antigen-binding molecule comprising an altered TRIM21-binding domain, which comprises introducing one or more amino acid alterations into a TRIM21-binding domain of a parent antigen-binding molecule, wherein the alterations result in a decrease or an increase in the binding affinity of the antigen-binding molecule for TRIM21 compared to that of the parent antigen-binding molecule, and
wherein the amount of the antigen-binding molecule present within the cytosol of a cell after a certain amount of the antigen-binding molecule is contacted with the cell is increased or decreased compared to the amount of the parent antigen-binding molecule present within the cytosol of a cell after the same amount of the parent antigen-binding molecule is contacted with the cell;
[2] the method of [1], which further comprises comparing the amount of the antigen-binding molecule comprising the altered TRIM21-binding domain present within the cytosol of a cell after the antigen-binding molecule is contacted with the cell with the amount of the parent antigen-binding molecule present in the cytosol of a cell after the same amount of the parent antigen-binding molecule is contacted with the cell;
[3] a method for producing an antigen-binding molecule comprising an altered TRIM21-binding domain, which comprises introducing one or more amino acid alterations into a TRIM21-binding domain of a parent antigen-binding molecule, wherein the alterations result in a decrease or an increase in the binding affinity of the antigen-binding molecule for TRIM21 compared to that of the parent antigen-binding molecule, and
wherein the cytosolic half-life of the antigen-binding molecule is increased or decreased compared to that of the parent antigen-binding molecule;
[4] a method for producing an antigen-binding molecule comprising an altered TRIM21-binding domain, which comprises introducing one or more amino acid alterations into a TRIM21-binding domain of a parent antigen-binding molecule, wherein the alterations result in an increase or a decrease in the binding affinity of the TRIM21-binding domain for TRIM21 compared to that of the parent antigen-binding molecule, and
wherein the antigen-binding molecule has an decreased or increased ability to remove a cytosolic antigen compared to the parent antigen-binding molecule;
[5] the method of any one of [1] to [4], wherein the TRIM21 is human TRIM21 and/or mouse TRIM21;
[6] the method of any one of [1] to [5], wherein the alterations further increase or decrease the resistance of the antigen-binding molecule to proteasomal degradation in the cytosol compared to that of the parent antigen-binding molecule;
[7] the method of any one of [1] to [6], which further comprises:

(a) obtaining an expression vector comprising an appropriate promoter operably linked to a gene encoding the antigen-binding molecule produced by the method of any one of [1] to [6];
(b) introducing the vector into a host cell, and culturing the host cell to produce the antigen-binding molecule; and
(c) recovering the antigen-binding molecule from the culture of the host cell;

[8] the method of any one of [1] to [7], wherein the TRIM21-binding domain is an antibody Fc region;
[9] the method of any one of [1] to [8], wherein the TRIM21-binding domain is an IgG Fc region;
[10] the method of any one of [1] to [9], wherein the TRIM21-binding domain is a human IgG Fc region;

[11] the method of any one of [1] to [10], wherein the TRIM21-binding domain is a human IgG Fc region CH2-CH3 domain;
[12] the method of any one of [1] to [11], wherein the one or more amino acid alterations are selected from the group consisting of EU253, EU309, EU311, EU312, EU314, EU315, EU345, EU428, EU432, EU433, EU434, EU435, EU436, EU437, EU438, EU439, and EU440, wherein the numbers indicate the positions of substitution according to EU numbering;
[13] the method of any one of [1] to [12], wherein the one or more amino acid alterations are selected from the group consisting of EU253, EU314, EU315, EU428, EU432, EU433, EU434, EU435, EU436, and EU437, wherein the numbers indicate the positions of substitution according to EU numbering;
[14] the method of any one of [1] to [13], wherein the one or more amino acid alterations are selected from the group consisting of EU253F, EU314K, EU428R, EU434G, EU436D, and EU437V, wherein the numbers indicate the positions of substitution according to EU numbering;
[15] the method of any one of [1] to [12], wherein the antigen-binding molecule comprising the altered TRIM21-binding domain has thermal stability that is not remarkably lowered compared to that of the parent antigen-binding molecule;
[16] the method of [15], wherein the thermal stability is represented by a Tm value;
[17] the method of [15] or [16], wherein the one or more amino acid alterations are selected from the group consisting of EU253, EU309, EU311, EU315, EU428, EU435, EU438, and EU440, wherein the numbers indicate the positions of substitution according to EU numbering;
[18] the method of any one of [15] to [17], wherein the one or more amino acid alterations are selected from the amino acid alterations of Group 1 in Table 5 (1) and (2), or a combination thereof;
[19] the method of any one of [1] to [7], wherein the antigen-binding molecule further comprises an FcRn-binding domain, and the alterations do not substantially change the binding of the FcRn-binding domain to FcRn;
[20] the method of any one of [1] to [7], wherein the antigen-binding molecule further comprises an FcRn-binding domain, and the alterations increase or decrease the binding of the FcRn-binding domain to FcRn compared to that of the parent antigen-binding molecule;
[21] the method of [19] or [20], which further comprises introducing one or more amino acid alterations into the FcRn-binding domain, and the alterations result in an increase or a decrease in the binding of the FcRn-binding domain to FcRn compared to that of the parent FcRn-binding domain;
[22] the method of any one of [19] to [21], wherein the FcRn-binding domain is an antibody Fc region;
[23] the method of any one of [19] to [22], wherein the FcRn-binding domain is an IgG Fc region;
[24] the method of any one of [19] to [23], wherein the FcRn-binding domain is a human IgG Fc region;
[25] the method of any one of [19] to [24], wherein the FcRn-binding domain is a human IgG Fc region CH2-CH3 domain;
[26] the method of any one of [19] to [25], wherein the one or more amino acid alterations are selected from the group consisting of EU309, EU311, EU312, EU428, EU433, EU434, EU436, and EU438, wherein the numbers indicate the positions of substitution according to EU numbering;
[27] the method of any one of [19] to [25], wherein the one or more amino acid alterations are selected from the group consisting of the amino acid alterations included in Groups A, C, E, and G of Table 4 (2);
[28] the method of any one of [19] to [25], wherein the one or more amino acid alterations are selected from the group consisting of EU253, EU309, EU311, EU312, EU314, EU315, EU345, EU428, EU432, EU433, EU434, EU435, EU436, EU437, EU438, EU439, and EU440, wherein the numbers indicate the positions of substitution according to EU numbering;
[29] the method of any one of [19] to [25], wherein the one or more amino acid alterations are selected from the group consisting of the amino acid alterations included in Groups B, D, F, and H of Table 4 (2);
[30] the method of any one of [1] to [12], [15] to [16], and [19] to [25], wherein the antigen-binding molecule further comprises a Protein A-binding domain, and the alterations do not substantially change the binding of the Protein A-binding domain to Protein A;
[31] the method of [30], wherein the Protein A-binding domain is an antibody Fc region;
[32] the method of [30] or [31], wherein the Protein A -binding domain is an IgG Fc region;
[33] the method of any one of [30] to [32], wherein the Protein A-binding domain is a human IgG Fc region;
[34] the method of any one of [30] to [33], wherein the Protein A-binding domain is a human IgG Fc region CH2-CH3 domain;
[35] the method of any one of [1] to [34], wherein the antigen-binding molecule has an ability to penetrate into cytosol;
[36] the method of [35], wherein the antigen-binding molecule has one or more cytosol-penetrating domains;
[37] the method of [36], wherein the cytosol-penetrating domain is selected from an antibody variable region, a peptide portion, and a nucleic acid portion;
[38] the method of [37], wherein the peptide portion is a cell-penetrating peptide (CPP), and the nucleic acid portion is a phosphorothioate DNA (PS-DNA);
[39] the method of any one of [35] to [38], wherein the alterations do not remarkably decrease the ability of the antigen-binding molecule to penetrate into cytosol;
[40] a method for producing an antigen-binding molecule comprising an altered TRIM21-binding domain, which comprises:

(a) providing a parent antigen-binding molecule having a TRIM21-binding domain;
(b) obtaining a candidate molecule comprising an altered TRIM21-binding domain by introducing one or more amino acid alterations into the TRIM21-binding domain of the parent antigen-binding molecule;
(c) determining the binding affinity of the altered TRIM21-binding domain for TRIM21;
(d) identifying the candidate molecule as a suitable molecule when the altered TRIM21-binding domain binds to TRIM21 with higher or lower binding affinity than the TRIM21-binding domain of the parent antigen-binding molecule;
(e) obtaining an expression vector comprising an appropriate promoter operably linked to a gene encoding the suitable molecule;
(d) introducing the vector into a host cell and culturing the host cell to produce the suitable molecule; and
(e) recovering the suitable molecule from the culture of the host cell;

wherein the suitable molecule has an increased or decreased cytosolic half-life compared to the parent antigen-binding molecule;
[41] the method of [40], further comprising:

(a) obtaining an expression vector comprising an appropriate promoter operably linked to a gene encoding the candidate molecule identified as the suitable molecule in [40];
(b) introducing the vector into a host cell and culturing the host cell to produce the antigen-binding molecule; and
(c) recovering the antigen-binding molecule from the culture of the host cell;

[42] a method of screening for an antigen-binding molecule comprising an altered TRIM21-binding domain, which comprises:

(a) providing a parent antigen-binding molecule having a TRIM21-binding domain;
(b) obtaining a candidate molecule comprising an altered TRIM21-binding domain by introducing one or more amino acid alterations into the TRIM21-binding domain of the parent antigen-binding molecule;
(c) determining the binding affinity of the altered TRIM21-binding domain for TRIM21; and
(d) identifying the candidate molecule as a suitable molecule when the altered TRIM21-binding domain binds to TRIM21 with a higher or lower binding affinity than the TRIM21-binding domain of the parent antigen-binding molecule;

wherein the suitable molecule has an increased or decreased cytosolic half-life compared to the parent antigen-binding molecule;
[43] a method for increasing or decreasing the cytosolic half-life of an antigen-binding molecule compared to that of a parent antigen-binding molecule, which comprises introducing one or more amino acid alterations into a TRIM21-binding domain of the parent antigen-binding molecule, wherein the alterations result in a decrease or an increase in the binding affinity of the TRIM21-binding domain for TRIM21 compared to that of the parent antigen-binding molecule;
[44] a method for producing an antigen-binding molecule comprising an altered TRIM21-binding domain, which comprises introducing an amino acid alteration into the TRIM21-binding domain of a parent antigen-binding molecule at one or more positions selected from the group consisting of EU253, EU428, EU433, and EU435,
wherein the amount of the antigen-binding molecule present within the cytosol of a cell after a certain amount of the antigen-binding molecule is contacted with the cell is increased compared to the amount of the parent antigen-binding molecule present within the cytosol of a cell after the same amount of the parent antigen-binding molecule is contacted with the cell, and wherein the numbers indicate the positions of substitution according to EU numbering;
[45] the method of [44], wherein the amino acid alteration at one or more positions is one or more amino acid alterations selected from the group consisting of I253F, M428R, H433A, and H435A;
[46] the method of [44] or [45], wherein the amino acid alteration at one or more positions is I253F and/or M428R;
[47] the method of any one of [44] to [46], wherein the antigen-binding molecule has a cytosol-penetrating ability;
[48] a method for producing an antigen-binding molecule comprising an altered FcRn-binding domain, which comprises introducing an amino acid alteration into the FcRn-binding domain of a parent antigen-binding molecule at one or more positions selected from the group consisting of EU253, EU428, EU433, and EU435, wherein the numbers indicate the positions of substitution according to EU numbering;
[49] the method of [48], wherein the amino acid alteration at one or more positions is one or more amino acid alterations selected from the group consisting of I253F, M428R, H433A, and H435A;
[50] the method of [48] or [49], wherein the amino acid alteration at one or more positions is I253F and/or M428R;
[51] the method of any one of [48] to [50], wherein the amount of the antigen-binding molecule comprising the altered FcRn-binding domain present within the cytosol of a cell after a certain amount of the antigen-binding molecule is contacted with the cell is increased compared to the amount of the parent antigen-binding molecule present within the cytosol of a cell after the same amount of the parent antigen-binding molecule is contacted with the cell;
[52] the method of any one of [48] to [51], wherein the antigen-binding molecule has an ability to penetrate into cytosol;
[53] an antigen-binding molecule comprising an altered TRIM21-binding domain, wherein the altered TRIM21-binding domain comprises one or more amino acid alterations that result in a decrease or an increase in binding affinity for TRIM21 compared to a wildtype TRIM21-binding domain, and wherein the antigen-binding molecule has an increased or decreased cytosolic half-life compared to an antigen-binding molecule comprising a TRIM21-binding domain which does not comprise the alterations;
[54] the antigen-binding molecule of [53], wherein the altered TRIM21-binding domain comprises an amino acid substitution at one or more positions selected from the group consisting of EU253, EU309, EU311, EU312, EU314, EU315, EU345, EU428, EU432, EU433, EU434, EU435, EU436, EU437, EU438, EU439, and EU440, wherein the numbers indicate the positions of substitution according to EU numbering;
[55] the antigen-binding molecule of [53] or [54], wherein the altered TRIM21-binding domain comprises an amino acid substitution at one or more positions selected from the group consisting of EU253, EU314, EU315, EU428, EU432, EU433, EU434, EU435, EU436, and EU437, wherein the numbers indicate the positions of substitution according to EU numbering;
[56] the antigen-binding molecule of any one of [53] to [55], wherein the altered TRIM21-binding domain comprises one or more amino acid substitutions selected from the group consisting of EU253F, EU314K, EU428R, EU434G, EU436D, and EU437V, wherein the numbers indicate the positions of substitution according to EU numbering;
[57] the antigen-binding molecule of any one of [53] to [56], which has a cytosol-penetrating ability;
[58] an antigen-binding molecule comprising an altered TRIM21-binding domain, wherein the altered TRIM21-binding domain comprises an amino acid alteration at one or more positions selected from the group consisting of EU253, EU428, EU433, and EU435, wherein the antigen-binding molecule has an increased cytosolic half-life compared to an antigen-binding molecule comprising a TRIM21-binding domain which does not comprise the alteration, and wherein the numbers indicate the positions of substitution according to EU numbering;
[59] the antigen-binding molecule of [58], wherein the amino acid alteration at one or more positions is one or more amino acid alterations selected from the group consisting of I253F, M428R, H433A, and H435A;
[60] the antigen-binding molecule of [58] or [59], wherein the amino acid alteration at one or more positions is I253F and/or M428R;
[61] the antigen-binding molecule of any one of [58] to [60], which has a cytosol-penetrating ability;
[62] an antigen-binding molecule comprising an altered FcRn-binding domain, wherein the altered FcRn-binding domain comprises an amino acid alteration at one or more positions selected from the group consisting of EU253, EU428, EU433, and EU435, wherein the numbers indicate the positions of substitution according to EU numbering;
[63] the antigen-binding molecule of [62], wherein the amino acid alteration at one or more positions is one or more amino acid alterations selected from the group consisting of I253F, M428R, H433A, and H435A;
[64] the antigen-binding molecule of [62] or [63], wherein the amino acid alteration at one or more positions is I253F and/or M428R;
[65] the method of any one of [62] to [64], wherein the amount of the antigen-binding molecule comprising the altered FcRn-binding domain present within the cytosol of a cell after a certain amount of the antigen-binding molecule is contacted with the cell is increased compared to the amount of the parent antigen-binding molecule present within the cytosol of a cell after the same amount of the parent antigen-binding molecule is contacted with the cell;
[66] the antigen-binding molecule of any one of [62] to [65], which has a cytosol-penetrating ability;
[67] a method for producing an antigen-binding molecule comprising an altered TRIM21-binding domain, which comprises introducing one or more amino acid alterations into a TRIM21-binding domain of a parent antigen-binding molecule, wherein the alterations result in a decrease or an increase in the binding affinity of the TRIM21-binding domain for TRIM21 compared to that of the parent antigen-binding molecule, and wherein the antigen-binding molecule has an increased or decreased ability to activate the NF-kB signaling pathway compared to the parent antigen-binding molecule;
[68] a method for producing an antigen-binding molecule comprising an altered TRIM21-binding domain, which comprises introducing one or more amino acid alterations into a TRIM21-binding domain of a parent antigen-binding molecule, wherein the alterations result in an increase or a decrease in the binding affinity of the TRIM21-binding domain for TRIM21 compared to that of the parent antigen-binding molecule, and wherein the antigen-binding molecule has an increased or decreased ability to induce inflammatory and antitumor responses compared to the parent antigen-binding molecule;

[69] a method for imaging a cytosolic antigen in a sample cell, which comprises contacting a labeled antigen-binding molecule comprising an altered TRIM21-binding domain with a sample cell, wherein the altered TRIM21-binding domain comprises one or more amino acid alterations which result in a decrease or an increase in binding affinity for TRIM21 compared to a wildtype TRIM21-binding domain;

[70] a method for imaging a cytosolic antigen in a sample cell, which comprises microinjecting into a sample cell a labeled antigen-binding molecule comprising an altered TRIM21-binding domain, wherein the altered TRIM21-binding domain comprises one or more amino acid alterations which result in a decrease or an increase in binding affinity for TRIM21 compared to a wildtype TRIM21-binding domain;

[71] the method of [69] or [70], wherein the antigen-binding molecule has a cytosolic antigen-binding domain;

[72] a nucleic acid encoding the antigen-binding molecule of any one of [53] to [66];

[73] a vector comprising the nucleic acid of [72];

[74] a cell comprising the nucleic acid of [72] or the vector of [73];

[75] a method for producing the antigen-binding molecule of any one of [53] to [66], which comprises culturing the cell of [74]; and

[76] a pharmaceutical composition comprising the antigen-binding molecule of any one of [53] to [66], and a pharmaceutically acceptable carrier.

[Brief Description of Drawings]

Fig. 1 is a schematic diagram showing the behavior of an antigen-binding molecule comprising an altered TRIM21-binding domain of the present disclosure in the cytosol. Fig. 1a is an example of an antibody with an attenuated ability to bind to TRIM21. Since an antibody with an attenuated TRIM21-binding ability induces reduced recruitment of the ubiquitin-proteasome system and suppresses proteasomal degradation of the antibody, the antibody can accumulate more in the cytosol and act on a greater amount of the cytosolic antigen. Fig. 1b is an example of an antibody with an enhanced ability to bind to TRIM21. An antibody with an enhanced TRIM21-binding ability promotes recruitment of the ubiquitin-proteasome system and accelerates proteasomal degradation, enabling degradation of a greater amount of the bound cytosolic antigen in the proteasome.

Fig. 2 shows the result of evaluating 3D8VH-G4T1E356K.Avi/hT4VL-KT0 (WT) and antibodies produced by introducing into the WT an alteration known to attenuate the hTRIM21-binding ability, H433A or H435A. Accumulation of these antibodies in the cytosol of CHO cells was evaluated by BirA assay two hours after removal of the antibody-containing medium. A band appearing at the position indicated by the arrow below 66 kDa is an HRP luminescence signal representing the antibody biotinylated by BirA.

Fig. 3 shows the result of evaluating 3D8VH-G4T1E356K.Avi/hT4VL-KT0 (WT) and antibodies produced by introducing into the WT an alteration known to attenuate the hTRIM21-binding ability, H433A or H435A. Accumulation of these antibodies in the cytosol of Hela cells was evaluated by BirA assay two hours after removal of the antibody-containing medium. A band appearing at the position indicated by the arrow below 66 kDa is an HRP luminescence signal representing the antibody biotinylated by BirA.

Fig. 4 shows the result of evaluating 3D8VH-G1m.Avi/hT4VL-KT0 (WT) and antibodies produced by introducing into the WT an alteration that attenuates or enhances the hTRIM21-binding ability. Accumulation of these antibodies in the cytosol of CHO cells was evaluated by BirA assay two hours and four hours after removal of the antibody-containing medium. A band appearing at the position indicated by the arrow below 66 kDa is an HRP luminescence signal representing the antibody biotinylated by BirA.

Fig. 5 shows the result of evaluating 3D8VH-G1m.Avi/hT4VL-KT0 (WT) and antibodies produced by introducing into the WT an alteration that attenuates or enhances the hTRIM21-binding ability. Accumulation of these antibodies in the cytosol of Hela cells was evaluated by BirA assay two hours and four hours after removal of the antibody-containing medium. A band appearing at the position indicated by the arrow below 66 kDa is an HRP luminescence signal representing the antibody biotinylated by BirA.

Fig. 6 shows the result of fluorescence microscopic imaging analysis in which antibodies produced by introducing an alteration that attenuates or enhances the hTRIM21-binding ability were evaluated for accumulation in the cytosol of Hela cells. Fig. 6(1) is a graph which plots values obtained by normalizing the fluorescence signal at each detection time with respect to the number of cells. The vertical axis shows the fluorescence signal (arbitrary units (A.U.)), and the horizontal axis shows time after removal of the antibody-containing medium. Fig. 6(2) shows fluorescence microscopic images at each detection time.

[Description of Embodiments]

## I. DEFINITIONS

An "acceptor human framework" for the purposes herein is a framework comprising the amino acid sequence of a light chain variable domain (VL) framework or a heavy chain variable domain (VH) framework derived from a human immunoglobulin framework or a human consensus framework, as defined below. An acceptor human framework "derived from" a human immunoglobulin framework or a human consensus framework may comprise the same amino acid sequence thereof, or it may contain amino acid sequence changes. In some embodiments, the number of amino acid changes are 10 or less, 9 or less, 8 or less, 7 or less, 6 or less, 5 or less, 4 or less, 3 or less, or 2 or less. In some embodiments, the VL acceptor human framework is identical in sequence to the VL human immunoglobulin framework sequence or human consensus framework sequence.

The term "antigen-binding molecule" refers in the broadest sense to a molecule that specifically binds to an antigenic determinant. In one embodiment, an antigen-binding molecule is an antibody, antibody fragment, or antibody derivative which has a TRIM21-binding domain.

"Affinity" refers to the strength of the sum total of noncovalent interactions between a single binding site of a molecule (e.g., an antibody) and its binding partner (e.g., an antigen). Unless indicated otherwise, as used herein, "binding affinity" refers to intrinsic binding affinity which reflects a 1:1 interaction between members of a binding pair (e.g., antibody and antigen). The affinity of a molecule X for its partner Y can generally be represented by the dissociation constant (Kd). Affinity can be measured by common methods known in the art, including those described herein. Specific illustrative and exemplary embodiments for measuring binding affinity are described in the following.

An "affinity matured" antibody refers to an antibody with one or more alterations in one or more hypervariable regions (HVRs), compared to a parent antibody which does not possess such alterations, such alterations resulting in an improvement in the affinity of the antibody for antigen.

The term "antibody" herein is used in the broadest sense and encompasses various antibody structures, including but not limited to monoclonal antibodies, polyclonal antibodies, multispecific antibodies (e.g., bispecific antibodies), and antibody fragments so long as they exhibit the desired antigen-binding activity.

An "agonist" antigen-binding molecule or "agonist" antibody," as used herein, is an antibody which significantly potentiates a biological activity of the antigen it binds.

A "blocking" antigen-binding molecule or "blocking" antibody, or an "antagonist" antigen-binding molecule or "antagonist" antibody, as used herein, is one which significantly inhibits (either partially or completely) a biological activity of the antigen it binds.

An "antibody fragment" refers to a molecule other than an intact antibody that comprises a portion of an intact antibody that binds the antigen to which the intact antibody binds. Examples of antibody fragments include but are not limited to Fv, Fab, Fab', Fab'-SH, $F(ab')_2$; diabodies; linear antibodies; single-chain antibody molecules (e.g. scFv); and multispecific antibodies formed from antibody fragments.

An "antibody that binds to the same epitope" as a reference antibody refers to an antibody that blocks binding of the reference antibody to its antigen in a competition assay by 50% or more, and conversely, the reference antibody blocks binding of the antibody to its antigen in a competition assay by 50% or more. An exemplary competition assay is provided herein.

The term "chimeric" antibody refers to an antibody in which a portion of the heavy and/or light chain is derived from a particular source or species, while the remainder of the heavy and/or light chain is derived from a different source or species.

The "class" of an antibody refers to the type of constant domain or constant region possessed by its heavy chain. There are five major classes of antibodies: IgA, IgD, IgE, IgG, and IgM, and several of these may be further divided into subclasses (isotypes), e.g., $IgG_1$, $IgG_2$, $IgG_3$, $IgG_4$, $IgA_1$, and $IgA_2$. The heavy chain constant domains that correspond to the different classes of immunoglobulins are called alpha, delta, epsilon, gamma, and mu, respectively.

The term "cytotoxic agent" as used herein refers to a substance that inhibits or prevents a cellular function and/or causes cell death or destruction. Cytotoxic agents include, but are not limited to, radioactive isotopes (e.g., $^{211}At$, $^{131}I$, $^{125}I$, $^{90}Y$, $^{186}Re$, $^{188}Re$, $^{153}Sm$, $^{212}Bi$, $^{32}P$, $^{212}Pb$ and radioactive isotopes of Lu); chemotherapeutic agents or drugs (e.g., methotrexate, adriamycin, vinca alkaloids (vincristine, vinblastine, etoposide), doxorubicin, melphalan, mitomycin C, chlorambucil, daunorubicin or other intercalating agents); growth inhibitory agents; enzymes and fragments thereof such as nucleolytic enzymes; antibiotics; toxins such as small molecule toxins or enzymatically active toxins of bacterial, fungal, plant or animal origin, including fragments and/or variants thereof; and the various antitumor or anticancer agents disclosed below.

"Effector functions" refer to those biological activities attributable to the Fc region of an antibody, which vary with the antibody isotype. Examples of antibody effector functions include: C1q binding and complement dependent cytotoxicity (CDC); Fc receptor binding; antibody-dependent cell-mediated cytotoxicity (ADCC); phagocytosis; down regulation of cell surface receptors (e.g. B cell receptor); and B cell activation.

An "effective amount" of an agent, e.g., a pharmaceutical formulation, refers to an amount effective, at dosages and for periods of time necessary, to achieve the desired therapeutic or prophylactic result.

The term "Fc region" herein is used to define a C-terminal region of an immunoglobulin heavy chain that contains at least a portion of the constant region. The term includes native sequence Fc regions and variant Fc regions. In one embodiment, a human IgG heavy chain Fc region extends from Cys226, or from Pro230, to the carboxyl-terminus of the heavy chain. However, the C-terminal lysine (Lys447) or glycine-lysine (residues 446-447) of the Fc region may or may not be present. Unless otherwise specified herein, numbering of amino acid residues in the Fc region or constant region is according to the EU numbering system, also called the EU index, as described in Kabat et al., Sequences of Proteins ofImmunological Interest, 5th Ed. Public Health Service, National Institutes of Health, Bethesda, MD, 1991.

Herein, amino acid alteration or substitution in the Fc region or constant region may be denoted by the EU numbering system and amino acid codes. For example, S424N denotes substitution of serine (Ser) at position 424 according to EU numbering with asparagine (Asn). Furthermore, EU424N denotes substitution of the amino acid at position 424 (which may be any amino acid) with asparagine (Asn).

The term "Fc region-comprising antibody" refers to an antibody that comprises an Fc region. The C-terminal lysine (residue 447 according to the EU numbering system) or C-terminal glycine-lysine (residues 446-447) of the Fc region may be removed, for example, during purification of the antibody or by recombinant engineering of the nucleic acid encoding the antibody. Accordingly, a composition comprising an antibody having an Fc region according to this disclosure can comprise an antibody with G446-K447, with G446 and without K447, with all G446-K447 removed, or a mixture of three types of antibodies described above.

"Framework" or "FR" refers to variable domain residues other than hypervariable region (HVR) residues. The FR of a variable domain generally consists of four FR domains: FR1, FR2, FR3, and FR4. Accordingly, the HVR and FR sequences generally appear in the following sequence in VH (or VL): FR1-H1(L1)-FR2-H2(L2)-FR3-H3(L3)-FR4.

The terms "full length antibody," "intact antibody," and "whole antibody" are used herein interchangeably to refer to an antibody having a structure substantially similar to a native antibody structure or having heavy chains that contain an Fc region as defined herein.

The terms "host cell," "host cell line," and "host cell culture" are used interchangeably and refer to cells into which exogenous nucleic acid has been introduced, including the progeny of such cells. Host cells include "transformants" and "transformed cells," which include the primary transformed cell and progeny derived therefrom without regard to the number of passages. Progeny may not be completely identical in nucleic acid content to a parent cell, but may contain mutations. Mutant progeny that have the same function or biological activity as screened or selected for in the originally transformed cell are included herein.

A "human antibody" is one which possesses an amino acid sequence which corresponds to that of an antibody produced by a human or a human cell or derived from a non-human source that utilizes human antibody repertoires or other human antibody-encoding sequences. This definition of a human antibody specifically excludes a humanized antibody comprising non-human antigen-binding residues.

A "human consensus framework" is a framework which represents the most commonly occurring amino acid residues in a selection of human immunoglobulin VL or VH framework sequences. Generally, the selection of human immunoglobulin VL or VH sequences is from a subgroup of variable domain sequences. Generally, the subgroup of sequences is a subgroup as in Kabat et al., Sequences of Proteins of Immunological Interest, Fifth Edition, NIH Publication 91-3242, Bethesda MD (1991), vols. 1-3. In one embodiment, for the VL, the subgroup is subgroup kappa I as in Kabat et al., *supra.* In one embodiment, for the VH, the subgroup is subgroup III as in Kabat et al., *supra.*

A "humanized" antibody refers to a chimeric antibody comprising amino acid residues from non-human HVRs and amino acid residues from human FRs. In certain embodiments, a humanized antibody will comprise substantially all of at least one, and typically two, variable domains, in which all or substantially all of the HVRs (e.g., CDRs) correspond to those of a non-human antibody, and all or substantially all of the FRs correspond to those of a human antibody. A humanized antibody optionally may comprise at least a portion of an antibody constant region derived from a human antibody. A "humanized form" of an antibody, e.g., a non-human antibody, refers to an antibody that has undergone humanization.

The term "hypervariable region" or "HVR" as used herein refers to each of the regions of an antibody variable domain which are hypervariable in sequence ("complementarity determining regions" or "CDRs") and/or form structurally defined loops ("hypervariable loops") and/or contain the antigen-contacting residues ("antigen contacts"). Generally, antibodies comprise six HVRs: three in the VH (HI, H2, H3), and three in the VL (L1, L2, L3). Exemplary HVRs herein include:

(a) hypervariable loops occurring at amino acid residues 26-32 (L1), 50-52 (L2), 91-96 (L3), 26-32 (HI), 53-55 (H2), and 96-101 (H3) (Chothia and Lesk, J. Mol. Biol. 196:901-917 (1987));

(b) CDRs occurring at amino acid residues 24-34 (L1), 50-56 (L2), 89-97 (L3), 31-35b (HI), 50-65 (H2), and 95-102 (H3) (Kabat et al., Sequences of Proteins ofImmunological Interest, 5th Ed. Public Health Service, National Institutes of Health, Bethesda, MD (1991));
(c) antigen contacts occurring at amino acid residues 27c-36 (L1), 46-55 (L2), 89-96 (L3), 30-35b (HI), 47-58 (H2), and 93-101 (H3) (MacCallum et al. J. Mol. Biol. 262: 732-745 (1996)); and
(d) combinations of (a), (b), and/or (c), including HVR amino acid residues 46-56 (L2), 47-56 (L2), 48-56 (L2), 49-56 (L2), 26-35 (HI), 26-35b (HI), 49-65 (H2), 93-102 (H3), and 94-102 (H3).

Unless otherwise indicated, HVR residues and other residues in the variable domain (e.g., FR residues) are numbered herein according to Kabat et al., *supra.*

An "immunoconjugate" is an antibody conjugated to one or more heterologous molecule(s), including but not limited to a cytotoxic agent.

An "individual" or "subject" is a mammal. Mammals include, but are not limited to, domesticated animals (e.g., cows, sheep, cats, dogs, and horses), primates (e.g., humans and non-human primates such as monkeys), rabbits, and rodents (e.g., mice and rats). In certain embodiments, the individual or subject is a human.

An "isolated" antibody is one which has been separated from a component of its natural environment. In some embodiments, an antibody is purified to greater than 95% or 99% purity as determined by, for example, electrophoretic (e.g., SDS-PAGE, isoelectric focusing (IEF), capillary electrophoresis) or chromatographic (e.g., ion exchange or reverse phase HPLC). For review of methods for assessment of antibody purity, see, e.g., Flatman et al., J. Chromatogr. B 848:79-87 (2007).

An "isolated" nucleic acid refers to a nucleic acid molecule that has been separated from a component of its natural environment. An isolated nucleic acid includes a nucleic acid molecule contained in cells that ordinarily contain the nucleic acid molecule, but the nucleic acid molecule is present extrachromosomally or at a chromosomal location that is different from its natural chromosomal location.

"Isolated nucleic acid encoding an antigen-binding molecule comprising a TRIM21-binding domain" refers to one or more nucleic acid molecules encoding the antigen-binding molecule, including such nucleic acid molecule(s) in a single vector or separate vectors, and such nucleic acid molecule(s) present at one or more locations in a host cell. When the antigen-binding molecule comprising a TRIM21-binding domain is an antibody, the "isolated nucleic acid encoding an antigen-binding molecule comprising a TRIM21-binding domain" refers to one or more nucleic acid molecules encoding heavy and light chains (or fragments thereof) of the antibody, including such nucleic acid molecule(s) in a single vector or separate vectors, and such nucleic acid molecule(s) present at one or more locations in a host cell.

The term "monoclonal antibody" as used herein refers to an antibody obtained from a population of substantially homogeneous antibodies, i.e., the individual antibodies composing the population are identical and/or bind the same epitope, except for possible variant antibodies, e.g., containing naturally occurring mutations or arising during production of a monoclonal antibody preparation, such variants generally being present in minor amounts. In contrast to polyclonal antibody preparations, which typically include different antibodies directed against different determinants (epitopes), each monoclonal antibody of a monoclonal antibody preparation is directed against a single determinant on an antigen. Thus, the modifier "monoclonal" indicates the character of the antibody as being obtained from a substantially homogeneous population of antibodies, and is not to be construed as requiring production of the antibody by any particular method. For example, the monoclonal antibodies to be used in accordance with the present disclosure may be made by a variety of techniques, including but not limited to the hybridoma method, recombinant DNA methods, phage-display methods, and methods utilizing transgenic animals containing all or part of the human immunoglobulin loci, such methods and other exemplary methods for making monoclonal antibodies being described herein.

A "naked antibody" refers to an antibody that is not conjugated to a heterologous moiety (e.g., a cytotoxic moiety) or radiolabel. The naked antibody may be present in a pharmaceutical formulation.

"Native antibodies" refer to naturally occurring immunoglobulin molecules with varying structures. For example, native IgG antibodies are heterotetrameric glycoproteins of about 150,000 daltons, composed of two identical light chains and two identical heavy chains that are disulfide-bonded. From N- to C-terminus, each heavy chain has a variable region (VH), also called a variable heavy domain or a heavy chain variable domain, followed by three constant domains (CH1, CH2, and CH3). Similarly, from N- to C-terminus, each light chain has a variable region (VL), also called a variable light domain or a light chain variable domain, followed by a constant light (CL) domain. The light chain of an antibody may be assigned to one of two types, called kappa ($\kappa$) and lambda ($\lambda$), based on the amino acid sequence of its constant domain.

The term "cytosol-penetrating antigen-binding molecule" refers to an antigen-binding molecule that can penetrate into the cytosol of a living cell. The mode for penetration into the cytosol is not particularly limited, and may be through incorporation via the endocytosis process followed by endosome escape, or through other processes. Examples other than endosome escape include penetration of the antigen-binding molecule directly through the cell membrane. One single antigen-binding molecule may penetrate into the cytosol both by endosome escape and by direct penetration through the cell membrane. Antigen-binding molecules capable of penetrating into the cytosol via methods other than endosome escape have been reported (e.g., WO 2013102659). In one embodiment, a cytosol-penetrating antigen-binding molecule is an antibody or antibody derivative. In another embodiment, a cytosol-penetrating antigen-binding molecule comprises a cytosol-penetrating domain.

The term "cytosol-penetrating domain" refers to a domain that can penetrate into the cytosol of a living cell. The mode for penetration into the cytosol is not particularly limited, and may be through incorporation via the endocytosis process followed by endosome escape, or through other processes. Examples other than endosome escape include penetration of the antigen-binding molecule directly through the cell membrane. One single antigen-binding molecule may penetrate into the cytosol both by endosome escape and by direct penetration through the cell membrane. The cytosol-penetrating domain may be of any type as long as it has a cytosol-penetrating ability, but in one preferred embodiment, the cytosol-penetrating domain is an antibody variable region (heavy chain variable region and/or light chain variable region), an antibody constant region, a peptide moiety, or a nucleic acid moiety. When the cytosol-penetrating domain is an antibody variable region or constant region, the cytosol-penetrating domain may be part or all of the variable region or constant region. Examples of the peptide moiety as a cytosol-penetrating domain include cell-penetrating peptides (CPP), protein transduction domains (PTD), etc. (see, for example, WO 2017156630). Examples of the nucleic acid moiety as a cytosol-penetrating domain include oligo-nucleic acids having a phosphorothioate backbone (see, for example, WO 2015031837). In one embodiment, a cytosol-penetrating antigen-binding molecule is a fusion or a complex of an antigen-binding molecule having no cytosol-penetrating ability with a cytosol-penetrating domain.

The term "package insert" is used to refer to instructions customarily included in commercial packages of therapeutic products, that contain information about the indications, usage, dosage, administration, combination therapy, contraindications and/or warnings concerning the use of such therapeutic products.

"Percent (%) amino acid sequence identity" with respect to a reference polypeptide sequence is defined as the percentage of amino acid residues in a candidate sequence that are identical with the amino acid residues in the reference polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN, Megalign (DNASTAR) software, or GENETYX® (Genetyx Co., Ltd.). Those skilled in the art can determine appropriate parameters for aligning sequences, including any algorithms needed to achieve maximal alignment over the full length of the sequences being compared.

The ALIGN-2 sequence comparison computer program was authored by Genentech, Inc., and the source code has been filed with user documentation in the U.S. Copyright Office, Washington D.C., 20559, where it is registered under U.S. Copyright Registration No. TXU510087. The ALIGN-2 program is publicly available from Genentech, Inc., South San Francisco, California, or may be compiled from the source code. The ALIGN-2 program should be compiled for use on a UNIX operating system, including digital UNIX V4.0D. All sequence comparison parameters are set by the ALIGN-2 program and do not vary. In situations where ALIGN-2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:

$$100 \text{ times the fraction } X/Y$$

where X is the number of amino acid residues scored as identical matches by the sequence alignment program ALIGN-2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A. Unless specifically stated otherwise, all % amino acid sequence identity values used herein are obtained as described in the immediately preceding paragraph using the ALIGN-2 computer program.

The term "pharmaceutical formulation" refers to a preparation which is in such form as to permit the biological activity of an active ingredient contained therein to be effective, and which contains no additional components which are unacceptably toxic to a subject to which the formulation would be administered.

A "pharmaceutically acceptable carrier" refers to an ingredient in a pharmaceutical formulation, other than an active ingredient, which is nontoxic to a subject. A pharmaceutically acceptable carrier includes, but is not limited to, a buffer, excipient, stabilizer, or preservative.

The term "TRIM21" refers to a protein having E3 ubiquitin ligase activity which is also known as tripartite motif-containing protein 21, E3 ubiquitin-protein ligase TRIM21, or Ro52. TRIM21 has RING, B-Box, and coiled-coil domains at the N-terminal side, and is a member of the tripartite motif (TRIM) family, which have these three domains in common.

The C-terminal B30.2 domain (PRYSPRY domain) of TRIM21 can bind to the CH2-CH3 domain of the antibody Fc domain, in particular, the CH2-CH3 domain interface. It has been reported that TRIM21 binds with a high affinity to the Fc domain of an antibody that has bound to a pathogen and been incorporated into the cytosol, and recruits the ubiquitin-proteasome system, thereby degrading the pathogen (RNA/DNA virus or bacteria) bound to the antibody. TRIM21 has also been reported to function as a detection system for intracellular infection as it binds to the Fc region of an antibody incorporated into the cytosol and activates natural immune responses via NFκB signaling and IFN signaling.

The term "TRIM21," as used herein, refers to any native TRIM21 from any vertebrate source, including mammals such as primates (e.g. humans) and rodents (e.g., mice and rats), unless otherwise indicated. The term encompasses "full-length" unprocessed TRIM21 as well as any form of TRIM21 that results from processing in the cell. The term also encompasses naturally occurring variants of TRIM21, e.g., splice variants or allelic variants. The amino acid sequence and nucleic acid sequence of an exemplary human TRIM21 is shown in SEQ ID NO: 1 (NCBI accession: NP_003132) and SEQ ID NO: 2 (NCBI accession: NM_003141), respectively. The amino acid sequence and nucleic acid sequence of an exemplary mouse TRIM21 is shown in SEQ ID NO: 3 (GenBank accession: CAJ18544) and SEQ ID NO: 4 (GenBank accession: CT010336), respectively. In addition, the amino acid sequence of an exemplary human TRIM21 PRYSPRY domain is shown in SEQ ID NO: 5, and the amino acid sequence of an exemplary mouse TRIM21 PRYSPRY domain is shown in SEQ ID NO: 6.

As used herein, "treatment" (and grammatical variations thereof such as "treat" or "treating") refers to clinical intervention in an attempt to alter the natural course of the individual being treated, and can be performed either for prophylaxis or during the course of clinical pathology. Desirable effects of treatment include, but are not limited to, preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. In some embodiments, antigen-binding molecules of the disclosure are used to delay development of a disease or to slow the progression of a disease.

The term "variable region" or "variable domain" refers to the domain of an antibody heavy or light chain that is involved in binding the antibody to antigen. The variable domains of the heavy chain and light chain (VH and VL, respectively) of a native antibody generally have similar structures, with each domain comprising four conserved framework regions (FRs) and three hypervariable regions (HVRs). (See, e.g., Kindt et al. Kuby Immunology, 6th ed., W.H. Freeman and Co., page 91 (2007).) A single VH or VL domain may be sufficient to confer antigen-binding specificity. Furthermore, antibodies that bind a particular antigen may be isolated using a VH or VL domain from an antibody that binds the antigen to screen a library of complementary VL or VH domains, respectively. See, e.g., Portolano et al., J. Immunol. 150:880-887 (1993); Clarkson et al., Nature 352:624-628 (1991).

The term "vector," as used herein, refers to a nucleic acid molecule capable of propagating another nucleic acid to which it is linked. The term includes the vector as a self-replicating nucleic acid structure as well as the vector incorporated into the genome of a host cell into which it has been introduced. Certain vectors are capable of directing the expression of nucleic acids to which they are operatively linked. Such vectors are referred to herein as "expression vectors."

The phrase "substantially reduced", "substantially increased", or "substantially different," as used herein, refers to a sufficiently high degree of difference between two numeric values (generally one associated with a molecule and the other associated with a reference/comparator molecule) such that one of skill in the art would consider the difference between the two values to be of statistical significance within the context of the biological characteristic measured by said values (e.g., KD values).

The term "substantially similar", "substantially unchanged", or "substantially the same," as used herein, refers to a sufficiently high degree of similarity between two numeric values (for example, one associated with an antigen-binding molecule of the disclosure and the other associated with a reference/comparator antigen-binding molecule), such that one of skill in the art would consider the difference between the two values to be of little or no biological and/or statistical significance within the context of the biological characteristic measured by said values (*e.g.,* KD values).

## II. **COMPOSITIONS AND METHODS**

In one aspect, the present disclosure is based, in part, on the discovery that alteration of the TRIM21-binding domain of an antigen-binding molecule has an effect on the half-life of the antigen-binding molecule in the cytosol. In certain embodiments, antigen-binding molecules that contain an altered TRIM21-binding domain are provided. Antigen-binding molecules of the present disclosure are useful, e.g., for the diagnosis or treatment of diseases resulting from cytosolic antigens.

### A. Antigen-binding molecules comprising an altered TRIM21-binding domain

In one aspect, the present disclosure provides an antigen-binding molecule comprising an altered TRIM21-binding domain wherein the antigen-binding molecule has an increased or decreased cytosolic half-life compared to a parent antigen-binding molecule. In one embodiment, the altered TRIM21-binding domain comprises one or more amino acid alterations that result in a decrease or an increase in binding affinity for TRIM21 compared to a wildtype TRIM21-binding domain. In one embodiment, the altered TRIM21-binding domain comprises an amino acid substitution in the TRIM21-binding domain at one or more positions selected from the group consisting of EU253, EU309, EU311, EU312, EU314, EU315, EU345, EU428, EU432, EU433, EU434, EU435, EU436, EU437, EU438, EU439, and EU440. Antigen-binding molecules of the present disclosure may contain a substitution at additional positions.

The altered TRIM21-binding domain of the present disclosure may contain substitutions at two or more positions In the present disclosure, this is referred to as a "combination" of substitutions. For example, a TRIM21-binding domain specified by the combination "EU424/EU434/EU436" is a TRIM21-binding domain comprising substitutions at positions EU424, EU434, and EU436. In one embodiment, an altered TRIM21-binding domain contains at least one combination of amino acid substitutions listed in Table 1-1. Specific examples of these combinations of amino acid substitutions are illustrated in Tables 5 and 6.

[Table 1-1]

| | |
|---|---|
| 1 | EU253/EU312 |
| 2 | EU253/EU315 |
| 3 | EU253/EU428 |
| 4 | EU253/EU432 |
| 5 | EU253/EU436 |
| 6 | EU253/EU437 |
| 7 | EU253/EU438 |

The term "TRIM21-binding domain" used herein means a protein domain that binds directly or indirectly to TRIM21. In one embodiment, TRIM21 is mammalian TRIM21, and preferably human TRIM21. In a more preferred embodiment, a TRIM21-binding domain binds to both human TRIM21 and mouse TRIM21. An example of a TRIM21-binding domain that directly binds to TRIM21 is an antibody Fc region. On the other hand, a region that may bind to a polypeptide having human TRIM21-binding activity such as IgG can bind indirectly to human TRIM21 through IgG or such. Therefore, such a human TRIM21-binding domain may be a region that binds to a polypeptide having human TRIM21-binding activity.

A TRIM21-binding domain that directly binds to TRIM21 in one embodiment, mammalian TRIM21 in a preferred embodiment, or a human TRIM21 in a more preferred embodiment, is an Fc region or an Fc region of an antigen-binding molecule. Specifically, the TRIM21-binding domain is an antibody Fc region. In a preferred embodiment, the TRIM21-binding domain is a mammalian Fc region, and in a more preferred embodiment, it is a human Fc region. Specifically, the TRIM21-binding domain of the present disclosure is an Fc region that contains the second and third constant domains (CH2 and CH3), and in a more preferred embodiment, contains the hinge, CH2, and CH3, of a human immunoglobulin. In a preferred embodiment, the immunoglobulin is IgG. In a preferred embodiment, the TRIM21-binding domain is a human IgG1 Fc region.

"An increase in binding affinity for TRIM21" refers to an increase compared to the binding affinity of a parent antigen-binding molecule for TRIM21. "A decrease in binding affinity for TRIM21" refers to a decrease compared to the binding affinity of a parent antigen-binding molecule for TRIM21.

The term "parent antigen-binding molecule" as used herein refers to an antigen-binding molecule which does not contain the amino acid alterations of the present disclosure, that is, one or more amino acid alterations that result in a decrease or an increase in binding affinity for TRIM21, but is identical in the rest of the molecule to an antigen-binding molecule containing an altered TRIM21-binding domain. A parent antigen-binding molecule may be an antigen-binding molecule comprising a wildtype Fc region, or an antigen-binding molecule comprising an altered Fc region. In a preferred embodiment, the antigen-binding molecule is an antibody or an antibody derivative. The term "wildtype" refers to a naturally-occurring sequence.

When binding affinity is represented by dissociation constant (KD), "an increase in binding affinity for TRIM21" may be shown by the fact that the KD value of an antigen-binding molecule comprising an altered TRIM21-binding domain for binding to TRIM21 is smaller than the KD value of a parent antigen-binding molecule for binding to TRIM21. Furthermore, "a decrease in binding affinity for TRIM21" may be shown by the fact that the KD value of an antigen-binding molecule comprising an altered TRIM21-binding domain for binding to TRIM21 is larger than the KD value of a parent antigen-binding molecule for binding to TRIM21.

In measuring binding affinity for TRIM21, the measurement conditions can be appropriately selected by those skilled in the art, and are not particularly limited. For example, as described in the Examples of the present disclosure, measurement can be carried out in HBS-P buffer at 25°C, pH7.4. Furthermore, the binding affinity (KD) of an antigen-binding molecule or a TRIM21-binding domain for TRIM21 can be measured by a method known to those skilled in the art, for example, by using a BIACORE® surface plasmon resonance assay. The binding affinity of an antigen-binding molecule (or TRIM21-binding domain) for TRIM21 can be assayed, for example, by immobilizing the antigen-binding molecule (or TRIM21-binding domain) onto a chip and allowing TRIM21 to flow over the chip as an analyte.

TRIM21 binds to the Fc region of an antibody incorporated into the cytosol, and activates the innate immune response and inflammation via NFκB and IFN signals. It has been reported that the NF-κB signaling activated by an anti-human adenovirus type 5 antibody containing an Fc with decreased binding affinity for TRIM21 is decreased compared to the NF-κB signaling activated by an anti-human adenovirus type 5 antibody containing a wildtype Fc (Nat Immunol. 2013 Apr;14(4):327-36). Therefore, in one embodiment, an antigen-binding molecule comprising an altered TRIM21-binding domain which comprises an amino acid alteration that results in a decrease in binding affinity for TRIM21, has a decreased ability to activate NF-κB signaling compared to the parent antigen-binding molecule. When an antigen-binding molecule comprising an altered TRIM21-binding domain provided by the present disclosure is used in a therapeutic composition, the antigen-binding molecule with a decreased ability to activate NFκB signaling can be expected to cause no or reduced inflammatory responses as a side effect.

B. Antigen-binding molecules comprising an altered TRIM21-binding domain and having an increased cytosolic half-life

In one aspect, the present disclosure provides an antigen-binding molecule comprising an altered TRIM21-binding domain, which has an increased cytosolic half-life compared to a parent antigen-binding molecule. When the antigen-binding molecule comprising an altered TRIM21-binding domain comprises a cytosolic antigen-binding domain, the antigen-binding molecule can be expected to have a decreased ability to remove cytosolic antigens compared to the parent antigen-binding molecule. Alternatively, when the antigen-binding molecule comprising an altered TRIM21-binding domain is an agonistic antigen-binding molecule or an antagonistic antigen-binding molecule against a cytosolic antigen, the antigen-binding molecule can be expected to exhibit prolonged or enhanced agonistic activity or antagonistic activity compared to the parent antigen-binding molecule. In one embodiment, the altered TRIM21-binding domain comprises one or more amino acid alterations that result in an increase or a decrease in binding affinity for TRIM21 compared to the wildtype TRIM21-binding domain. In a preferred embodiment, the altered TRIM21-binding domain comprises one or more amino acid alterations that result in a decrease in binding affinity for human TRIM21 or mouse TRIM21 compared to the wildtype TRIM21-binding domain. In a more preferred embodiment, the altered TRIM21-binding domain comprises one or more amino acid alterations that result in a decrease in binding affinity for human TRIM21 and mouse TRIM21 compared to the wildtype TRIM21-binding domain.

An amino acid after substitution (an amino acid to substitute for an amino acid in a parent TRIM21-binding domain) may be any amino acid including, but not limited to, the group consisting of alanine (Ala, A), arginine (Arg, R), asparagine (Asn, N), aspartic acid (Asp, D), cysteine (Cys, C), glutamic acid (Glu, E), glutamine (Gln, Q), glycine (Gly, G), histidine (His, H), isoleucine (Ile, I), leucine (Leu, L), lysine (Lys, K), methionine (Met, M), phenylalanine (Phe, F), proline (Pro, P), serine (Ser, S), threonine (thr, T), tryptophan (trp, W), tyrosine (tyr, Y), and valine (Val, V), unless specifically mentioned herein.

In one embodiment, an antigen-binding molecule comprising an altered TRIM21-binding domain and having an increased cytosolic half-life contains an amino acid substitution in the TRIM21-binding domain at one or more positions selected from the group consisting of EU253, EU309, EU311, EU312, EU314, EU315, EU345, EU428, EU432, EU433, EU434, EU435, EU436, EU437, EU438, EU439, and EU440. In a preferred embodiment, an antigen-binding molecule of the present disclosure contains an amino acid substitution in the TRIM21-binding domain at one or more positions selected from the group consisting of EU253, EU314, EU315, EU428, EU432, EU433, EU434, EU435, EU436, and EU437. The amino acid after substitution may be any amino acid unless specifically mentioned herein.

In one embodiment, an antigen-binding molecule comprising an altered TRIM21-binding domain and having an increased cytosolic half-life contains one or more amino acid alterations that result in a decrease in binding affinity for TRIM21 compared to the wildtype TRIM21-binding domain. For such amino acid alterations, preferred amino acids after substitution at EU253, EU314, EU315, EU428, EU432, EU433, EU434, EU435, EU436, and EU437 are shown in Table 1-2. In a preferred embodiment, an antigen-binding molecule of the present disclosure contains at least one of the amino acid substitutions set forth in Table 1-2. In another embodiment, an antigen-binding molecule of the present disclosure may include a combination of amino acid alterations, and specific examples of such a combination of amino acid substitutions are provided in Tables 5 and 6.

[Table 1-2]

| Preferred amino acids after substitution | |
|---|---|
| Position (according to EU numbering scheme) | Amino acid after substitution |
| EU253 | Ala, Val |
| EU314 | Asp |
| EU315 | Asp |
| EU428 | Lys, Arg |
| EU432 | Asp, Trp, Tyr |
| EU433 | Ala, Asp, Glu, Phe, Gly, Ile, Lys, Leu, Met, Asn, Pro, Gln, Arg, Ser, Thr, Val, Trp, Tyr |
| EU434 | Ala, Asp, Glu, Phe, Gly, His, Ile, Lys, Met, Pro, Gln, Arg, Ser, Thr, Val, Trp, Tyr |
| EU435 | Asp, Glu, Ile |
| EU436 | Ala, Glu, His, Lys, Leu, Met, Asn, Gln, Arg, Val |
| EU437 | Pro |

In one embodiment, an antigen-binding molecule comprising an altered TRIM21-binding domain and having an increased cytosolic half-life contains one or more amino acid alterations that result in an increase in binding affinity for TRIM21 compared to the wildtype TRIM21-binding domain. An example of such an amino acid alteration is substitution at EU253 with Phe.

In one embodiment, an increase in the cytosolic half-life of an antigen-binding molecule comprising an altered TRIM21-binding domain means that a) the amount of the antigen-binding molecule comprising an altered FcRn-binding domain present within the cytosol of cells after the antigen-binding molecule is contacted with the cells is increased compared to b) the amount of a parent antigen-binding molecule present within the cytosol of cells after the parent antigen-binding molecule is contacted with the cells. The cells in the above a) and b) are derived from the same cell line. In a preferred embodiment, the cell line is a Hela cell line, CHO cell line, or HepG2 cell line. The amount of the antigen-binding molecule to be contacted with the cells may be decided arbitrarily, provided that the amount of the antigen-binding molecule in the above a) and the amount of the parent antigen-binding molecule in the above b) are the same. The antigen-binding molecule may be contacted with the cells by any method including incubation and microinjection.

In one embodiment, the amount of an antigen-binding molecule present within the cytosol is measured 0 hours, 0.25 hours, 0.5 hours, 1 hour, 1.5 hours, 2 hours, 2.5 hours, 3 hours, 3.5 hours, 4 hours, 4.5 hours, 5 hours, 5.5 hours, 6 hours, 6.5 hours, 7 hours, 7.5 hours, 8 hours, 8.5 hours, 9 hours, 9.5 hours, 10 hours, 11 hours, 12 hours, 13 hours, 14 hours, 15 hours, and/or 16 hours after contacting the antigen-binding molecule with cells. The amount of the antigen-binding molecule present within the cytosol may be measured only once or measured multiple times after contacting the antigen-binding molecule with cells. Measuring the amount of the antigen-binding molecule present within the cytosol multiple times enables observation of changes in the amount of the antigen-binding molecule present within the cytosol over time.

For example, the amount of each antigen-binding molecule within the cytosol can be measured at 0 hours, 0.5 hours, 1 hour, 2 hours, and 2 hours, or at 2 hours and 4 hours, after the antigen-binding molecule is contacted with cells, to compare a) the amount of the antigen-binding molecule comprising an altered TRIM21-binding domain present within the cytosol with b) the amount of the parent antigen-binding molecule present within the cytosol at each time point. In this case, the cytosolic half-life of the antigen-binding molecule comprising an altered TRIM21-binding domain is shown to be increased if a) the amount of the antigen-binding molecule comprising an altered TRIM21-binding domain present within the cytosol is greater than b) the amount of the parent antigen-binding molecule present within the cytosol at an arbitrary time point, or if a) the time taken for the antigen-binding molecule comprising an altered TRIM21-binding domain present within the cytosol to become undetectable after the antigen-binding molecule is contacted with the cells is longer than b) the time taken for the parent antigen-binding molecule present within the cytosol to become undetectable after the antigen-binding molecule is contacted with the cells.

In a preferred embodiment, a) the amount of an antigen-binding molecule comprising an altered TRIM21-binding domain present within the cytosol of cells at an arbitrary time point after the antigen-binding molecule is contacted with the cells is at least 1.1 times, at least 1.2 times, at least 1.3 times, at least 1.4 times, at least 1.5 times, at least 1.6 times, at least 1.7 times, at least 1.8 times, at least 1.9 times, at least 2 times, at least 3 times, at least 4 times, at least 5 times, at least 6 times, at least 7 times, at least 8 times, at least 9 times, or at least 10 times greater than b) the amount of a parent antigen-binding molecule present within the cytosol of the cells at the same time point. In another preferred embodiment, the time taken for a) an antigen-binding molecule comprising an altered TRIM21-binding domain present within the cytosol to become undetectable after the antigen-binding molecule is contacted with the cells is at least 1.1 times, at least 1.2 times, at least 1.3 times, at least 1.4 times, at least 1.5 times, at least 1.6 times, at least 1.7 times, at least 1.8 times, at least 1.9 times, at least 2 times, at least 3 times, at least 4 times, at least 5 times, at least 6 times, at least 7 times, at least 8 times, at least 9 times, or at least 10 times longer than the time taken for b) a parent antigen-binding molecule present within the cytosol to become undetectable after the antigen-binding molecule is contacted with the cell.

In one embodiment, when the amount of the antigen-binding molecule present within the cytosol at an arbitrary time point is represented by HRP luminescence signal or fluorescence signal, a) the intensity of the HRP luminescence signal or the fluorescence signal at an arbitrary time point after the antigen-binding molecule comprising an altered TRIM21-binding domain is contacted with cells is at least 1.1 times, at least 1.2 times, at least 1.3 times, at least 1.4 times, at least 1.5 times, at least 1.6 times, at least 1.7 times, at least 1.8 times, at least 1.9 times, at least 2 times, at least 3 times, at least 4 times, at least 5 times, at least 6 times, at least 7 times, at least 8 times, at least 9 times, or at least 10 times greater than b) the intensity of the HRP luminescence signal or the fluorescence signal at the same time point as a) after the parent antigen-binding molecule is contacted with the cell. In another preferred embodiment, a) the time taken for the HRP luminescence signal or the fluorescence signal to become undetectable after the antigen-binding molecule comprising an altered TRIM21-binding domain is contacted with the cells is at least 1.1 times, at least 1.2 times, at least 1.3 times, at least 1.4 times, at least 1.5 times, at least 1.6 times, at least 1.7 times, at least 1.8 times, at least 1.9 times, at least 2 times, at least 3 times, at least 4 times, at least 5 times, at least 6 times, at least 7 times, at least 8 times, at least 9 times, or at least 10 times longer than b) the time taken for the HRP luminescence signal or the fluorescence signal to become undetectable after the parent antigen-binding molecule is contacted with the cells.

C. Antigen-binding molecules comprising an altered TRIM21-binding domain and having a decreased cytosolic half-life

In one aspect, the present disclosure provides an antigen-binding molecule comprising an altered TRIM21-binding domain, wherein the antigen-binding molecule has a decreased cytosolic half-life compared to a parent antigen-binding molecule. When the antigen-binding molecule comprising an altered TRIM21-binding domain comprises a cytosolic antigen-binding domain, the antigen-binding molecule can be expected to have an improved ability to remove cytosolic antigens compared to the parent antigen-binding molecule. In one embodiment, the altered TRIM21-binding domain comprises one or more amino acid alterations that result in an increase in binding affinity for TRIM21 compared to the wildtype TRIM21-binding domain. In a preferred embodiment, the altered TRIM21-binding domain comprises one or more amino acid alterations that result in an increase in binding affinity for human TRIM21 or mouse TRIM21 compared to the wildtype TRIM21-binding domain. In a more preferred embodiment, the altered TRIM21-binding domain comprises one or more amino acid alterations that result in an increase in binding affinity for human TRIM21 and mouse TRIM21 compared to the wildtype TRIM21-binding domain.

An amino acid after substitution (an amino acid to substitute for an amino acid in the parent TRIM21-binding domain) may be any amino acid including, but not limited to, the group consisting of alanine (Ala, A), arginine (Arg, R), asparagine (Asn, N), aspartic acid (Asp, D), cysteine (Cys, C), glutamic acid (Glu, E), glutamine (Gln, Q), glycine (Gly, G), histidine (His, H), isoleucine (Ile, I), leucine (Leu, L), lysine (Lys, K), methionine (Met, M), phenylalanine (Phe, F), proline (Pro, P), serine (Ser, S), threonine (thr, T), tryptophan (trp, W), tyrosine (tyr, Y), and valine (Val, V), unless specifically mentioned herein.

In one embodiment, an antigen-binding molecule comprising an altered TRIM21-binding domain and having a decreased cytosolic half-life comprises an amino acid substitution in the TRIM21-binding domain at one or more positions selected from the group consisting of EU253, EU309, EU311, EU312, EU314, EU315, EU345, EU428, EU432, EU433, EU434, EU435, EU436, EU437, EU438, EU439, and EU440. In a preferred embodiment, an antigen-binding molecule of the present disclosure includes an amino acid substitution in the TRIM21-binding domain at one or more positions selected from the group consisting of EU253, EU309, EU311, EU312, EU314, EU315, EU428, EU432, EU434, EU436, EU437, EU438, EU439, and EU440. The amino acid after substitution may be any amino acid unless specifically mentioned herein.

In one embodiment, a decrease in the cytosolic half-life of an antigen-binding molecule comprising an altered TRIM21-binding domain means that a) the amount of the antigen-binding molecule comprising an altered TRIM21-binding domain present within the cytosol of cells after the antigen-binding molecule is contacted with the cells is decreased compared to b) the amount of a parent antigen-binding molecule present within the cytosol of cells after the parent antigen-binding molecule is contacted with the cells. Here, the cells in the above a) and b) are derived from the same cell line. In a preferred embodiment, the cells are from a Hela cell line, CHO cell line, or HepG2 cell line. The amount of the antigen-binding molecule to be contacted with the cells may be decided arbitrarily, provided that the amount of the antigen-binding molecule in the above a) and the amount of the parent antigen-binding molecule in the above b) are the same. The antigen-binding molecule is contacted with the cells by any method including incubation and microinjection.

In one embodiment, the amount of an antigen-binding molecule present within the cytosol is measured 0 hours, 0.25 hours, 0.5 hours, 1 hour, 1.5 hours, 2 hours, 2.5 hours, 3 hours, 3.5 hours, 4 hours, 4.5 hours, 5 hours, 5.5 hours, 6 hours, 6.5 hours, 7 hours, 7.5 hours, 8 hours, 8.5 hours, 9 hours, 9.5 hours, 10 hours, 11 hours, 12 hours, 13 hours, 14 hours, 15 hours, and/or 16 hours after the antigen-binding molecule is contacted with the cells. The amount of the antigen-binding molecule present within the cytosol may be measured only once or measured multiple times after the antigen-binding molecule is contacted with the cells. Measuring the amount of the antigen-binding molecule present within the cytosol multiple times enables observation of changes in the amount of the antigen-binding molecule present within the cytosol over time.

For example, the amount of each antigen-binding molecule within the cytosol can be measured at 0 hours, 0.5 hours, 1 hour, 1.5 hours, and 2 hours after the antigen-binding molecule is contacted with cells to compare a) the amount of the antigen-binding molecule comprising an altered TRIM21-binding domain present within the cytosol with b) the amount of the parent antigen-binding molecule present within the cytosol at each time point. In this case, the cytosolic half-life of the antigen-binding molecule comprising an altered TRIM21-binding domain is shown to be increased if a) the amount of the antigen-binding molecule comprising an altered TRIM21-binding domain present within the cytosol is less than b) the amount of the parent antigen-binding molecule present within the cytosol at an arbitrary time point, or if the time taken for a) the antigen-binding molecule comprising an altered TRIM21-binding domain present within the cytosol to become undetectable after the antigen-binding molecule is contacted with the cells is shorter than the time taken for b) the parent antigen-binding molecule present within the cytosol to become undetectable after the antigen-binding molecule is contacted with the cells.

In a preferred embodiment, a) the amount of the antigen-binding molecule comprising an altered TRIM21-binding domain present within the cytosol of cells at an arbitrary time point after the antigen-binding molecule is contacted with the cells is 0.9 times or less, 0.8 times or less, 0.7 times or less, 0.6 times or less, 0.5 times or less, 0.4 times or less, or 0.3 times or less than b) the amount of the parent antigen-binding molecule present within the cytosol of the cells at the same time point. In another preferred embodiment, a) the time taken for the antigen-binding molecule comprising an altered TRIM21-binding domain present within the cytosol to become undetectable after the antigen-binding molecule is contacted with cells is 0.9 times or less, 0.8 times or less, 0.7 times or less, 0.6 times or less, 0.5 times or less, 0.4 times or less, or 0.3 times or less than b) the time taken for the parent antigen-binding molecule present within the cytosol to become undetectable after the antigen-binding molecule is contacted with cells.

When the amount of the antigen-binding molecule present within the cytosol at an arbitrary time point is represented by HRP luminescence signal or fluorescence signal, in a preferred embodiment, a) the intensity of the HRP luminescence signal or the fluorescence signal at an arbitrary time point after the antigen-binding molecule comprising an altered TRIM21-binding domain is contacted with cells is 0.9 times or less, 0.8 times or less, 0.7 times or less, 0.6 times or less, 0.5 times or less, 0.4 times or less, or 0.3 times or less than b) the intensity of the HRP luminescence signal or the fluorescence signal at the same time point as in a) after the parent antigen-binding molecule is contacted with cells. In another preferred embodiment, a) the time taken for the HRP luminescence signal or the fluorescence signal to become undetectable after the antigen-binding molecule comprising an altered TRIM21-binding domain is contacted with cells is 0.9 times or less, 0.8 times or less, 0.7 times or less, 0.6 times or less, 0.5 times or less, 0.4 times or less, or 0.3 times or less than b) the time taken for the HRP luminescence signal or the fluorescence signal to become undetectable after the parent antigen-binding molecule is contacted with cells.

D. FcRn-binding domain

Herein, an "Fc receptor" or "FcR" refers to a receptor that binds to the Fc region of an antibody. Fc receptors are proteins on the surface of immune cells such as natural killer cells, macrophages, neutrophils, and mast cells. Fc receptors bind to the Fc (crystalline fragment) region of antibodies adhered to infected cells or invasive pathogens, stimulate phagocytes or cytotoxic cells, and destroy microorganisms or infected cells by antibody-mediated phagocytosis or antibody-dependent cell-mediated cytotoxicity. In some embodiments, an FcR is one which binds an IgG antibody (a gamma receptor) and includes receptors of the Fc gamma RI, Fc gamma RII, and Fc gamma RIII subclasses, including allelic variants and alternatively spliced forms of those receptors. Fc gamma RII receptors include Fc gamma RIIA (an "activating receptor") and Fc gamma RIIB (an "inhibiting receptor"), which have similar amino acid sequences that differ primarily in the cytoplasmic domains thereof. Activating receptor Fc gamma RIIA contains an immunoreceptor tyrosine-based activation motif (ITAM) in its cytoplasmic domain. Inhibiting receptor Fc gamma RIIB contains an immunoreceptor tyrosine-based inhibition motif (ITIM) in its cytoplasmic domain. (see, *e.g.,* Daëron, Annu. Rev. Immunol. 15:203-234 (1997)). FcRs are reviewed, for example, in Ravetch and Kinet, Annu. Rev. Immunol 9:457-92 (1991); Capel et al., Immunomethods 4:25-34 (1994); and de Haas et al., J. Lab. Clin. Med. 126:330-41 (1995). Other FcRs, including those to be identified in the future, are encompassed by the term "FcR" herein.

The term "Fc receptor" or "FcR" also includes the neonatal receptor, FcRn, which is responsible for the transfer of maternal IgGs to the fetus (Guyer et al., J. Immunol. 117:587 (1976) and Kim et al., J. Immunol. 24:249 (1994)) and regulation of homeostasis of immunoglobulins. Methods of measuring binding to FcRn are known (see, *e.g.,* Ghetie and Ward., Immunol. Today 18(12):592-598 (1997); Ghetie et al., Nature Biotechnology, 15(7):637-640 (1997); Hinton et al., J. Biol. Chem. 279(8):6213-6216 (2004); WO 2004/92219 (Hinton *et al.*).

Binding to human FcRn *in vivo* and plasma half life of human FcRn high affinity binding polypeptides can be assayed, e.g., in transgenic mice or transfected human cell lines expressing human FcRn, or in primates to which the polypeptides with a variant Fc region are administered. WO 2000/42072 (Presta) describes antibody variants with increased or decreased binding to FcRs. See also, *e.g.,* Shields et al. J. Biol. Chem. 9(2):6591-6604 (2001).

The term "FcRn-binding domain" as used herein refers to a protein domain that directly or indirectly binds to FcRn. In a preferred embodiment, FcRn is mammalian FcRn, and in a more preferred embodiment, it is human FcRn. An example of an FcRn-binding domain that binds directly to FcRn is an antibody Fc region. Meanwhile, a region that may bind to a polypeptide having human FcRn-binding activity such as albumin or IgG, can indirectly bind to human FcRn via albumin, IgG, or such. Thus, such a human FcRn-binding region may be a region that binds to a polypeptide having human FcRn-binding activity.

In one embodiment, the FcRn-binding domain that directly binds to FcRn, mammalian FcRn in a preferred embodiment, or human FcRn in a more preferred embodiment, is an Fc region or an Fc region of an antigen-binding molecule. Specifically, the FcRn-binding domain is an antibody Fc region. In a preferred embodiment, the FcRn-binding domain is a mammalian Fc region, and in a more preferred embodiment, it is a human Fc region. Specifically, the FcRn-binding domain of the present disclosure is an Fc region that contains the second and third constant domains (CH2 and CH3), and in a more preferred embodiment, contains a hinge, CH2, and CH3, of a human immunoglobulin. In a preferred embodiment, the immunoglobulin is IgG. In a preferred embodiment, the FcRn-binding domain is a human IgG1 Fc region.

In one embodiment, the FcRn-binding domain may be a separate domain from the TRIM21-binding domain. In another embodiment, the FcRn-binding domain and the TRIM21-binding domain may completely or partially overlap each other. In a preferred embodiment, the FcRn-binding domain and the TRIM21-binding domain are the same Fc region, and in a more preferred embodiment, they are the same human Fc region.

In one embodiment, the antigen-binding molecule comprising an altered TRIM21-binding domain of the present disclosure has substantially the same FcRn-binding affinity compared to the parent antigen-binding molecule. In one embodiment, the antigen-binding molecule comprising an altered TRIM21-binding domain of the present disclosure has enhanced FcRn-binding affinity compared to the parent antigen-binding molecule. In another embodiment, the antigen-binding molecule comprising an altered TRIM21-binding domain of the present disclosure has decreased FcRn-binding affinity compared to the parent antigen-binding molecule.

In one embodiment, the antigen-binding molecule comprising an altered TRIM21-binding domain and having enhanced FcRn-binding affinity compared to the parent antigen-binding molecule comprises one or more amino acid alterations selected from the group consisting of the amino acid alterations included in Groups A, C, E, and G of Table 4(2). In another embodiment, the antigen-binding molecule comprising an altered TRIM21-binding domain and having decreased FcRn-binding affinity compared to the parent antigen-binding molecule comprises one or more amino acid alterations selected from the group consisting of the amino acid alterations included in Groups B, D, F, and H of Table 4(2).

E. Protein A-binding domain

The term "Protein A" as used herein is intended to refer to a 56 kDa MSCRAMM surface protein originally found in the cell wall of the bacterium *Staphylococcus aureus.* It is encoded by the spa gene and its regulation is controlled by DNA topology, cellular osmolarity, and a two-component system called ArlS-ArlR. It has found use in biochemical research because of its ability to bind immunoglobulins. It is composed of five homologous Ig-binding domains that fold into a three-helix bundle. Each domain is able to bind proteins from many mammalian species, most notably IgGs. It binds the heavy chain Fc region of most immunoglobulins (overlapping the conserved binding site of FcRn receptors) and also interacts with the Fab region of the human VH3 family. Through these interactions in serum, IgG molecules bind the bacteria via their Fc region instead of solely via their Fab regions, by which the bacteria disrupts opsonization, complement activation, and phagocytosis.

The term "Protein A-binding domain" as used herein refers to a protein domain that directly or indirectly binds to Protein A. An example of the Protein A-binding domain that directly binds to Protein A is an antibody Fc region. In a preferred embodiment, the Protein A-binding domain is a mammalian Fc region, and in a more preferred embodiment, it is a human Fc region. In one embodiment, the Protein A-binding domain overlaps with the FcRn-binding domain.

In one embodiment, the antigen-binding molecule comprising an altered TRIM21-binding domain of the present disclosure has substantially the same, enhanced, or decreased Protein A-binding affinity compared to the parent antigen-binding molecule. In a preferred embodiment, the antigen-binding molecule comprising an altered TRIM21-binding domain of the present disclosure has substantially the same Protein A-binding affinity compared to the parent antigen-binding molecule.

F. Stability

In the present disclosure, "stability" means, for example, the thermodynamic stability of an antigen-binding molecule, but is not limited thereto. The thermodynamic stability of an antigen-binding molecule can be evaluated and judged using, for example, the thermal denaturation midpoint (Tm) of the CH2 region as an index. That is, the stability of the antigen-binding molecule of the present invention is preferably evaluated or judged using the thermal denaturation midpoint (Tm) as an indicator. Tm can be measured by circular dichroism (CD), differential scanning calorimetry (DSC), and differential scanning fluorometry (DSF).

When an IgG sample is measured, the above-mentioned thermal stability evaluation method allows the thermal stability of its CH2, CH3, and Fab regions to be evaluated individually. For the Fc region, CH2 is less thermally stable than CH3, and therefore improving the thermal stability of CH2 may lead to improving the thermal stability of the Fc region.

In addition, IgG maintains a highly controlled three-dimensional structure, and its domains affect one another in terms of their respective three-dimensional structure and physical stability. That is, an alteration introduced into a certain domain may affect other domains, resulting in a change in the three-dimensional structure and physical stability of the whole IgG. For this reason, it is desired that the effect of introducing an alteration be evaluated on a molecule in the form of IgG. For the above reasons, in the present disclosure, the CH2 regions of altered antibodies prepared in the form of IgG were evaluated for thermal stability.

Altering the Fc region of an antibody is known to adversely affect the physical properties of the antibody. For example, an Fc region altered to have enhanced ADCC activity has been reported to have a thermal denaturation midpoint that is decreased by approximately 20°C (Biol. Crystallogr. 2008 Jun;64(Pt 6):700-4). In addition, an Fc region altered to have reduced ADCC activity has been reported to have a thermal denaturation midpoint that is decreased by approximately 5°C, and become susceptible to degradation by hydrolases and degradation under acidic conditions (Immunol. Lett. 2006 Aug, 106(2): 144-53; Pharm. Res. 2008 Aug, 25(8):1881-90; and Biochem. Biophys. Res. Commun. 2006 Mar, 341(3):797-803). Furthermore, there is also a report of an Fc region altered to improve blood retention that shows decreased thermal stability and storage stability (WO2007092772).

In one embodiment, the present disclosure provides antigen-binding molecules with stability that is not remarkably decreased, not substantially decreased (maintained), or improved compared to that of the parent antigen-binding molecule, even though they contain alterations in the TRIM21-binding domain. Preferably, the TRIM21-binding domain is an antibody Fc region.

In one embodiment, "the stability of an antigen-binding molecule comprising an altered TRIM21-binding domain is not decreased (is maintained)" means, for example, that the Tm of the TRIM21-binding domain of a test antigen-binding molecule determined based on the above-mentioned measurement method is the same as the Tm of the TRIM21-binding domain of a parent antigen-binding molecule serving as a control. In another embodiment, "the stability of an altered TRIM21-binding domain polypeptide is improved" means, for example, that the Tm of the TRIM21-binding domain of a test antigen-binding molecule determined based on the above-mentioned measurement method is higher than the Tm of the TRIM21-binding domain of a parent antigen-binding molecule serving as a control. For example, it refers to an increase of 0.1 degrees or more, preferably 0.2 degrees or more, 0.3 degrees or more, 0.4 degrees or more, 0.5 degrees or more, 1 degree or more, 2 degrees or more, 3 degrees or more, 4 degrees or more, 5 degrees or more, 10 degrees or more, or 20 degrees or more.

In one embodiment, an antigen-binding molecule comprising an altered TRIM21-binding domain and having stability that is not decreased (is maintained) or is improved compared to that of the parent antigen-binding molecule contains an amino acid alteration or a combination of amino acid alterations set forth in Group 1 of Table 5 (1) and (2).

G. Cytosol-penetrating antigen-binding molecule

In one aspect, an antigen-binding molecule in this disclosure has a cytosol-penetrating ability. In one embodiment, an antigen-binding molecule in this disclosure has a cytosol-penetrating domain. In a further embodiment, an antigen-binding molecule in the present disclosure has a cytosol-penetrating domain and a cytosolic antigen-binding domain. In a preferred embodiment, the cytosol-penetrating domain is a heavy chain variable region and/or a light chain variable region of antibody.

The term "antigen-binding domain", as used herein, refers to the portion of an antigen-binding molecule that comprises a region that specifically binds and is complementary to part or all of an antigen. When the antigen has a large molecular weight, the antigen-binding domain can only bind to a particular portion of the antigen. This particular portion is called an epitope. An antigen-binding domain may be provided from one or more antibody variable domains. In a preferred embodiment, the antigen-binding domain contains an antibody light chain variable region (VL), an antibody heavy chain variable region (VH), or a combination of an antibody light chain variable region (VL) and an antibody heavy chain variable region (VH). Preferable examples of such antigen-binding domains include "single-chain Fv (scFv)", "single-chain antibody", "Fv", "single-chain Fv2 (scFv2)", "Fab", and "F(ab')2".

The "cytosolic antigen-binding domain" in an antigen-binding molecule of the present disclosure can bind to an epitope present in an antigen expressed in the cytosol. A "cytosolic antigen" refers to an antigen structure that is expressed by cells and present within the cytosol. A cytosolic antigen may be a protein or nucleic acid such as DNA and RNA. When an antigen-binding molecule of the present disclosure comprises a cytosolic antigen-binding domain, the antigen-binding molecule binds to the cytosolic antigen within the cytosol, and can neutralize, inhibit, or activate the function of the antigen.

H. Antibody

In a further aspect of the invention, an antigen-binding molecule according to any of the above embodiments is an antibody, and in a preferred embodiment, it is a monoclonal antibody including a chimeric, humanized or human antibody. In one embodiment, an antigen-binding molecule is an antibody fragment, e.g., a Fv, Fab, Fab', scFv, diabody, or $F(ab')_2$ fragment, as long as it comprises a TRIM21-binding domain. In another embodiment, the antibody is a full length antibody, e.g., an intact IgG1 antibody or other antibody class or isotype as defined herein.

In a further aspect, an antibody comprising an altered TRIM21-binding domain according to any of the above embodiments may incorporate any of the features, singly or in combination, as described in Sections 1-7 below:

### *1. Antibody Fragments*

In certain embodiments, an antibody provided herein is an antibody fragment. Antibody fragments include, but are not limited to, Fab, Fab', Fab'-SH, $F(ab')_2$, Fv, and scFv fragments, and other fragments described below. For a review of certain antibody fragments, see Hudson et al. Nat. Med. 9:129-134 (2003). For a review of scFv fragments, see, e.g., Pluckthün, in The Pharmacology of Monoclonal Antibodies, vol. 113, Rosenburg and Moore eds., (Springer-Verlag, New York), pp. 269-315 (1994); see also WO 93/16185; and U.S. Patent Nos. 5,571,894 and 5,587,458. For discussion of Fab and $F(ab')_2$ fragments comprising salvage receptor binding epitope residues and having increased in vivo half-life, see U.S. Patent No. 5,869,046.

Diabodies are antibody fragments with two antigen-binding sites that may be bivalent or bispecific. See, for example, EP 404,097; WO 1993/01161; Hudson et al., Nat. Med. 9:129-134 (2003); and Hollinger et al., Proc. Natl. Acad. Sci. USA 90: 6444-6448 (1993). Triabodies and tetrabodies are also described in Hudson et al., Nat. Med. 9:129-134 (2003).

Single-domain antibodies are antibody fragments comprising all or a portion of the heavy chain variable domain or all or a portion of the light chain variable domain of an antibody. In certain embodiments, a single-domain antibody is a human single-domain antibody (Domantis, Inc., Waltham, MA; *see,* e.g., U.S. Patent No. 6,248,516 B1).

Antibody fragments can be made by various techniques, including but not limited to proteolytic digestion of an intact antibody as well as production by recombinant host cells (e.g. E. *coli* or phage), as described herein.

### *2. Chimeric and Humanized Antibodies*

In certain embodiments, an antibody provided herein is a chimeric antibody. Certain chimeric antibodies are described, e.g., in U.S. Patent No. 4,816,567; and Morrison et al., Proc. Natl. Acad. Sci. USA, 81:6851-6855 (1984)). In one example, a chimeric antibody comprises a non-human variable region (e.g., a variable region derived from a mouse, rat, hamster, rabbit, or non-human primate, such as a monkey) and a human constant region. In a further example, a chimeric antibody is a "class switched" antibody in which the class or subclass has been changed from that of the parent antibody. Chimeric antibodies include antigen-binding fragments thereof.

In certain embodiments, a chimeric antibody is a humanized antibody. Typically, a non-human antibody is humanized to reduce immunogenicity to humans, while retaining the specificity and affinity of the parental non-human antibody. Generally, a humanized antibody comprises one or more variable domains in which HVRs, e.g., CDRs, (or portions thereof) are derived from a non-human antibody, and FRs (or portions thereof) are derived from human antibody sequences. A humanized antibody optionally will also comprise at least a portion of a human constant region. In some embodiments, some FR residues in a humanized antibody are substituted with corresponding residues from a non-human antibody (e.g., the antibody from which the HVR residues are derived), e.g., to restore or improve antibody specificity or affinity.

Humanized antibodies and methods of making them are reviewed, e.g., in Almagro and Fransson, Front. Biosci.

13:1619-1633 (2008), and are further described, e.g., in Riechmann et al., Nature 332:323-329 (1988); Queen et al., Proc. Nat'l Acad. Sci. USA 86:10029-10033 (1989); US Patent Nos. 5, 821,337, 7,527,791, 6,982,321, and 7,087,409; Kashmiri et al., Methods 36:25-34 (2005) (describing specificity determining region (SDR) grafting); Padlan, Mol. Immunol. 28:489-498 (1991) (describing "resurfacing"); Dall'Acqua et al., Methods 36:43-60 (2005) (describing "FR shuffling"); and Osbourn et al., Methods 36:61-68 (2005) and Klimka et al., Br. J. Cancer, 83:252-260 (2000) (describing the "guided selection" approach to FR shuffling).

### *3. Human Antibodies*

In certain embodiments, an antibody provided herein is a human antibody. Human antibodies can be produced using various techniques known in the art. Human antibodies are described generally in van Dijk and van de Winkel, Curr. Opin. Pharmacol. 5: 368-74 (2001) and Lonberg, Curr. Opin. Immunol. 20:450-459 (2008).

### *4. Library-Derived Antibodies*

Antibodies of the disclosure may be isolated by screening combinatorial libraries for antibodies with the desired activity or activities. For example, a variety of methods are known in the art for generating phage display libraries and screening such libraries for antibodies possessing the desired binding characteristics. Such methods are reviewed, e.g., in Hoogenboom et al. in Methods in Molecular Biology 178:1-37 (O'Brien et al., ed., Human Press, Totowa, NJ, 2001) and further described, e.g., in the McCafferty et al., Nature 348:552-554; Clackson et al., Nature 352: 624-628 (1991); Marks et al., J. Mol. Biol. 222: 581-597 (1992); Marks and Bradbury, in Methods in Molecular Biology 248:161-175 (Lo, ed., Human Press, Totowa, NJ, 2003); Sidhu et al., J. Mol. Biol. 338(2): 299-310 (2004); Lee et al., J. Mol. Biol. 340(5): 1073-1093 (2004); Fellouse, Proc. Natl. Acad. Sci. USA 101(34): 12467-12472 (2004); and Lee et al., J. Immunol. Methods 284(1-2): 119-132(2004).

### *5. Multispecific Antibodies*

In certain embodiments, an antibody provided herein is a multispecific antibody, e.g. a bispecific antibody. Multispecific antibodies are monoclonal antibodies that have binding specificities for at least two different sites. Bispecific antibodies can be prepared as full length antibodies or antibody fragments.
Engineered antibodies with three or more functional antigen binding sites, including "Octopus antibodies," are also included herein (see, e.g. US 2006/0025576A1).
The antibody or fragment herein also includes a "Dual Acting Fab" or "DAF" (see, US 2008/0069820, for example).

### *6. Antibody Variants*

In certain embodiments, amino acid sequence variants of the antibodies provided herein are contemplated. For example, it may be desirable to improve the binding affinity and/or other biological properties of the antibody. Amino acid sequence variants of an antibody may be prepared by introducing appropriate modifications into the nucleotide sequence encoding the antibody, or by peptide synthesis. Such modifications include, for example, deletions from, and/or insertions into and/or substitutions of residues within the amino acid sequences of the antibody. Any combination of deletion, insertion, and substitution can be made to arrive at the final construct, provided that the final construct possesses the desired characteristics, e.g., antigen-binding.

#### a) Substitution, Insertion, and Deletion Variants

In certain embodiments, antibody variants having one or more amino acid substitutions are provided. Sites of interest for substitutional mutagenesis include the HVRs and FRs. Conservative substitutions are shown in Table 2 under the heading of "preferred substitutions." More substantial changes are provided in Table 2 under the heading of "exemplary substitutions," and as further described below in reference to amino acid side chain classes. Amino acid substitutions may be introduced into an antibody of interest and the products screened for a desired activity, e.g., retained/improved antigen binding, decreased immunogenicity, or improved ADCC or CDC.

[Table 2]

| Original Residue | Exemplary Substitutions | Preferred Substitutions |
|---|---|---|
| Ala (A) | Val; Leu; Ile | Val |

(continued)

| Original Residue | Exemplary Substitutions | Preferred Substitutions |
|---|---|---|
| Arg (R) | Lys; Gln; Asn | Lys |
| Asn (N) | Gln; His; Asp, Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |
| His (H) | Asn; Gln; Lys; Arg | Arg |
| Ile (I) | Leu; Val; Met; Ala; Phe; Norleucine | Leu |
| Leu (L) | Norleucine; Ile; Val; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Norleucine | Leu |

Amino acids may be grouped according to common side-chain properties:

(1) hydrophobic: Norleucine, Met, Ala, Val, Leu, Ile;

(2) neutral hydrophilic: Cys, Ser, Thr, Asn, Gln;

(3) acidic: Asp, Glu;

(4) basic: His, Lys, Arg;

(5) residues that influence chain orientation: Gly, Pro;

(6) aromatic: Trp, Tyr, Phe.

Non-conservative substitutions will entail exchanging a member of one of these classes for another class.

One type of substitutional variant involves substituting one or more hypervariable region residues of a parent antibody (*e.g.* a humanized or human antibody). Generally, the resulting variant(s) selected for further study will have modifications (e.g., improvements) in certain biological properties (e.g., increased affinity, reduced immunogenicity) relative to the parent antibody and/or will have substantially retained certain biological properties of the parent antibody. An exemplary substitutional variant is an affinity matured antibody, which may be conveniently generated, e.g., using phage display-based affinity maturation techniques such as those described herein. Briefly, one or more HVR residues are mutated and the variant antibodies displayed on phage and screened for a particular biological activity (e.g. binding affinity).

Alterations (e.g., substitutions) may be made in HVRs, e.g., to improve antibody affinity. Such alterations may be made in HVR "hotspots," i.e., residues encoded by codons that undergo mutation at high frequency during the somatic maturation process (see, e.g., Chowdhury, Methods Mol. Biol. 207:179-196 (2008)), and/or residues that contact antigen, with the resulting variant VH or VL being tested for binding affinity. Affinity maturation by constructing and reselecting from secondary libraries has been described, e.g., in Hoogenboom et al. in Methods in Molecular Biology 178:1-37 (O'Brien et al., ed., Human Press, Totowa, NJ, (2001).) In some embodiments of affinity maturation, diversity is introduced into the variable genes chosen for maturation by any of a variety of methods (e.g., error-prone PCR, chain shuffling, or oligonucleotide-directed mutagenesis). A secondary library is then created. The library is then screened to identify any antibody variants with the desired affinity. Another method to introduce diversity involves HVR-directed approaches, in which several HVR residues (e.g., 4-6 residues at a time) are randomized. HVR residues involved in antigen binding may be specifically identified, e.g., using alanine scanning mutagenesis or modeling. CDR-H3 and CDR-L3 in particular are often targeted.

In certain embodiments, substitutions, insertions, or deletions may occur within one or more HVRs so long as such alterations do not substantially reduce the ability of the antibody to bind antigen. For example, conservative alterations (e.g., conservative substitutions as provided herein) that do not substantially reduce binding affinity may be made in HVRs. Such alterations may, for example, be outside of antigen contacting residues in the HVRs. In certain embodiments of the variant VH and VL sequences provided above, each HVR either is unaltered, or contains no more than one, two or three amino acid substitutions.

A useful method for identification of residues or regions of an antibody that may be targeted for mutagenesis is called "alanine scanning mutagenesis" as described by Cunningham and Wells (1989) Science, 244:1081-1085. In this method, a residue or group of target residues (e.g., charged residues such as arg, asp, his, lys, and glu) are identified and replaced by a neutral or negatively charged amino acid (e.g., alanine or polyalanine) to determine whether the interaction of the antibody with antigen is affected. Further substitutions may be introduced at the amino acid locations demonstrating functional sensitivity to the initial substitutions. Alternatively, or additionally, a crystal structure of an antigen-antibody complex may be analyzed to identify contact points between the antibody and antigen. Such contact residues and neighboring residues may be targeted or eliminated as candidates for substitution. Variants may be screened to determine whether they contain the desired properties.

Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include an antibody with an N-terminal methionyl residue. Other insertional variants of the antibody molecule include the fusion of an enzyme (e.g. for ADEPT) or a polypeptide which increases the plasma half-life of the antibody to the N- or C-terminus of the antibody.

### b) Glycosylation variants

In certain embodiments, an antibody provided herein is altered to increase or decrease the extent to which the antibody is glycosylated. Addition or deletion of glycosylation sites to an antibody may be conveniently accomplished by altering the amino acid sequence such that one or more glycosylation sites is created or removed.

Where the antibody comprises an Fc region, the carbohydrate attached thereto may be altered. Native antibodies produced by mammalian cells typically comprise a branched, biantennary oligosaccharide that is generally attached by an N-linkage to Asn297 of the CH2 domain of the Fc region. See, e.g., Wright et al. TIBTECH 15:26-32 (1997). The oligosaccharide may include various carbohydrates, e.g., mannose, N-acetyl glucosamine (GlcNAc), galactose, and sialic acid, as well as a fucose attached to a GlcNAc in the "stem" of the biantennary oligosaccharide structure. In some embodiments, modifications of the oligosaccharide in an antibody of the disclosure may be made in order to create antibody variants with certain improved properties.

In one embodiment, antibody variants are provided having a carbohydrate structure that lacks fucose attached (directly or indirectly) to an Fc region. For example, the amount of fucose in such antibody may be from 1% to 80%, from 1% to 65%, from 5% to 65% or from 20% to 40%. The amount of fucose is determined by calculating the average amount of fucose within the sugar chain at Asn297, relative to the sum of all glycostructures attached to Asn 297 (e. g. complex, hybrid and high mannose structures) as measured by MALDI-TOF mass spectrometry, as described in WO 2008/077546, for example. Asn297 refers to the asparagine residue located at about position 297 in the Fc region (EU numbering of Fc region residues); however, Asn297 may also be located about +/- 3 amino acids upstream or downstream of position 297, i.e., between positions 294 and 300, due to minor sequence variations in antibodies. Such fucosylation variants may have improved ADCC function. See, e.g., US Patent Publication Nos. US 2003/0157108 (Presta, L.); US 2004/0093621 (Kyowa Hakko Kogyo Co., Ltd). Examples of publications related to "defucosylated" or "fucose-deficient" antibody variants include: US 2003/0157108; WO 2000/61739; WO 2001/29246; US 2003/0115614; US 2002/0164328; US 2004/0093621; US 2004/0132140; US 2004/0110704; US 2004/0110282; US 2004/0109865; WO 2003/085119; WO 2003/084570; WO 2005/035586; WO 2005/035778; WO2005/053742; WO2002/031140; Okazaki et al. J. Mol. Biol. 336:1239-1249 (2004); Yamane-Ohnuki et al. Biotech. Bioeng. 87: 614 (2004). Examples of cell lines capable of producing defucosylated antibodies include Lec13 CHO cells deficient in protein fucosylation (Ripka et al. Arch. Biochem. Biophys. 249:533-545 (1986); US Pat Appl No US 2003/0157108 A1, Presta, L; and WO 2004/056312 A1, Adams *et al.,* especially at Example 11), and knockout cell lines, such as alpha-1,6-fucosytransferase gene, *FUT8,* knockout CHO cells (see, e.g., Yamane-Ohnuki et al. Biotech. Bioeng. 87: 614 (2004); Kanda, Y. et al., Biotechnol. Bioeng., 94(4):680-688 (2006); and WO2003/085107).

Antibodies variants are further provided with bisected oligosaccharides, e.g., in which a biantennary oligosaccharide attached to the Fc region of the antibody is bisected by GlcNAc. Such antibody variants may have reduced fucosylation and/or improved ADCC function. Examples of such antibody variants are described, e.g., in WO 2003/011878 (Jean-Mairet et al.); US Patent No. 6,602,684 (Umana et al.); and US 2005/0123546 (Umana et al.). Antibody variants with at least one galactose residue in the oligosaccharide attached to the Fc region are also provided. Such antibody variants may have improved CDC function. Such antibody variants are described, e.g., in WO 1997/30087 (Patel et al.); WO 1998/58964 (Raju, S.); and WO 1999/22764 (Raju, S.).

**c) Fc region variants**

In certain embodiments, one or more amino acid alterations that are different from amino acid substitutions in the TRIM21-binding domain provided herein may be introduced into the Fc region of an antibody provided herein, thereby generating an Fc region variant. The Fc region variant may comprise a human Fc region sequence (*e.g.,* a human IgG1, IgG2, IgG3 or IgG4 Fc region) comprising an amino acid alteration (*e.g.* a substitution) at one or more amino acid positions.

In certain embodiments, the disclosure contemplates an antibody variant that possesses some but not all effector functions, which make it a desirable candidate for applications in which the half life of the antibody *in vivo* is important yet certain effector functions (such as complement and ADCC) are unnecessary or deleterious. *In vitro* and/or *in vivo* cytotoxicity assays can be conducted to confirm the reduction/depletion of CDC and/or ADCC activities. For example, Fc receptor (FcR) binding assays can be conducted to ensure that the antibody lacks Fc gamma R binding (hence likely lacking ADCC activity), but retains FcRn binding ability. The primary cells for mediating ADCC, NK cells, express Fc gamma RIII only, whereas monocytes express Fc gamma RI, Fc gamma RII and Fc gamma RIII. FcR expression on hematopoietic cells is summarized in Table 3 on page 464 of Ravetch and Kinet, Annu. Rev. Immunol. 9:457-492 (1991). Non-limiting examples of *in vitro* assays to assess ADCC activity of a molecule of interest is described in U.S. Patent No. 5,500,362 (see, e.g. Hellstrom, I. et al. Proc. Nat'l Acad. Sci. USA 83:7059-7063 (1986)) and Hellstrom, I et al., Proc. Nat'l Acad. Sci. USA 82:1499-1502 (1985); 5,821,337 (see Bruggemann, M. et al., J. Exp. Med. 166:1351-1361 (1987)). Alternatively, non-radioactive assays methods may be employed (see, for example, ACT1™ non-radioactive cytotoxicity assay for flow cytometry (CellTechnology, Inc. Mountain View, CA; and CytoTox 96® non-radioactive cytotoxicity assay (Promega, Madison, WI). Useful effector cells for such assays include peripheral blood mononuclear cells (PBMC) and Natural Killer (NK) cells. Alternatively, or additionally, ADCC activity of the molecule of interest may be assessed *in vivo, e.g.,* in an animal model such as that disclosed in Clynes et al. Proc. Nat'l Acad. Sci. USA 95:652-656 (1998). C1q binding assays may also be carried out to confirm that the antibody is unable to bind C1q and hence lacks CDC activity. See, e.g., C1q and C3c binding ELISA in WO 2006/029879 and WO 2005/100402. To assess complement activation, a CDC assay may be performed (see, for example, Gazzano-Santoro et al., J. Immunol. Methods 202:163 (1996); Cragg, M.S. et al., Blood 101:1045-1052 (2003); and Cragg, M.S. and M.J. Glennie, Blood 103:2738-2743 (2004)). FcRn binding and *in vivo* clearance/half life determinations can also be performed using methods known in the art (see, e.g., Petkova, S.B. et al., Int'l. Immunol. 18(12):1759-1769 (2006)).

Antibodies with reduced effector function include those with substitution of one or more of Fc region residues 238, 265, 269, 270, 297, 327 and 329 (U.S. Patent No. 6,737,056). Such Fc mutants include Fc mutants with substitutions at two or more of amino acid positions 265, 269, 270, 297 and 327, including the so-called "DANA" Fc mutant with substitution of residues 265 and 297 to alanine (US Patent No. 7,332,581).

Certain antibody variants with increased or decreased binding to FcRs are described. (See, e.g., U.S. Patent No. 6,737,056; WO 2004/056312, and Shields et al., J. Biol. Chem. 9(2): 6591-6604 (2001).)

In certain embodiments, an antibody variant comprises an Fc region with one or more amino acid substitutions which improve ADCC, e.g., substitutions at positions 298, 333, and/or 334 of the Fc region (EU numbering of residues).

In some embodiments, alterations are made in the Fc region that result in altered (*i.e.,* either increased or decreased) C1q binding and/or Complement Dependent Cytotoxicity (CDC), e.g., as described in US Patent No. 6,194,551, WO 99/51642, and Idusogie et al. J. Immunol. 164: 4178-4184 (2000).

Antibodies with increased half lives and increased binding to the neonatal Fc receptor (FcRn), which is responsible for the transfer of maternal IgGs to the fetus (Guyer et al., J. Immunol. 117:587 (1976) and Kim et al., J. Immunol. 24:249 (1994)), are described in US2005/0014934A1 (Hinton et al.). Those antibodies comprise an Fc region with one or more substitutions therein which increase binding of the Fc region to FcRn. Such Fc variants include those with substitutions at one or more of Fc region residues: 238, 256, 265, 272, 286, 303, 305, 307, 311, 312, 317, 340, 356, 360, 362, 376, 378, 380, 382, 413, 424 or 434, e.g., substitution of Fc region residue 434 (US Patent No. 7,371,826).

In one embodiment, an antibody comprising an altered TRIM21-binding domain can further contain within its Fc region one or more substitutions that increase the affinity to FcRn. In another embodiment, an antibody comprising an altered TRIM21-binding domain can further contain within its Fc region one or more substitutions that decrease the affinity to FcRn.

See also Duncan & Winter, Nature 322:738-40 (1988); U.S. Patent No. 5,648,260; U.S. Patent No. 5,624,821; and WO 94/29351 concerning other examples of Fc region variants.

**d) Cysteine engineered antibody variants**

In certain embodiments, it may be desirable to create cysteine engineered antibodies, e.g., "thioMAbs," in which one or more residues of an antibody are substituted with cysteine residues. In particular embodiments, the substituted residues occur at accessible sites of the antibody. By substituting those residues with cysteine, reactive thiol groups are thereby positioned at accessible sites of the antibody and may be used to conjugate the antibody to other moieties, such as drug moieties or linker-drug moieties, to create an immunoconjugate, as described further herein. In certain embodiments, any one or more of the following residues may be substituted with cysteine: V205 (Kabat numbering) of the light chain; A118 (EU numbering) of the heavy chain; and S400 (EU numbering) of the heavy chain Fc region. Cysteine engineered antibodies may be generated as described, e.g., in U.S. Patent No. 7,521,541.

**e) Antibody Derivatives**

In certain embodiments, an antibody provided herein may be further modified to contain additional nonproteinaceous moieties that are known in the art and readily available. The moieties suitable for derivatization of the antibody include but are not limited to water soluble polymers. Non-limiting examples of water soluble polymers include, but are not limited to, polyethylene glycol (PEG), copolymers of ethylene glycol/propylene glycol, carboxymethylcellulose, dextran, polyvinyl alcohol, polyvinyl pyrrolidone, poly-1, 3-dioxolane, poly-1,3,6-trioxane, ethylene/maleic anhydride copolymer, polyaminoacids (either homopolymers or random copolymers), and dextran or poly(n-vinyl pyrrolidone)polyethylene glycol, polypropylene glycol homopolymers, polypropylene oxide/ethylene oxide co-polymers, polyoxyethylated polyols (e.g., glycerol), polyvinyl alcohol, and mixtures thereof. Polyethylene glycol propionaldehyde may have advantages in manufacturing due to its stability in water. The polymer may be of any molecular weight, and may be branched or unbranched. The number of polymers attached to the antibody may vary, and if more than one polymer are attached, they can be the same or different molecules. In general, the number and/or type of polymers used for derivatization can be determined based on considerations including, but not limited to, the particular properties or functions of the antibody to be improved, whether the antibody derivative will be used in a therapy under defined conditions, etc.

In another embodiment, conjugates of an antibody and nonproteinaceous moiety that may be selectively heated by exposure to radiation are provided. In one embodiment, the nonproteinaceous moiety is a carbon nanotube (Kam et al., Proc. Natl. Acad. Sci. USA 102: 11600-11605 (2005)). The radiation may be of any wavelength, and includes, but is not limited to, wavelengths that do not harm ordinary cells, but which heat the nonproteinaceous moiety to a temperature at which cells proximal to the antibody-nonproteinaceous moiety are killed.

**H. Recombinant Methods and Compositions**

Antibodies may be produced using recombinant methods and compositions, e.g., as described in U.S. Patent No. 4,816,567. In one embodiment, isolated nucleic acid encoding an antigen-binding molecule comprising an altered TRIM21-binding domain described herein is provided. Such nucleic acid may encode an amino acid sequence comprising the VL and/or an amino acid sequence comprising the VH of the antibody (e.g., the light and/or heavy chains of the antibody). In a further embodiment, one or more vectors (e.g., expression vectors) comprising such nucleic acid are provided. In a further embodiment, a host cell comprising such nucleic acid is provided. In one such embodiment, a host cell comprises (e.g., has been transformed with): (1) a vector comprising a nucleic acid that encodes an amino acid sequence comprising the VL of the antibody and an amino acid sequence comprising the VH of the antibody, or (2) a first vector comprising a nucleic acid that encodes an amino acid sequence comprising the VL of the antibody and a second vector comprising a nucleic acid that encodes an amino acid sequence comprising the VH of the antibody. In one embodiment, the host cell is eukaryotic, e.g. a Chinese Hamster Ovary (CHO) cell or lymphoid cell (e.g., Y0, NS0, Sp2/0 cell). In one embodiment, a method of making an antigen-binding molecule comprising an altered TRIM21-binding domain is provided, wherein the method comprises culturing a host cell comprising a nucleic acid encoding the antigen-binding molecule, as provided above, under conditions suitable for expression of the antigen-binding molecule comprising an altered TRIM21-binding domain, and optionally recovering the antigen-binding molecule from the host cell (or host cell culture medium).

For recombinant production of an antigen-binding molecule comprising an altered TRIM21-binding domain, nucleic acid encoding an antigen-binding molecule, e.g., as described above, is isolated and inserted into one or more vectors for further cloning and/or expression in a host cell. Such nucleic acid may be readily isolated and sequenced using conventional procedures (e.g., by using oligonucleotide probes that are capable of binding specifically to genes encoding the heavy and light chains of the antibody).

When the antigen-binding molecule of the disclosure is an antibody, suitable host cells for cloning or expression of antibody-encoding vectors include prokaryotic or eukaryotic cells described herein. For example, antibodies may be produced in bacteria, in particular when glycosylation and Fc effector function are not needed. For expression of antibody fragments and polypeptides in bacteria, see, e.g., U.S. Patent Nos. 5,648,237, 5,789,199, and 5,840,523. (See also Charlton, Methods in Molecular Biology, Vol. 248 (B.K.C. Lo, ed., Humana Press, Totowa, NJ, 2003), pp. 245-254, describing expression of antibody fragments in *E. coli.*) After expression, the antibody may be isolated from the bacterial cell paste in a soluble fraction and can be further purified.

In addition to prokaryotes, eukaryotic microbes such as filamentous fungi or yeast are suitable cloning or expression hosts for antibody-encoding vectors, including fungi and yeast strains whose glycosylation pathways have been "humanized," resulting in the production of an antibody with a partially or fully human glycosylation pattern. See Gerngross, Nat. Biotech. 22:1409-1414 (2004), and Li et al., Nat. Biotech. 24:210-215 (2006).

Suitable host cells for the expression of glycosylated antibody are also derived from multicellular organisms (invertebrates and vertebrates). Examples of invertebrate cells include plant and insect cells. Numerous baculoviral strains have been identified which may be used in conjunction with insect cells, particularly for transfection of *Spodoptera frugiperda* cells.

Plant cell cultures can also be utilized as hosts. *See,* e.g., US Patent Nos. 5,959,177, 6,040,498, 6,420,548, 7,125,978, and 6,417,429 (describing PLANTIBODIES™ technology for producing antibodies in transgenic plants).

Vertebrate cells may also be used as hosts. For example, mammalian cell lines that are adapted to grow in suspension may be useful. Other examples of useful mammalian host cell lines are monkey kidney CV1 line transformed by SV40 (COS-7); human embryonic kidney line (293 or 293 cells as described, e.g., in Graham et al., J. Gen Virol. 36:59 (1977)); baby hamster kidney cells (BHK); mouse sertoli cells (TM4 cells as described, e.g., in Mather, Biol. Reprod. 23:243-251 (1980)); monkey kidney cells (CV1); African green monkey kidney cells (VERO-76); human cervical carcinoma cells (HELA); canine kidney cells (MDCK); buffalo rat liver cells (BRL 3A); human lung cells (W138); human liver cells (Hep G2); mouse mammary tumor (MMT 060562); TRI cells, as described, e.g., in Mather et al., Annals N.Y. Acad. Sci. 383:44-68 (1982); MRC 5 cells; and FS4 cells. Other useful mammalian host cell lines include Chinese hamster ovary (CHO) cells, including $DHFR^-$ CHO cells (Urlaub et al., Proc. Natl. Acad. Sci. USA 77:4216 (1980)); and myeloma cell lines such as Y0, NS0 and Sp2/0. For a review of certain mammalian host cell lines suitable for antibody production, see, e.g., Yazaki and Wu, Methods in Molecular Biology, Vol. 248 (B.K.C. Lo, ed., Humana Press, Totowa, NJ), pp. 255-268 (2003).

**I. Assays**

Antigen-binding molecules comprising an altered TRIM21-binding domain provided herein may be identified, screened for, or characterized for their physical/chemical properties and/or biological activities by various assays known in the art.

1. Measurement of binding affinity for TRIM21

In certain embodiments, an antigen-binding molecule provided herein has a dissociation constant (KD) for TRIM21 of ≤1 μM, ≤100 nM, ≤10 nM, ≤1 nM, ≤0.1 nM, ≤0.01 nM, or ≤0.001 nM (e.g. $10^{-8}$ M or less, e.g. from $10^{-8}$ M to $10^{-13}$ M, e.g. from $10^{-9}$ M to $10^{-13}$ M).

In one embodiment, binding affinity and KD are measured by a radiolabeled antigen binding assay (RIA). In one embodiment, an RIA is performed using the TRIM21-binding domain of an antigen-binding molecule of interest and TRIM21. For example, solution binding affinity of an antigen-binding molecule (IgG antibody) for an antigen (TRIM21, FcRn or such) is measured by equilibrating the IgG antibody with a minimal concentration of ($^{125}$I)-labeled antigen in the presence of a titration series of unlabeled antigen, then capturing the bound antigen with an anti-IgG antibody-coated plate (see for example, Chen et al., J. Mol. Biol. 293:865-881 (1999)). To establish conditions for the assay, MICROTITER® multi-well plates (Thermo Scientific) are coated overnight with 5 μg/mL of a capturing anti-IgG antibody (Cappel Labs) in 50 mM sodium carbonate (pH 9.6), and subsequently blocked with 2% (w/v) bovine serum albumin in PBS for two to five hours at room temperature (approximately 23°C). In a non-adsorbent plate (Nunc #269620), 100 pM or 26 pM of [$^{125}$I]-antigen are mixed with serial dilutions of an antigen-binding molecule of interest (e.g., consistent with assessment of the anti-VEGF antibody, Fab-12, in Presta et al., Cancer Res. 57:4593-4599 (1997)). The antigen-binding molecule of interest is then incubated overnight; however, the incubation may continue for a longer period (e.g., about 65 hours) to ensure that equilibrium is reached. Thereafter, the mixtures are transferred to the capture plate for incubation at room temperature (e.g., for one hour). The solution is then removed and the plate washed eight times with 0.1% polysorbate 20 (TWEEN-20®) in PBS. When the plates have dried, 150 μL/well of scintillant (MICROSCINT-20™; Packard) is added, and the plates are counted on a TOPCOUNT™ gamma counter (Packard) for ten minutes. Concentrations of each antigen-binding molecule that gives not more than 20% of maximal binding are chosen for use in competitive binding assays.

According to another embodiment, binding affinity and KD are measured using a BIACORE® surface plasmon resonance assay. For example, an assay using BIACORE®-2000, BIACORE®-3000, BIACORE® T200, or BIACORE®-4000 (GE Healthcare) is performed at 25°C with CM5 chips onto which an antigen-binding molecule is immobilized at ~10 response units (RU). In one embodiment, carboxymethylated dextran biosensor chips (CM5, GE Healthcare) are activated with *N*-ethyl-*N'*-(3-dimethylaminopropyl)-carbodiimide hydrochloride (EDC) and *N*-hydroxysuccinimide (NHS) according to the supplier's instructions. The antigen-binding molecule is diluted with 10 mM sodium acetate, pH 4.8, to 5 μg/ml (~0.2 μM) before injection at a flow rate of 5 μl/minute to achieve approximately 10 response units (RU) of coupled protein. Following the injection of the antigen-binding molecule, 1 M ethanolamine is injected to block unreacted groups. For kinetics measurements, two-fold serial dilutions of an antigen (such as TRIM21 or FcRn) (0.78 nM to 500 nM) are injected in PBS with 0.05% polysorbate 20 (TWEEN-20™) surfactant (PBST) at 25 degrees C at a flow rate of approximately 25 micro l/min. Association rates ($k_{on}$) and dissociation rates ($k_{off}$) are calculated using a simple one-to-one Langmuir binding model (BIACORE® Evaluation Software version 3.2) by simultaneously fitting the association and dissociation sensorgrams. The equilibrium dissociation constant (KD) is calculated as the ratio $k_{off}/k_{on}$. See, e.g., Chen et al., J. Mol. Biol. 293:865-881 (1999). If the on-rate exceeds $10^6$ $M^{-1}$ $s^{-1}$ by the surface plasmon resonance assay above, then the on-rate can be determined by using a fluorescent quenching technique that measures the increase or decrease in fluorescence emission intensity (excitation = 295 nm; emission = 340 nm, 16 nm band-pass) at 25°C of a 20 nM antigen-binding molecule in PBS, pH 7.2, in the presence of increasing concentrations of antigen as measured in a spectrometer, such as a stop-flow equipped spectrophotometer (Aviv Instruments) or a 8000-series SLM-AMINCO™ spectrophotometer (ThermoSpectronic) with a stirred cuvette. In a further embodiment, the binding affinity of an antigen-binding molecule can be confirmed using a BIACORE® surface plasmon resonance assay according to the method described in the Examples below.

2. Measurement of antigen-binding molecule within cytosol

In one embodiment, an antigen-binding molecule within the cytosol is measured at any time point after the antigen molecule is contacted with cells. An antigen-binding molecule is contacted with cells by any method including incubation and microinjection. When an antigen-binding molecule is contacted with cells by incubation, the time of incubation and the time before measuring the antigen-binding molecule within the cytosol after the incubation are arbitrarily determined. For example, when an antigen-binding molecule present within the cytosol is measured only once after the antigen-binding molecule is contacted with cells, the antigen-binding molecule is incubated with the cells for 1 hour, 2 hours, 3 hours, 4 hours, 5 hours, or 6 hours, and then the medium containing the antigen-binding molecule is removed, immediately after which the antigen-binding molecule within the cytosol may be measured. For example, when an antigen-binding molecule present within the cytosol is measured multiple times after the antigen-binding molecule is contacted with cells, the antigen-binding molecule is incubated with the cells for 1 hour, 2 hours, 3 hours, 4 hours, 5 hours, or 6 hours, and then the medium containing the antigen-binding molecule is removed. After further incubation is performed in a fresh medium without the antigen-binding molecule for 0 hours, 0.25 hours, 0.5 hours, 1 hour, 1.5 hours, 2 hours, 2.5 hours, 3 hours, 3.5 hours, 4 hours, 4.5 hours, 5 hours, 5.5 hours, 6 hours, 6.5 hours, 7 hours, 7.5 hours, 8 hours, 8.5 hours, 9 hours, 9.5 hours, 10 hours, 11 hours, 12 hours, 13 hours, 14 hours, 15 hours, and/or 16 hours, the antigen-binding molecule within the cytosol may be measured. In the present disclosure, an antigen-binding molecule present within the cytosol at any time point can be measured by a suitable method that allows detection of antigen-binding molecules within the cytosol. Examples of such a method include but are not limited to BirA assay, imaging by fluorescence microscopy, and Split-GFP.

a) BirA assay

In one embodiment, the amount of an antigen-binding molecule present within the cytosol at any time point can be evaluated by BirA assay, in which the antigen-binding molecule fused with a biotinylated Avi tag is detected using a labeled biotin-binding protein. The biotin-labeled Avi tag is measured by using an avidin-peroxidase conjugate and an appropriate substrate. For example, when the biotinylated Avi tag is detected using streptavidin-HRP, the amount of the antigen-binding molecule present within the cytosol can be represented by the intensity of the luminescence signal of HRP. BirA assay is a method known to those skilled in the art, and it is described, for example, in W.P.R. Verdurmen et al., Journal of Controlled Release 200 (2015) 13-22 and in the Examples of this disclosure. The conditions used in the assay for determining the amount of the antigen-binding molecule present within the cytosol may be selected appropriately by those skilled in the art, and are therefore not particularly limited. Furthermore, the antigen-binding molecule fused with a biotinylated Avi tag within the cytosol may be labeled by another labeling substance that can be detected or measured. Specifically, radioactive labeling, fluorescence labeling, or such are known. Detection of the labeled biotin-binding protein in a BirA assay (for example, detection of the HRP luminescence signal in streptavidin HRP) may be performed appropriately by a method known to those skilled in the art. Specifically, western blotting, capillary immunoassay (Protein Simple), or such are known.

b) Imaging by fluorescence microscopy

In another embodiment, the amount of an antigen-binding molecule present within the cytosol at any time point can be evaluated by detecting a labeled protein that binds to the antigen-binding molecule. For example, when the antigen-binding molecule includes an IgG antibody, the antigen-binding molecule present within the cytosol can be detected using a labeled anti-IgG antibody. Known labels include, for example, radioactive labels or fluorescent labels. Labeled antigen-binding molecules may be detected appropriately by methods known to those skilled in the art. For example, as described herein and in the Examples of the present disclosure, labeled antigen-binding molecules can be detected by imaging the fluorescence signals using a fluorescence microscope.

c) Split-GFP

In another embodiment, the amount of an antigen-binding molecule present within the cytosol at any time point can be evaluated by detecting GFP fluorescence signals in a split-GFP complementary system. Specific methods are known to those skilled in the art, and for example, described in WO2016/013870. Specifically, while the green fluorescence protein, GFP, is deprived of its fluorescence property when split into fragment 1-10 and fragment 11, the fluorescence property can be restored when the two fragments come closer and bind to each other (Cabantous *et al.,* 2005). Utilizing this characteristics, the GFP 1-10 fragment is expressed within the cytosol, and the GFP 11 fragment is fused to any portion of the antigen-binding molecule. GFP fluorescence observed by this method indicates that the two fragments of GFP are bound, or more specifically, the antigen-binding molecule is present within the cytosol.

J. Methods for altering, methods for producing, and methods of screening for antigen-binding molecules

(1) Methods for altering antigen-binding molecules

In one aspect, the present disclosure provides a method for altering an antigen-binding molecule comprising a TRIM21-binding domain, which comprises introducing one or more amino acid alterations into a TRIM21-binding domain of a parent antigen-binding molecule, wherein the alterations result in (i) a decrease or an increase in the binding affinity of the antigen-binding molecule for TRIM21, (ii) an increase or a decrease in the cytosolic half-life of the antigen-binding molecule, (iii) reduction or improvement in the ability of the antigen-binding molecule to remove a cytosolic antigen, and/or (iv) an increase or a decrease in the resistance of the antigen-binding molecule to proteasomal degradation within the cytosol, compared to the parent antigen-binding molecule.

In one embodiment, the amount of the altered antigen-binding molecule present within the cytosol of a cell after a certain amount of that antigen-binding molecule is contacted with the cell is increased or decreased compared to the amount of the parent antigen-binding molecule present within the cytosol of a cell after the same amount of the parent antigen-binding molecule is contacted with the cell. In one embodiment, the aforementioned alteration method further comprises comparing the amount of the antigen-binding molecule comprising the altered TRIM21-binding domain present within the cytosol of a cell after that antigen-binding molecule is contacted with the cell with the amount of the parent antigen-binding molecule present within the cytosol of a cell after the same amount of the parent antigen-binding molecule is contacted with the cell.

In one embodiment, the one or more amino acid alterations introduced into the TRIM21-binding domain of the parent antigen-binding molecule may be substitution, deletion, insertion, and/or addition of one or more amino acids, for example, substitution, deletion, insertion, and/or addition of 1 to 20 amino acids, preferably 1 to 15 amino acids, more preferably 1 to 10 amino acids, 1 to 8 amino acids, 1 to 7 amino acids, 1 to 6 amino acids, 1 to 5 amino acids, 1 to 4 amino acids, 1 to 3 amino acids, 1 to 2 amino acids, or one amino acid. In another embodiment, the amino acid sequence of an altered TRIM21-binding domain is 70% or more, for example, 80% or more, 85% or more, 90% or more, 95% or more, 97% or more, 98% or more, or 99% or more identical to the amino acid sequence of its unaltered parent TRIM21-binding domain.

(2) Methods for producing antigen-binding molecules

In one aspect, the present disclosure provides a method for producing an antigen-binding molecule comprising an altered TRIM21-binding domain, which comprises introducing one or more amino acid alterations into a TRIM21-binding domain of a parent antigen-binding molecule, wherein the alterations result in (i) a decrease or an increase in the binding affinity of the antigen-binding molecule for TRIM21, (ii) an increase or a decrease in the cytosolic half-life of the antigen-binding molecule, (iii) reduction or improvement in the ability of the antigen-binding molecule to remove a cytosolic antigen, and/or (iv) an increase or a decrease in the resistance of the antigen-binding molecule to proteasomal degradation within the cytosol, compared to the parent antigen-binding molecule.

In one embodiment, the amount of the altered antigen-binding molecule present within the cytosol of a cell after a certain amount of that antigen-binding molecule is contacted with the cell is increased or decreased compared to the amount of the parent antigen-binding molecule present within the cytosol of a cell after the same amount of the parent antigen-binding molecule is contacted with the cell. In one embodiment, the aforementioned production method further comprises comparing the amount of the antigen-binding molecule comprising the altered TRIM21-binding domain present within the cytosol of a cell after the antigen-binding molecule is contacted with the cell with the amount of the parent antigen-binding molecule present within the cytosol of a cell after the same amount of the parent antigen-binding molecule is contacted with the cell.

In one embodiment, the aforementioned production method further comprises

(a) obtaining an expression vector comprising an appropriate promoter operably linked to a gene encoding the antigen-binding molecule comprising the altered TRIM21-binding domain;
(b) introducing the vector into a host cell, and culturing the host cell to produce the antigen-binding molecule; and
(c) recovering the antigen-binding molecule from the culture of the host cell.

(3) Methods of screening for antigen-binding molecules

In one aspect, the present disclosure provides a method of screening for an antigen-binding molecule comprising an altered TRIM21-binding domain, which comprises

(a) providing a parent antigen-binding molecule having a TRIM21-binding domain;
(b) obtaining a candidate molecule comprising an altered TRIM21-binding domain by introducing one or more amino acid alterations into the TRIM21-binding domain of the parent antigen-binding molecule;
(c) determining (i) the binding affinity of the altered TRIM21-binding domain of the candidate molecule for TRIM21, (ii) the cytosolic half-life of the candidate molecule, (iii) the ability of the candidate molecule to remove a cytosolic antigen, or (iv) the resistance of the candidate molecule to proteasomal degradation in the cytosol; and
(d) identifying the candidate molecule as a suitable molecule when the candidate molecule has (i) decreased or increased binding affinity for TRIM21, (ii) increased or decreased cytosolic half-life, (iii) reduced or improved ability to remove a cytosolic antigen , and/or (iv) increased or decreased resistance to proteasomal degradation within cytosol, compared to the parent antigen-binding molecule. In one embodiment, the amount of the suitable molecule present within the cytosol of a cell after a certain amount of the suitable molecule is contacted with the cell is increased or decreased compared to the amount of the parent antigen-binding molecule present within the cytosol of a cell after the same amount of the parent antigen-binding molecule is contacted with the cell. In one embodiment, the aforementioned screening method further comprises comparing the amount of the antigen-binding molecule comprising the altered TRIM21-binding domain present within the cytosol of a cell after the antigen-binding molecule is contacted with the cell with the amount of the parent antigen-binding molecule present within the cytosol of a cell after the same amount of the parent antigen-binding molecule is contacted with the cell.

(4) Methods for imaging cytosolic antigens

In another aspect, the present disclosure provides a method for imaging a cytosolic antigen in a sample cell, which comprises using an antigen-binding molecule comprising an altered TRIM21-binding domain of the present disclosure, an antigen-binding molecule altered by the aforementioned alteration method, an antigen-binding molecule produced by the aforementioned production method, or an antigen-binding molecule identified as a suitable molecule by the aforementioned screening method.

Furthermore, in another aspect, the present disclosure provides an antigen-binding molecule comprising an altered TRIM21-binding domain of the present disclosure, an antigen-binding molecule altered by the aforementioned alteration method, an antigen-binding molecule produced by the aforementioned production method, or an antigen-binding molecule identified as a suitable molecule by the aforementioned screening method, for use in imaging a cytosolic antigen in a sample cell.

In another aspect, the present disclosure provides use of an antigen-binding molecule comprising an altered TRIM21-binding domain of the present disclosure, an antigen-binding molecule altered by the aforementioned alteration method, an antigen-binding molecule produced by the aforementioned production method, or an antigen-binding molecule identified as a suitable molecule by the aforementioned screening method, in producing an agent for imaging a cytosolic antigen.

In an embodiment of these aspects, the antigen-binding molecule used has a cytosol-penetrating ability by itself or is altered to have a cytosol-penetrating ability, and can penetrate into the cytosol by contact with a cell (for example, by being incubated with a cell). In another embodiment of these aspects, the antigen-binding molecule used is microinjected into the cytosol. In specific embodiments of these aspects, the antigen-binding molecule used is labeled.

In an embodiment of these aspects, the antigen-binding molecule used has an increased cytosolic half-life, decreased ability to remove a cytosolic antigen, increased resistance to proteasomal degradation in the cytosol, decreased ability to activate the NF-kB signaling pathway, and/or decreased ability to induce inflammatory and antitumor responses, compared to the parent antigen-binding molecule.

**K. Immunoconjugates**

The disclosure also provides immunoconjugates comprising an antigen-binding molecule comprising an altered TRIM21-binding domain herein conjugated to one or more cytotoxic agents, such as chemotherapeutic agents or drugs, growth inhibitory agents, toxins (e.g., protein toxins, enzymatically active toxins of bacterial, fungal, plant, or animal origin, or fragments thereof), or radioactive isotopes.

L. Methods for knockdown of a cytosolic antigen

In a specific embodiment, when an antigen-binding molecule comprising an altered TRIM21-binding domain has a decreased cytosolic half-life and contains a cytosolic antigen-binding domain, the antigen-binding molecule has an improved ability to remove a cytosolic antigen compared to the parent antigen-binding molecule. Therefore, the antigen-binding molecules comprising an altered TRIM21-binding domain and having a decreased cytosolic half-life provided herein are useful in knocking down cytosolic antigens at the protein level. The term "knockdown" as used herein means a decrease in the expression level or amount of a specific protein. Specifically, knockdown of a cytosolic antigen at the protein level results in a decrease in the amount of the cytosolic antigen per cell.

**M. Methods and Compositions for Diagnostics and Detection**

In certain embodiments, any of the antigen-binding domains comprising an altered TRIM21-binding domain provided herein is useful for detecting the presence of cytosolic antigens in a biological sample. The term "detecting" as used herein encompasses quantitative or qualitative detection.

In one embodiment, an antigen-binding molecule comprising an altered TRIM21-binding domain for use in a method of diagnosis or detection is provided. In a further aspect, a method of detecting the presence of a cytosolic antigen in a biological sample is provided. In certain embodiments, the method comprises contacting the biological sample with an antigen-binding molecule comprising an altered TRIM21-binding domain as described herein under conditions permissive for binding of the antigen-binding molecule comprising an altered TRIM21-binding domain to the cytosolic antigen, and detecting whether a complex is formed between the antigen-binding molecule comprising an altered TRIM21-binding domain and the cytosolic antigen. Such method may be an *in vitro* or *in vivo* method.

In a further embodiment, when an antigen-binding molecule comprising an altered TRIM21-binding domain has an increased cytosolic half-life, the antigen-binding molecule can remain within the cytosol for a longer time than the parent antigen-binding molecule. Therefore, the antigen-binding molecules comprising an altered TRIM21-binding domain and having an increased cytosolic half-life provided herein are useful in detecting cytosolic antigens by methods such as imaging. Furthermore, since the use of an antigen-binding molecule having an increased cytosolic half-life can lead to an increased cytosolic concentration of antigen-binding molecule at the time of detection, it can be expected to increase detection sensitivity of cytosolic antigen.

In certain embodiments, labeled antigen-binding molecules comprising an altered TRIM21-binding domain are provided. Labels include, but are not limited to, labels or moieties that are detected directly (such as fluorescent, chromophoric, electron-dense, chemiluminescent, and radioactive labels), as well as moieties, such as enzymes or ligands, that are detected indirectly, e.g., through an enzymatic reaction or molecular interaction. Exemplary labels include, but are not limited to, the radioisotopes $^{32}P$, $^{14}C$, $^{125}I$, $^{3}H$, and $^{131}I$, fluorophores such as rare earth chelates or fluorescein and its derivatives, rhodamine and its derivatives, dansyl, umbelliferone, luceriferases, e.g., firefly luciferase and bacterial luciferase (U.S. Patent No. 4,737,456), luciferin, 2,3-dihydrophthalazinediones, horseradish peroxidase (HRP), alkaline phosphatase, beta-galactosidase, glucoamylase, lysozyme, saccharide oxidases, e.g., glucose oxidase, galactose oxidase, and glucose-6-phosphate dehydrogenase, heterocyclic oxidases such as uricase and xanthine oxidase, those coupled with an enzyme that employs hydrogen peroxide to oxidize a dye precursor such as HRP, lactoperoxidase, or microperoxidase, biotin/avidin, spin labels, bacteriophage labels, stable free radicals, and the like.

[IIF01]

## N. Pharmaceutical Formulations

Pharmaceutical formulations of an antigen-binding molecule comprising an altered TRIM21-binding domain as described herein are prepared by mixing such an antigen-binding molecule having the desired degree of purity with one or more optional pharmaceutically acceptable carriers (Remington's Pharmaceutical Sciences 16th edition, Osol, A. Ed. (1980)), in the form of lyophilized formulations or aqueous solutions. Pharmaceutically acceptable carriers are generally nontoxic to recipients at the dosages and concentrations employed, and include, but are not limited to: buffers such as phosphate, citrate, and other organic acids; antioxidants including ascorbic acid and methionine; preservatives (such as octadecyldimethylbenzyl ammonium chloride; hexamethonium chloride; benzalkonium chloride; benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g. Zn-protein complexes); and/or non-ionic surfactants such as polyethylene glycol (PEG). Exemplary pharmaceutically acceptable carriers herein further include interstitial drug dispersion agents such as soluble neutral-active hyaluronidase glycoproteins (sHASEGP), for example, human soluble PH-20 hyaluronidase glycoproteins, such as rHuPH20 (HYLENEX®, Baxter International, Inc.). Certain exemplary sHASEGPs and methods of use, including rHuPH20, are described in US Patent Publication Nos. 2005/0260186 and 2006/0104968. In one aspect, a sHASEGP is combined with one or more additional glycosaminoglycanases such as chondroitinases.

Exemplary lyophilized antibody formulations are described in US Patent No. 6,267,958. Aqueous antibody formulations include those described in US Patent No. 6,171,586 and WO2006/044908, the latter formulations including a histidine-acetate buffer.

The formulation herein may also contain more than one active ingredients as necessary for the particular indication being treated, preferably those with complementary activities that do not adversely affect each other. Such active ingredients are suitably present in combination in amounts that are effective for the purpose intended.

Active ingredients may be entrapped in microcapsules prepared, for example, by coacervation techniques or by interfacial polymerization, for example, hydroxymethylcellulose or gelatin-microcapsules and poly-(methylmethacrylate) microcapsules, respectively, in colloidal drug delivery systems (for example, liposomes, albumin microspheres, microemulsions, nano-particles and nanocapsules) or in macroemulsions. Such techniques are disclosed in Remington's Pharmaceutical Sciences 16th edition, Osol, A. Ed. (1980).

Sustained-release preparations may be prepared. Suitable examples of sustained-release preparations include semipermeable matrices of solid hydrophobic polymers containing the antigen-binding molecule, which matrices are in the form of shaped articles, e.g. films, or microcapsules.

The formulations to be used for *in vivo* administration are generally sterile. Sterility may be readily accomplished, e.g., by filtration through sterile filtration membranes.

## O. Therapeutic Methods and Compositions

Any of the antigen-binding molecules comprising an altered TRIM21-binding domain provided herein may be used in therapeutic methods.

In one aspect, an antigen-binding molecule comprising an altered TRIM21-binding domain for use as a medicament is provided. In certain embodiments, an antigen-binding molecule comprising an altered TRIM21-binding domain for use in a method of treatment is provided. In one such embodiment, the method further comprises administering to the individual an effective amount of at least one additional therapeutic agent, e.g., as described below. An "individual" according to any of the above embodiments is preferably a human.

In a further aspect, the disclosure provides for the use of an antigen-binding molecule comprising an altered TRIM21-binding domain in the manufacture or preparation of a medicament. In one such embodiment, the method further comprises administering to the individual an effective amount of at least one additional therapeutic agent. An "individual" according to any of the above embodiments may be a human.

In a further aspect, the present disclosure provides a pharmaceutical formulation comprising any of the antigen-binding molecules comprising an altered TRIM21-binding domain provided herein (for example, for use in any of the therapeutic methods described above). In one embodiment, the pharmaceutical formulation includes any of the antigen-binding molecules comprising an altered TRIM21-binding domain provided herein and a pharmaceutically acceptable carrier. In another embodiment, a pharmaceutical formulation contains any of the antigen-binding molecules comprising an altered TRIM21-binding domain provided herein, and at least one additional therapeutic agent.

An antigen-binding molecule of the disclosure (and any additional therapeutic agent) can be administered by any suitable means, including parenteral, intrapulmonary, and intranasal, and, if desired for local treatment, intralesional administration. Parenteral infusions include intramuscular, intravenous, intraarterial, intraperitoneal, or subcutaneous administration. Dosing can be by any suitable route, e.g. by injections, such as intravenous or subcutaneous injections, depending in part on whether the administration is brief or chronic. Various dosing schedules including but not limited to single or multiple administrations over various time-points, bolus administration, and pulse infusion are contemplated herein.

Antigen-binding molecules of the disclosure would be formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners. The antigen-binding molecule need not be, but is optionally formulated with one or more agents currently used to prevent or treat the disorder in question. The effective amount of such other agents depends on the amount of antigen-binding molecule present in the formulation, the type of disorder or treatment, and other factors discussed above. These are generally used in the same dosages and with administration routes as described herein, or about from 1 to 99% of the dosages described herein, or in any dosage and by any route that is empirically/clinically determined to be appropriate.

For the prevention or treatment of disease, the appropriate dosage of an antigen-binding molecule of the disclosure (when used alone or in combination with one or more other additional therapeutic agents) will depend on the type of disease to be treated, the type of antibody (when the antigen-binding molecule is an antibody), the severity and course of the disease, whether the antigen-binding molecule is administered for preventive or therapeutic purposes, previous therapy, the patient's clinical history and response to the antigen-binding molecule, and the discretion of the attending physician. The antigen-binding molecule is suitably administered to the patient at one time or over a series of treatments. Depending on the type and severity of the disease, about 1 micro g/kg to 15 mg/kg (e.g. 0.1mg/kg-10mg/kg) of antigen-binding molecule can be an initial candidate dosage for administration to the patient, whether, for example, by one or more separate administrations, or by continuous infusion. One typical daily dosage might range from about 1 micro g/kg to 100 mg/kg or more, depending on the factors mentioned above. For repeated administrations over several days or longer, depending on the condition, the treatment would generally be sustained until a desired suppression of disease symptoms occurs. One exemplary dosage of the antigen-binding molecule would be in the range from about 0.05 mg/kg to about 10 mg/kg. Thus, one or more doses of about 0.5 mg/kg, 2.0 mg/kg, 4.0 mg/kg or 10 mg/kg (or any combination thereof) may be administered to the patient. Such doses may be administered intermittently, e.g. every week or every three weeks (*e.g.* such that the patient receives from about two to about twenty, or *e.g.* about six doses of the antigen-binding molecule). An initial higher loading dose, followed by one or more lower doses may be administered. The progress of this therapy is easily monitored by conventional techniques and assays.

It is understood that any of the above formulations or therapeutic methods may be carried out using an immunoconjugate of the disclosure in place of or in addition to an antigen-binding molecule comprising an altered TRIM21-binding domain.

## P. Articles of Manufacture

In another aspect of the disclosure, an article of manufacture containing materials useful for the treatment, prevention and/or diagnosis of the disorders described above is provided. The article of manufacture comprises a container and a label on or a package insert associated with the container. Suitable containers include, for example, bottles, vials, syringes, IV solution bags, etc. The containers may be formed from a variety of materials such as glass or plastic. The container holds a composition which is by itself or combined with another composition effective for treating, preventing and/or diagnosing the condition and may have a sterile access port (for example the container may be an intravenous solution bag or a vial having a stopper pierceable by a hypodermic injection needle). At least one active ingredient in the composition is an antigen-binding molecule of the disclosure. The label or package insert indicates that the composition is used for treating the condition of choice. Moreover, the article of manufacture may comprise (a) a first container with a composition contained therein, wherein the composition comprises an antigen-binding molecule of the disclosure; and (b) a second container with a composition contained therein, wherein the composition comprises a further cytotoxic or otherwise therapeutic agent. The article of manufacture in this embodiment of the disclosure may further comprise a package insert indicating that the compositions can be used to treat a particular condition. Alternatively, or additionally, the article of manufacture may further comprise a second (or third) container comprising a pharmaceutically-acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

It is understood that any of the above articles of manufacture may include an immunoconjugate of the disclosure in place of or in addition to an antigen-binding molecule comprising an altered TRIM21-binding domain.

[Examples]

The following are examples of methods and compositions of the disclosure. It is understood that various other embodiments may be practiced, given the general description provided above.

Although the foregoing invention has been described in some detail by way of illustration and example for purposes of clarity of understanding, the descriptions and examples should not be construed as limiting the scope of the disclosure. The disclosures of all patent and scientific literature cited herein are expressly incorporated in their entirety by reference.

[Example 1]

Concept

As described in Background Art above, it has been reported that when an antiviral antibody binds to an antigen and enters the cytosol, TRIM21 binds to the Fc region of the antibody, and autopolyubiquitination of TRIM21 causes the virus-antibody complex bound to TRIM21 to be guided to the proteasome and degraded (Trends Immunol. 2017 Dec; 38(12):916-926). This results in degradation of the virus-bound antibody as well as the virus.

On the other hand, antibodies that act on cytosolic antigens can be expected to have a prolonged cytosolic half-life if mutations that attenuate the TRIM21-binding ability of antibody are identified. The prolonged cytosolic half-life of an antibody allows the antibody to accumulate more within the cytosol and be more effective on the cytosolic antigen (Fig. 1a).

Furthermore, Dean Clift *et al.* have reported that microinjection of antibodies into the cytosol can cause TRIM21-mediated degradation of antigens within the cytosol (Cell. 2017 Dec 14;171(7):1692-1706.e18). If the ability of antibodies to bind to TRIM21 can be enhanced, guidance of cytosolic antigen-antibody complexes to the proteasome can be improved, accelerating degradation of cytosolic antigens (Fig. 1b).

Accordingly, amino acid mutations were introduced into the antibody constant regions to identify alterations that attenuate or enhance the TRIM21-binding ability. Furthermore, the obtained alterations were introduced into cytosol-penetrating antibodies and tested for prolongation or shortening of the cytosolic half-life of the antibodies.

[Example 2]

Introduction of mutations into antibody and antibody preparation

The heavy chain variable region, heavy chain constant region, light chain variable region, and light chain constant region of the anti-IL6R antibody MRAH-G1d/MRAL-KT0, namely MRAH (SEQ ID NO: 7), G1d (SEQ ID NO: 8), MRAL (SEQ ID NO: 9), KT0 (SEQ ID NO: 10), respectively, were used as templates. Mutations were introduced into the gene encoding the antibody heavy chain constant region by a method known to those skilled in the art to construct expression vectors for variants into which the amino acid alterations shown in Table 3 were introduced (18 variants for each position of alteration). The nucleotide sequences of the obtained expression vectors were determined by a method known to those skilled in the art.

The antibody expression vectors were transiently introduced into Expi293 cells (Thermo Fisher Scientific) to express the antibodies. The antibodies were purified from the obtained culture supernatants by a method known to those skilled in the art using Bravo AssayMAP (Agilent) and Protein A (PA-W) Cartridge (Agilent), or using MabSelect SuRe pcc (5mL) (GE Healthcare) and AKTA Xpress (GE Healthcare). The 280 nm absorbance of each purified antibody was measured using a spectrophotometer, and the concentration of the antibody was calculated from the measured value using an extinction coefficient calculated by the PACE method (Protein Science 1995; 4: 2411-2423). The purified antibodies were concentrated using Amicon Ultra-4 (30 K) (Merck Millipore) when necessary.

[Table 3]

| Position of alteration | Amino acid after alteration | | | | | | | | | | | | | | | | | | |
|---|---|---|---|---|---|---|---|---|---|---|---|---|---|---|---|---|---|---|---|
| I253 | A | D | E | F | G | H | | K | L | M | N | P | Q | R | S | T | V | W | Y |
| L309 | A | D | E | F | G | H | I | K | | M | N | P | Q | R | S | T | V | W | Y |
| Q311 | A | D | E | F | G | H | I | K | L | M | N | P | | R | S | T | V | W | Y |

(continued)

| Position of alteration | Amino acid after alteration | | | | | | | | | | | | | | | | | | |
|---|---|---|---|---|---|---|---|---|---|---|---|---|---|---|---|---|---|---|---|
| D312 | A | | E | F | G | H | I | K | L | M | N | P | Q | R | S | T | V | W | Y |
| L314 | A | D | E | F | G | H | I | K | | M | N | P | Q | R | S | T | V | W | Y |
| N315 | A | D | E | F | G | H | I | K | L | M | | P | Q | R | S | T | V | W | Y |
| E345 | A | D | | F | G | H | I | K | L | M | N | P | Q | R | S | T | V | W | Y |
| M428 | A | D | E | F | G | H | I | K | L | | N | P | Q | R | S | T | V | W | Y |
| L432 | A | D | E | F | G | H | I | K | / | M | N | P | Q | R | S | T | V | W | Y |
| H433 | A | D | E | F | G | | I | K | L | M | N | P | Q | R | S | T | V | W | Y |
| N434 | A | D | E | F | G | H | I | K | L | M | | P | Q | R | S | T | V | W | Y |
| H435 | A | D | E | F | G | | I | K | L | M | N | P | Q | R | S | T | V | W | Y |
| Y436 | A | D | E | F | G | H | I | K | L | M | N | P | Q | R | S | T | V | W | |
| T437 | A | D | E | F | G | H | I | K | L | M | N | P | Q | R | S | | V | W | Y |
| Q438 | A | D | E | F | G | H | I | K | L | M | N | P | | R | S | T | V | W | Y |
| K439 | A | D | E | F | G | H | I | | L | M | N | P | Q | R | S | T | V | W | Y |
| S440 | A | D | E | F | G | H | I | K | L | M | N | P | Q | R | | T | V | W | Y |

[Example 3]

Preparation of human TRIM21 and mouse TRIM21

The PRYSPRY domain of human TRIM21 (hTRIM21) and the PRYSPRY domain of mouse TRIM21 (mTRIM21) were prepared by the following method.

Genes encoding proteins in which a 6x His tag is attached to the N terminus of human TRIM21. PRYSPRY (SEQ ID NO: 5) or mouse TRIM21. PRYSPRY (SEQ ID NO: 6), which is the PRYSPRY domain of human TRIM21 (NCBI accession: NP_003132) or mouse TRIM21 (GenBank accession: CAJ18544), respectively, were each introduced into the pET28a vector, and the resulting expression vectors were each introduced into *E. coli* BL21 (DE3) cells by a method known to those skilled in the art. The two expression cell lines were cultured using 1 L of LB medium containing 50 μg/mL Kanamycin at 37°C with shaking. When OD600 nm reached 0.5 to 0.6, IPTG was added at a final concentration of 0.2 mM. The temperature was then lowered to 18°C and culture was continued for another 18 hours to express TRIM21.

The bacterial cells were collected by centrifugation, and 30 mL of a lysis buffer (50 mM Tris-HCl, pH8.0, 300 mM NaCl, 20 mM imidazole, 10% glycerol, 3 mM β-ME) supplemented with cOmplete Protease Inhibitor Cocktail (Roche) and Benzonase Nuclease (Merck) was added. The cells were then homogenized by sonication. After homogenization, the supernatant was obtained by centrifugation, filtered through a 0.22-μm filter, and then loaded onto a Ni-NTA agarose column (QIAGEN) equilibrated with 50 mM Tris-HCl, pH8.0, 300 mM NaCl, 20 mM imidazole, 10% glycerol, and 3 mM β-ME. After washing the column with an equilibration buffer, the adsorbed protein was eluted by increasing the imidazole concentration to 300 mM.

The eluted fraction was concentrated using AMICON ULTRA, 10 KDa cutoff (Merck), and diluted ten-fold with 20 mM Tris-HCl, pH8.0, 60 mM NaCl, and 0.5 mM TCEP. This was then loaded onto a Mono Q 5/50 GL column (GE Healthcare) equilibrated with the same buffer, and the pass-through fraction was collected as purified TRIM21. The purified TRIM21 was subjected to concentration and solvent exchange with HBS-EP buffer (GE Healthcare) simultaneously using AMICON ULTRA, 10 KDa cutoff. The 280 nm absorbance of the purified protein was measured using a spectrophotometer, and the concentration of the protein was calculated from the measured value using an extinction coefficient calculated by the PACE method (Protein Science 1995; 4: 2411-2423).

[Example 4]

Evaluation of the binding ability of antibodies to TRIM21 and FcRn by surface plasmon resonance (SPR)

Using Amine Coupling Kit (Cat. # BR-1000-50, GE Healthcare), rProtein L (Cat. # 6530-1, BioVision) prepared

at 10 μg/mL in 10 mM sodium acetate buffer pH4.5 (Cat. # BR-1003-50,GE Healthcare) was immobilized on sensor chip Series S CM3 (Cat. # BR-1005-36, GE Healthcare) at approximately 2000 RU per flow cell to produce rProL-CM3 chip.

To examine binding of the antibodies produced in Example 2 to hTRIM21 and mTRIM21, each antibody was prepared at 0.60 μg/mL in HBS-P buffer and allowed to react at 25°C at a flow rate of 10 μL/min for 60 seconds so that approximately 200 RU of the antibody was captured on the rProL-CM3 chip. Next, hTRIM21 or mTRIM21 prepared at 100 nM in HBS-P buffer as an analyte was allowed to act at a flow rate of 30 μL/min for 120 seconds, then the dissociation phase was monitored for 120 seconds to measure the level of binding between hTRIM21 or mouse TRIM21 and the antibody.

In addition, to examine binding to human FcRn (hFcRn), the antibody was prepared at 1.29 μg/mL in a phosphate buffer (0.05 M sodium phosphate, 0.15 M NaCl, pH6.0, 0.05 w/v % P20) (Nacalai Tesque) and allowed to react at 25°C at a flow rate of 10 μL/min for 60 seconds so that approximately 400 RU of the antibody was captured on the rProL-CM3 chip. Next, hFcRn prepared at 1 μM in a phosphate buffer as an analyte was allowed to act at a flow rate of 10 μL/min for 120 seconds, then the dissociation phase was monitored for 120 seconds to measure the level of binding between the antibody and human FcRn. The hFcRn was prepared by the method described in WO2013/046704.

The level of binding between each analyte and the antibody was normalized to the level of the antibody immobilized onto the rProL-CM3 chip. As a result, (1) alterations that attenuated the binding to hTRIM21 and mTRIM21, (2) alterations that attenuated the binding to hTRIM21 and maintained or enhanced the binding to mTRIM21, (3) alterations that attenuated the binding to mTRIM21 and maintained or enhanced the binding to hTRIM21, and (4) alterations that enhanced the binding to hTRIM21 and mTRIM21, were identified. Furthermore, of the alterations falling under (1) to (4) above, alterations that maintained or enhanced the binding to hFcRn or attenuated the binding to hFcRn were identified. The antibody variants prepared in Example 2 were classified according to their effects on binding to hTRIM21, mTRIM21, and hFcRn, and the results are shown in Table 4 (1). The alterations falling under (1) to (4) above are shown in Table 4 (2). The group "Other" in Table 4 (1) indicates a group of antibodies with an undetectable level of binding to hTRIM21 and to mTRIM21. Furthermore, in Table 4 (1), "+" indicates an increased level of binding compared to the control antibody (MRAH-G1d/MRAL-KT0), and "-" indicates a decreased level of binding compared to the control antibody (MRAH-G1d/MRAL-KT0).

[Table 4]

Levels of binding between antibody and hTRIM21, mTRIM21, and hFcRn measured by Biacore

(1) Classification of the antibodies according to their effect on binding to hTRIM21, mTRIM21, and human FcRn

| Group | hTRIM21 binding level | mTRIM21 binding level | hFcRn binding level | Number of variants |
|---|---|---|---|---|
| A | + | + | + | 3 |
| B | + | + | - | 77 |
| C | + | - | + | 0 |
| D | + | - | - | 1 |
| E | - | + | + | 11 |
| F | - | + | - | 130 |
| G | - | - | + | 10 |
| H | - | - | - | 48 |
| Other | | | | 9 |

(2) Comparison between the amino acid alteration-introduced variants and the control antibody (MRAH-G1d/MRAL-KT0) for the level of binding to hTRIM21, mTRIM21, and human FcRn

| Amino acid alteration | hTRIM21 binding level ratio (variant/control antibody) | mTRIM21 binding level ratio (variant/control antibody) | hFcRn binding level ratio (variant/control antibody) | Group |
|---|---|---|---|---|
| I253A | 0.567 | 0.668 | 0.01 | H |
| I253F | 1.453 | 2.311 | 0.00 | B |
| I253H | 0.928 | 1.316 | 0.00 | F |
| I253L | 1.47 | 3.283 | 0.03 | B |

(continued)

| (2) Comparison between the amino acid alteration-introduced variants and the control antibody (MRAH-G1d/MRAL-KT0) for the level of binding to hTRIM21, mTRIM21, and human FcRn | | | | |
|---|---|---|---|---|
| Amino acid alteration | hTRIM21 binding level ratio (variant/control antibody) | mTRIM21 binding level ratio (variant/control antibody) | hFcRn binding level ratio (variant/control antibody) | Group |
| I253M | 1.376 | 2.713 | 0.31 | B |
| I253V | 0.902 | 0.832 | 0.03 | H |
| I253W | 0.912 | 2.354 | 0.00 | F |
| I253Y | 1.461 | 1.249 | 0.00 | B |
| L309A | 0.774 | 2.001 | 0.71 | F |
| L309D | 0.209 | 1.036 | 0.41 | F |
| L309E | 0.229 | 1.278 | 0.02 | F |
| L309F | 0.885 | 2.118 | 0.78 | F |
| L309G | 0.93 | 1.922 | 1.20 | E |
| L309H | 0.831 | 2.107 | 0.06 | F |
| L309I | 0.849 | 1.711 | 0.87 | F |
| L309K | 0.988 | 2.439 | 0.16 | F |
| L309M | 0.831 | 2.217 | 0.88 | F |
| L309N | 0.963 | 2.376 | 0.32 | F |
| L309P | 0.944 | 2.205 | 0.81 | F |
| L309Q | 0.705 | 2.168 | 0.09 | F |
| L309R | 0.924 | 2.512 | 0.26 | F |
| L309S | 1.138 | 2.584 | 0.65 | B |
| L309T | 1.18 | 2.828 | 0.73 | B |
| L309V | 0.806 | 1.625 | 0.21 | F |
| L309W | 0.907 | 2.191 | 1.05 | E |
| L309Y | 0.942 | 2.267 | 0.12 | F |
| Q311A | 0.906 | 2.516 | 0.13 | F |
| Q311D | 0.345 | 1.034 | 0.67 | F |
| Q311E | 0.486 | 1.039 | 0.44 | F |
| Q311F | 1.059 | 2.807 | 0.76 | B |
| Q311G | 1.05 | 3.057 | 0.35 | B |
| Q311H | 1.221 | 2.707 | 0.83 | B |
| Q311I | 0.702 | 2.587 | 0.38 | F |
| Q311K | 0.791 | 2.981 | 1.01 | E |
| Q311L | 0.562 | 1.97 | 1.15 | E |
| Q311M | 0.953 | 2.548 | 1.42 | E |
| Q311N | 0.782 | 2.638 | 0.20 | F |
| Q311P | 1.151 | 2.729 | 0.27 | B |
| Q311R | 1.24 | 2.946 | 0.73 | B |

(continued)

| (2) Comparison between the amino acid alteration-introduced variants and the control antibody (MRAH-G1d/MRAL-KT0) for the level of binding to hTRIM21, mTRIM21, and human FcRn | | | | |
|---|---|---|---|---|
| Amino acid alteration | hTRIM21 binding level ratio (variant/control antibody) | mTRIM21 binding level ratio (variant/control antibody) | hFcRn binding level ratio (variant/control antibody) | Group |
| Q311S | 1.209 | 2.67 | 0.83 | B |
| Q311T | 1.023 | 2.707 | 1.14 | A |
| Q311V | 0.989 | 2.615 | 0.14 | F |
| Q311W | 1.162 | 2.788 | 0.40 | B |
| Q311Y | 1.224 | 2.817 | 0.15 | B |
| D312A | 1.135 | 2.566 | 1.14 | A |
| D312E | 0.933 | 2.351 | 0.50 | F |
| D312F | 1.236 | 2.858 | 0.12 | B |
| D312G | 1.138 | 2.734 | 0.91 | B |
| D312H | 1.288 | 2.858 | 0.85 | B |
| D312I | 1.054 | 2.878 | 0.46 | B |
| D312K | 1.186 | 2.957 | 0.90 | B |
| D312L | 0.769 | 1.985 | 0.03 | F |
| D312M | 1.088 | 2.762 | 0.78 | B |
| D312N | 1.078 | 2.581 | 0.24 | B |
| D312P | 1.109 | 2.961 | 0.20 | B |
| D312Q | 1.115 | 2.849 | 0.16 | B |
| D312R | 1.174 | 2.741 | 0.05 | B |
| D312S | 1.142 | 2.532 | 1.05 | A |
| D312T | 1.108 | 2.748 | 0.94 | B |
| D312V | 0.937 | 2.589 | 0.73 | F |
| D312W | 1.255 | 2.674 | 0.04 | B |
| D312Y | 1.272 | 2.901 | 0.06 | B |
| L314A | 0.717 | 2.308 | 0.01 | F |
| L314D | 0.724 | 0.727 | 0.01 | H |
| L314E | - | - | 0.00 | Other |
| L314F | - | - | 0.27 | Other |
| L314G | - | - | 0.38 | Other |
| L314H | - | - | 0.26 | Other |
| L314I | - | - | 0.90 | Other |
| L314K | - | - | 0.01 | Other |
| L314M | - | - | 0.08 | Other |
| L314N | - | - | 0.00 | Other |
| L314P | 0.989 | 2.048 | 0.02 | F |
| L314Q | 0.896 | 2.153 | 0.01 | F |

(continued)

| (2) Comparison between the amino acid alteration-introduced variants and the control antibody (MRAH-G1d/MRAL-KT0) for the level of binding to hTRIM21, mTRIM21, and human FcRn | | | | |
|---|---|---|---|---|
| Amino acid alteration | hTRIM21 binding level ratio (variant/control antibody) | mTRIM21 binding level ratio (variant/control antibody) | hFcRn binding level ratio (variant/control antibody) | Group |
| L314R | 0.409 | 2.368 | 0.01 | F |
| L314S | 0.98 | 2.086 | 0.44 | F |
| L314T | 1.079 | 1.995 | 0.35 | B |
| L314V | 1.034 | 2.357 | 0.01 | B |
| L314W | 0.777 | 3.01 | 0.27 | F |
| L314Y | 1.108 | 3.135 | 0.02 | B |
| N315A | 0.943 | 2.29 | 0.05 | F |
| N315D | 0.558 | 0.849 | 0.47 | H |
| N315E | 0.61 | 1.185 | 0.40 | F |
| N315F | 0.991 | 2.456 | 0.03 | F |
| N315G | 1.091 | 2.661 | 0.16 | B |
| N315H | 0.88 | 2.394 | 0.41 | F |
| N315I | 1.245 | 2.5 | 0.27 | B |
| N315K | 0.994 | 2.776 | 0.80 | F |
| N315L | 1.127 | 2 | 0.94 | B |
| N315M | 1.296 | 2.386 | 0.88 | B |
| N315P | 1.208 | 2.926 | 0.34 | B |
| N315Q | 1.212 | 2.31 | 0.87 | B |
| N315R | 1.181 | 2.974 | 0.13 | B |
| N315S | 1.181 | 2.697 | 0.13 | B |
| N315T | 1.072 | 2.443 | 0.78 | B |
| N315V | 1.187 | 2.514 | 0.92 | B |
| N315W | 0.982 | 2.384 | 0.95 | F |
| N315Y | 0.941 | 2.449 | 0.06 | F |
| E345A | 0.46 | 2.697 | 0.21 | F |
| E345D | 0.908 | 2.261 | 0.92 | F |
| E345F | 0.496 | 2.434 | 0.92 | F |
| E345H | 0.591 | 2.64 | 0.67 | F |
| E345I | 0.518 | 2.516 | 0.88 | F |
| E345K | 0.096 | 2.496 | 0.83 | F |
| E345L | 0.459 | 2.361 | 0.86 | F |
| E345M | 0.638 | 2.738 | 0.13 | F |
| E345N | 0.665 | 2.759 | 0.79 | F |
| E345P | 0.53 | 2.408 | 0.88 | F |
| E345Q | 0.657 | 2.921 | 0.29 | F |

(continued)

| (2) Comparison between the amino acid alteration-introduced variants and the control antibody (MRAH-G1d/MRAL-KT0) for the level of binding to hTRIM21, mTRIM21, and human FcRn | | | | |
|---|---|---|---|---|
| Amino acid alteration | hTRIM21 binding level ratio (variant/control antibody) | mTRIM21 binding level ratio (variant/control antibody) | hFcRn binding level ratio (variant/control antibody) | Group |
| E345R | 0.073 | 2.367 | 0.76 | F |
| E345S | 0.989 | 2.877 | 0.90 | F |
| E345T | 0.887 | 2.849 | 0.91 | F |
| E345V | 0.422 | 2.334 | 0.92 | F |
| E345W | 0.516 | 2.586 | 0.91 | F |
| E345Y | 0.6 | 2.657 | 0.89 | F |
| M428A | 0.593 | 2.252 | 0.02 | F |
| M428D | 1.272 | 2.699 | 0.24 | B |
| M428E | 1.16 | 1.47 | 0.32 | B |
| M428F | 1.281 | 1.449 | 0.14 | B |
| M428G | 0.674 | 2.239 | 0.32 | F |
| M428H | 1.089 | 1.588 | 0.16 | B |
| M428I | 0.935 | 1.995 | 0.00 | F |
| M428K | 0.66 | 0.878 | 0.31 | H |
| M428L | 1.217 | 2.1 | 0.40 | B |
| M428N | 0.891 | 2.746 | 0.52 | F |
| M428P | 0.476 | 1.797 | 0.00 | F |
| M428Q | 0.919 | 2.022 | 0.30 | F |
| M428R | -0.019 | 0.078 | 0.05 | H |
| M428S | 0.826 | 3.238 | 0.19 | F |
| M428T | 0.951 | 3.409 | 0.41 | F |
| M428V | 1.046 | 2.378 | 0.57 | B |
| M428W | 1.042 | 0.758 | 0.66 | D |
| M428Y | 0.699 | 1.245 | 1.27 | E |
| L432A | 0.575 | 2.377 | 0.08 | F |
| L432D | 0.397 | 0.479 | 0.30 | H |
| L432E | 0.996 | 2.234 | 0.91 | F |
| L432F | 0.765 | 1.019 | 0.65 | F |
| L432G | 0.245 | 1.806 | 0.56 | F |
| L432H | 0.864 | 1.813 | 0.05 | F |
| L432I | 0.657 | 2.294 | 0.59 | F |
| L432K | 0.472 | 2.008 | 0.00 | F |
| L432M | 1.098 | 2.15 | 0.75 | B |
| L432N | 0.462 | 1.606 | 0.57 | F |
| L432P | 1.209 | 2.01 | 0.02 | B |

(continued)

| (2) Comparison between the amino acid alteration-introduced variants and the control antibody (MRAH-G1d/MRAL-KT0) for the level of binding to hTRIM21, mTRIM21, and human FcRn | | | | |
|---|---|---|---|---|
| Amino acid alteration | hTRIM21 binding level ratio (variant/control antibody) | mTRIM21 binding level ratio (variant/control antibody) | hFcRn binding level ratio (variant/control antibody) | Group |
| L432Q | 1.198 | 2.218 | 0.61 | B |
| L432R | 0.099 | 1.134 | 0.00 | F |
| L432S | 0.67 | 2.159 | 0.03 | F |
| L432T | 0.752 | 2.757 | 0.64 | F |
| L432V | 0.848 | 2.674 | 0.17 | F |
| L432W | 0.297 | 0.139 | 0.25 | H |
| L432Y | 0.673 | 0.695 | 0.43 | H |
| H433A | -0.054 | -0.25 | 1.07 | G |
| H433D | -0.009 | -0.188 | 0.48 | H |
| H433E | 0.008 | -0.098 | 0.55 | H |
| H433F | -0.06 | -0.271 | 0.23 | H |
| H433G | 0.015 | -0.15 | 0.87 | H |
| H433I | -0.002 | -0.097 | 0.56 | H |
| H433K | -0.059 | -0.21 | 1.26 | G |
| H433L | -0.016 | -0.18 | 0.63 | H |
| H433M | -0.004 | -0.1 | 0.93 | H |
| H433N | -0.071 | -0.303 | 0.77 | H |
| H433P | -0.019 | -0.204 | 1.20 | G |
| H433Q | -0.005 | -0.127 | 0.96 | H |
| H433R | -0.061 | -0.053 | 1.26 | G |
| H433S | -0.028 | -0.203 | 1.00 | H |
| H433T | -0.007 | -0.116 | 0.57 | H |
| H433V | -0.101 | -0.403 | 0.62 | H |
| H433W | -0.027 | -0.218 | 0.36 | H |
| H433Y | -0.008 | -0.138 | 0.13 | H |
| N434A | 0.081 | -0.382 | 0.96 | H |
| N434D | 0.037 | -0.151 | 0.04 | H |
| N434E | -0.005 | -0.117 | 0.26 | H |
| N434F | 0.12 | -0.205 | 2.13 | G |
| N434G | 0.108 | 0.305 | 1.37 | G |
| N434H | 0.554 | 0.097 | 1.80 | G |
| N434I | 0.632 | 0.569 | 0.03 | H |
| N434K | -0.026 | -0.234 | 0.15 | H |
| N434L | 1.223 | 2.183 | 0.12 | B |
| N434M | 0.824 | 0.771 | 0.27 | H |

(continued)

| (2) Comparison between the amino acid alteration-introduced variants and the control antibody (MRAH-G1d/MRAL-KT0) for the level of binding to hTRIM21, mTRIM21, and human FcRn | | | | |
|---|---|---|---|---|
| Amino acid alteration | hTRIM21 binding level ratio (variant/control antibody) | mTRIM21 binding level ratio (variant/control antibody) | hFcRn binding level ratio (variant/control antibody) | Group |
| N434P | -0.032 | -0.23 | 0.49 | H |
| N434Q | 0.608 | 0.415 | 0.98 | H |
| N434R | 0.399 | -0.045 | 0.32 | H |
| N434S | 0.107 | 0.393 | 1.73 | G |
| N434T | 0.03 | -0.089 | 0.66 | H |
| N434V | 0.388 | 0.101 | 0.84 | H |
| N434W | 0.78 | 0.238 | 2.13 | G |
| N434Y | 0.15 | -0.277 | 0.26 | H |
| H435A | -0.022 | 1.62 | 0.01 | F |
| H435D | 0.007 | 0.208 | 0.03 | H |
| H435E | 0.229 | 0.708 | 0.25 | H |
| H435F | 0.018 | 2.789 | 0.05 | F |
| H435G | -0.008 | 1.28 | 0.03 | F |
| H435I | -0.08 | 0.461 | 0.01 | H |
| H435K | - | - | 3.52 | Other |
| H435L | 0.01 | 1.949 | 0.04 | F |
| H435M | -0.032 | 2.043 | 0.05 | F |
| H435N | -0.021 | 1.145 | 0.23 | F |
| H435Q | 0.005 | 2.061 | 0.03 | F |
| H435S | -0.082 | 1.231 | 0.02 | F |
| H435T | -0.021 | 1.586 | 0.02 | F |
| H435V | -0.003 | 1.347 | 0.03 | F |
| H435W | 0.067 | 1.874 | 0.01 | F |
| H435Y | 0.077 | 1.791 | 0.02 | F |
| Y436A | 0.065 | 0.516 | 0.55 | H |
| Y436D | 0.178 | 1.44 | 0.24 | F |
| Y436E | 0.01 | 0.191 | 0.59 | H |
| Y436F | 1.08 | 2.469 | 0.91 | B |
| Y436G | 0.101 | 1.693 | 0.51 | F |
| Y436H | 0.164 | 0.88 | 0.47 | H |
| Y436I | 0.487 | 1.02 | 1.46 | E |
| Y436K | -0.071 | -0.226 | 0.22 | H |
| Y436L | 0.343 | 0.744 | 1.02 | G |
| Y436M | 0.247 | 0.892 | 0.88 | H |
| Y436N | 0.002 | 0.888 | 0.34 | H |

(continued)

| (2) Comparison between the amino acid alteration-introduced variants and the control antibody (MRAH-G1d/MRAL-KT0) for the level of binding to hTRIM21, mTRIM21, and human FcRn | | | | |
|---|---|---|---|---|
| Amino acid alteration | hTRIM21 binding level ratio (variant/control antibody) | mTRIM21 binding level ratio (variant/control antibody) | hFcRn binding level ratio (variant/control antibody) | Group |
| Y436Q | -0.035 | 0.15 | 0.93 | H |
| Y436R | -0.028 | 0.295 | 0.47 | H |
| Y436S | 0.2 | 1.197 | 0.58 | F |
| Y436T | 1.08 | 1.994 | 0.94 | B |
| Y436V | 0.122 | 0.483 | 0.79 | H |
| Y436W | 0.63 | 1.758 | 0.60 | F |
| T437A | 1.015 | 2.411 | 0.96 | B |
| T437D | 1.336 | 2.186 | 0.91 | B |
| T437E | 1.191 | 1.961 | 0.95 | B |
| T437F | 0.728 | 2.177 | 0.84 | F |
| T437G | 1.049 | 2.65 | 0.21 | B |
| T437H | 0.655 | 2.176 | 0.93 | F |
| T437K | 0.11 | 2.177 | 0.96 | F |
| T437L | 0.233 | 2.148 | 0.86 | F |
| T437M | 0.554 | 2.032 | 0.91 | F |
| T437N | 1.089 | 2.375 | 0.95 | B |
| T437P | -0.024 | -0.129 | 0.03 | H |
| T437Q | 0.634 | 2.161 | 0.98 | F |
| T437R | 0.068 | 2.251 | 0.94 | F |
| T437S | 0.852 | 2.515 | 0.97 | F |
| T437V | 0.19 | 1.961 | 0.87 | F |
| T437W | 1.026 | 1.75 | 0.82 | B |
| T437Y | 0.746 | 2.273 | 0.84 | F |
| Q438A | 0.886 | 1.972 | 0.92 | F |
| Q438D | 0.845 | 2.6 | 0.77 | F |
| Q438E | 1.353 | 2.402 | 0.84 | B |
| Q438F | 0.742 | 1.579 | 0.34 | F |
| Q438G | 0.632 | 1.993 | 0.85 | F |
| Q438H | 0.649 | 2.11 | 0.38 | F |
| Q438I | 0.735 | 1.848 | 1.07 | E |
| Q438K | 0.389 | 1.831 | 1.04 | E |
| Q438L | 0.887 | 1.886 | 1.12 | E |
| Q438M | 0.971 | 1.8 | 0.95 | F |
| Q438N | 0.564 | 1.547 | 0.78 | F |
| Q438P | 0.419 | 2.081 | 0.05 | F |

(continued)

| (2) Comparison between the amino acid alteration-introduced variants and the control antibody (MRAH-G1d/MRAL-KT0) for the level of binding to hTRIM21, mTRIM21, and human FcRn | | | | |
|---|---|---|---|---|
| Amino acid alteration | hTRIM21 binding level ratio (variant/control antibody) | mTRIM21 binding level ratio (variant/control antibody) | hFcRn binding level ratio (variant/control antibody) | Group |
| Q438R | 0.404 | 1.706 | 0.97 | F |
| Q438S | 0.744 | 2.21 | 0.91 | F |
| Q438T | 0.755 | 2.21 | 0.97 | F |
| Q438V | 0.828 | 1.958 | 1.01 | E |
| Q438W | 0.605 | 1.457 | 0.96 | F |
| Q438Y | 0.924 | 1.859 | 0.91 | F |
| K439A | 1.033 | 1.897 | 0.91 | B |
| K439D | 1.151 | 1.725 | 0.64 | B |
| K439E | 1.086 | 1.619 | 0.90 | B |
| K439F | 0.932 | 1.894 | 0.79 | F |
| K439G | 1.122 | 2.025 | 0.88 | B |
| K439H | 1.103 | 2.173 | 0.89 | B |
| K439I | 0.964 | 2.036 | 0.93 | F |
| K439L | 1.039 | 2.1 | 0.93 | B |
| K439M | 1.086 | 2.199 | 0.92 | B |
| K439N | 1.026 | 1.908 | 0.91 | B |
| K439Q | 1.056 | 2.011 | 0.94 | B |
| K439R | 0.857 | 2.54 | 0.92 | F |
| K439S | 1.021 | 2.009 | 0.90 | B |
| K439T | 1.062 | 2.013 | 0.91 | B |
| K439V | 1.098 | 2.181 | 0.08 | B |
| K439W | 0.948 | 1.775 | 0.08 | F |
| K439Y | 0.984 | 1.998 | 0.59 | F |
| S440A | 0.898 | 2.351 | 0.54 | F |
| S440D | 1.306 | 1.999 | 0.88 | B |
| S440E | 1.172 | 1.541 | 0.89 | B |
| S440F | 1.043 | 2.623 | 0.94 | B |
| S440G | 0.945 | 2.385 | 0.95 | F |
| S440H | 0.755 | 2.303 | 0.96 | F |
| S440I | 1.003 | 2.341 | 0.92 | B |
| S440K | 0.528 | 2.803 | 0.95 | F |
| S440L | 0.96 | 2.274 | 0.93 | F |
| S440M | 0.999 | 2.55 | 0.97 | F |
| S440N | 0.962 | 2.459 | 0.93 | F |
| S440P | 0.905 | 2.179 | 0.95 | F |

(continued)

| (2) Comparison between the amino acid alteration-introduced variants and the control antibody (MRAH-G1d/MRAL-KT0) for the level of binding to hTRIM21, mTRIM21, and human FcRn | | | | |
|---|---|---|---|---|
| Amino acid alteration | hTRIM21 binding level ratio (variant/control antibody) | mTRIM21 binding level ratio (variant/control antibody) | hFcRn binding level ratio (variant/control antibody) | Group |
| S440R | 0.602 | 2.649 | 0.95 | F |
| S440T | 0.98 | 2.45 | 0.92 | F |
| S440V | 0.908 | 2.214 | 0.92 | F |
| S440W | 1.11 | 2.658 | 0.93 | B |
| S440Y | 1.08 | 2.766 | 0.92 | B |
| MRAH-G1d/ MRAL-KT0 | 1 | 1 | 1.00 | - |

[Example 5]

Detection of antibodies that entered the cytosol by an assay using biotin ligase

To assess the effect of modulating the TRIM21-binding ability of an antibody on its cytosolic half-life, the antibody that entered the cytosol was detected, referring to the method described in W.P.R. Verdurmen et al. Journal of Controlled Release 200 (2015) 13-22. More specifically, by the method described below, cells expressing biotin ligase (Bir A) are incubated with an antibody attached to a known Avi tag sequence, which is known to be biotinylated by BirA (Protein Science 1999, 8:921-929). When the antibody entered the cytosol, BirA expressed within the cytosol adds biotin to the Avi tag. By detecting the biotinylated antibody with Streptavidin-HRP, the amount of the antibody present within the cytosol can be evaluated.

(1) Cell-antibody reaction

Based on the results of Example 4, the alterations of I253F, L314K, M428R, N434G, Y436D, and T437V, which attenuate or enhance the binding ability to hTRIM21 and/or mTRIM21 and/or hFcRn, were selected for evaluating antibody degradation within the cytosol. Known alterations attenuating the hTRIM21-binding ability, H433A and H435A, were also evaluated (J. Immunol. 2016 Apr 15; 196(8): 3452-3459). Antibodies were prepared from known cytosol-penetrating antibodies described in WO2016013871 and Biochemical and Biophysical Research Communications 379(2009)314-318, namely, 3D8VH-G1m.Avi/hT4VL-KT0 consisting of heavy chain variable region 3D8VH (SEQ ID NO: 11), heavy chain constant region G1m.Avi (SEQ ID NO: 12), light chain variable region hT4VL (SEQ ID NO: 13), and light chain constant region KT0 (SEQ ID NO: 10), and 3D8VH-G4T1E356K.Avi/hT4VL-KT0 consisting of heavy chain variable region 3D8VH (SEQ ID NO: 11), heavy chain constant region G4T1E356K.Avi (SEQ ID NO: 14), light chain variable region hT4VL (SEQ ID NO: 13), and light chain constant region KT0 (SEQ ID NO: 10), by introducing the selected alterations into the heavy chain constant region by the method of Example 2. Here, an Avi tag-encoding gene was linked to a gene encoding the antibody heavy chain to prepare an antibody with the Avi tag fused to the C terminus of the heavy chain.

A suspension of HeLa cells prepared in Minimum Essential Medium Eagle (Sigma, Cat. # M4655-500ML) containing 10% FBS (Sigma Aldrich, Cat. # 182012-500ML) and Penicillin-Streptomycin (Gibco, Cat. # 15140-122) was seeded into Costar® 48-well cell culture cluster Flat bottom with Lid (Corning, Cat. # 3548) at 250 μL/well (4.5 x $10^4$ cells/mL), and this was cultured overnight at 37°C, 5% $CO_2$. A suspension of Flp-In-CHO cells (Invitrogen) made to overexpress IL6R (GenPept Accession No. NP_000556) by a method known to those skilled in the art (CHO+IL6R cells), which was prepared in Ham's F-12 Nutrient Mix (Gibco, Cat. # 11765-054) containing 10% FBS (Sigma Aldrich, Cat. # 182012-500ML) and Penicillin-Streptomycin (Gibco, Cat. # 15140-122), was seeded into Costar® 48-well cell culture cluster Flat bottom with Lid (Corning, Cat. # 3548) at 250 μL/well (3.0 x $10^4$ cells/mL), and this was cultured overnight at 37°C, 5% $CO_2$.

Next, the Hela cells and CHO cells were transiently transfected with an expression vector for Biotin ligase (BirA) (SEQ ID NO: 15) by a method known to those skilled in the art using Opti-MEM® I (1x) (Gibco, Cat. # 31985-062) and Lipofectamin 3000 kit (Life Technologies, Cat. # L3000-015), and cultured overnight at 37°C, 5% $CO_2$ to express BirA.

After the culture supernatant was removed, a medium containing the antibody (final concentration: 2 μM) and D-Biotin (Sigma Aldrich, Cat. # B4501-10G) (final concentration: 0.1 mM) was added at 225 μL/well, and incubated at 37°C for one hour. The antibody-containing solution was removed using an aspirator, and the cells were washed three times with 250 μL of D-PBS (-). Thereafter, the medium was added at 250 μL/well and incubated at 37°C for two hours or four hours. Then, the medium was removed, and the cells were washed with D-PBS (-). Accutase (Nacalai Tesque, Cat. # 12679-54) was added at 100 μL/well and incubated at 37°C, then the medium was added at 800 μL/well, and the cells were collected in microtubes. After centrifugation (400 x g, 2 minutes), the supernatant was removed, and 800 μL of D-PBS (-) was added. This was followed by centrifugation and removal of the supernatant again. Sample Buffer Solution with 3-Mercapto-1,2-propanediol (Wako, Cat. # 199-16132) was mixed with an equal amount of MQ, and 10 uL of this solution pre-heated to 96°C was added to the collected cells. This was immediately heated at 96°C for 8 minutes. The samples were then cooled on ice, and stored at -20°C.

(2) Detection of biotinylated antibodies

Biotinylated antibodies in the cell lysates prepared in (1) were detected using Simple Western Wes (Protein Simple) and 12-230 kDa Wes Separation Module, 8 x 25 capillary cartridges (Protein Simple, Cat. # SM-W004). The prepared cell lysate was thawed at room temperature, and then centrifuged (15,000 rpm for three minutes) to collect the supernatant. The supernatant was diluted eight-fold with MQ. The diluted supernatant and 5x Fluorescent Master Mix prepared by pre-mixing 20 μL of 10x Sample Buffer, 20 μL of 400 mM DTT, and one vial of Fluorescent Standard, were mixed at a volume ratio of 4:1. The mixture was heated at 95°C for five minutes, and then stirred and stored on ice for use as a measurement sample. Furthermore, the HRP substrate was prepared by mixing 200 μL of Luminol-S and 200 μL of Peroxide.

Three μL of the measurement sample, 10 μL of Antibody Diluent II, 10 μL of Streptavidin-HRP, and 15 μL of HRP substrate were added to rows A, B, C, and E, respectively, of Pre-filled Micro Plate of 12-230 kDa Wes Separation Module, 8 x 25 capillary cartridges, and measurement was conducted. The measurement was carried out using at the same time 5 μL of Biotin Ladder prepared by pre-mixing 16 μL of MilliQ, 22 μL of 10 x Sample Buffer, 2 μL of 400 mM DTT, and one vial of Biotin Ladder, as a molecular weight marker. All reagents used were included in Simple Western Wes (Protein Simple) and 12-230 kDa Wes Separation Module, 8 x 25 capillary cartridges (Protein Simple, Cat. # SM-W004) and Biotin Detection Module for Wes, Peggy Sue or Sally Sue (Cat. # DM-004).

The measurement by Wes was performed as follows: Separation Matrix Load Time, 200 seconds; Stacking Matrix Load Time, 15 seconds; Sample Load Time, 9 seconds; Separation Time, 25 seconds; Separation voltage, 375 V; Standards Exposure, 4 seconds; EE Immobilization Time, 230 seconds; Matrix washes, 3 times; Matrix Wash Soak Time, 150 seconds; Wash Soak Time, 150 seconds, Antibody Diluent Time, 5 minutes; Primary Antibody Time, 30 minutes; Washes, twice; Wash Soak Time, 150 seconds; and Detection Profile, HDR.

In addition, β-Actin (13E5) Rabbit mAb (HRP conjugate) (Cell Signaling, Cat. #5125S) diluted 50-fold with Antibody Diluent II was used instead of Streptavidin-HRP to detect actin contained in the measurement sample. In this case, the supernatant collected from the cell lysate that was thawed at room temperature and centrifuged (15,000 rpm for three minutes) was diluted 2-fold with MQ and used to prepare a measurement sample.

The detected HRP signals were analyzed using Compass for Simple Western Version 3.1.7 (Protein Simple), and the lane images as shown in Figures 2 to 5 were generated.

As a result, for 3D8VH-G4T1E356K.Avi/hT4VL-KT0 with H433A or H435A, a known alteration attenuating the hTRIM21-binding ability, introduced into the heavy chain constant region, the antibody biotinylated by BirA was detected in the cell lysates collected two hours after antibody removal from the CHO cells and Hela cells that had been incubated with the antibody for one hour. The detected approximately 60-kDa protein, corresponding to the antibody heavy chain, showed a higher HRP luminescence signal than the unaltered 3D8VH-G4T1E356K.Avi/hT4VL-KT0 (Figs. 2 and 3). This suggests that introduction of an alteration attenuating the TRIM21-binding ability into the heavy chain constant region resulted in more accumulation of the cytosol-penetrating antibody within the cytosol.

Furthermore, for 3D8VH-G1m.Avi/hT4VL-KT0 with an alteration attenuating or enhancing the hTRIM21-binding ability selected based on the results of Example 4, the antibody biotinylated by BirA was detected in the cell lysates collected two hours and four hours after one-hour incubation of CHO cells and Hela cells. For unaltered 3D8VH-G1m.Avi/hT4VL-KT0, an approximately 60-kDa protein corresponding to the antibody heavy chain was detected two hours after but not four hours after removal of the antibody, suggesting that the antibody that entered the cytosol was degraded. On the other hand, the M428R- and I253F-introduced antibodies were detected even after four hours (Figs. 4 and 5).

[Example 6]

Fluorescence microscopic imaging analysis of antibodies within the cytosol

Imaging analysis was carried out using a fluorescence microscope for the known cell-penetrating antibody 3D8VH-G1m.Avi/hT4VL-KT0 (heavy chain variable region 3D8VH (SEQ ID NO: 11), heavy chain constant region G1m.Avi (SEQ ID NO: 12), light chain variable region hT4VL (SEQ ID NO: 13), and light chain constant region KT0 (SEQ ID NO: 10)), and antibodies prepared by introducing selected alterations into its heavy chain constant region by the method of Example 2.

A HeLa cell suspension prepared in Minimum Essential Medium Eagle (Sigma, Cat. # M4655-500ML) containing 10% FBS (Sigma Aldrich, Cat. # 182012-500ML) and Penicillin-Streptomycin (Gibco, Cat. # 15140-122) was seeded at 50 μL/well (6.0 x $10^5$ cells/mL) in a 96-well plate coated with type I collagen on the bottom (Corning, Cat. # 354649), and cultured overnight at 37°C, 5% $CO_2$. On the following day, the culture supernatant was removed, and a medium containing the antibody (final concentration: 3 μM) was added at 30 μL/well, and this was incubated at 37°C for an hour. Next, the antibody-containing medium was removed by aspiration, replaced with 50 μL/well of antibody-free medium, and this was incubated at 37°C for 0.5 hours, 1 hour, or 2 hours. Samples to be subjected to fixation treatment at this point proceeded directly to the washing step. After the prescribed incubation step, the medium was removed from the sample, and this was washed with D-PBS(-). Furthermore, to remove antibodies remaining on the cellular membrane, washing was conducted for 30 seconds twice with a washing solution consisting of 200 mM Glycine (Wako Pure Chemical Industries, Cat. # 077-00735) - HCl (Wako Pure Chemical Industries, Cat. # 083-01095) and 150 mM NaCl (Wako Pure Chemical Industries, Cat. # 191-01665), pH 2.5. Then, the cells were fixed by reaction with 4% paraformaldehyde-phosphate buffer (Nacalai Tesque, Cat. # 09154-56) at room temperature for 15 minutes. The cells were then permeabilized by incubation overnight at 4°C in PBS containing 0.5% Triton X-100 (Bio-Rad, Cat. # 161-0407) and 5% FBS. The solution used for permeabilization was also used in the subsequent steps for incubating with the labeled antibody and washing. The antibodies that entered the cells were labeled by incubation with 250-fold diluted Goat Anti-Human IgG-Alexa Fluor 488 (SouthernBiotech, Cat. # 2040-30) for 1 hour at 4°C. In this incubation, HCS CellMask Deep Red (Thermo, Cat. # H32721) diluted 10000 times was also added to visualize the cells. Finally, the cells were washed twice with PBS and used as a measurement sample. The sample was measured using IN Cell Analyzer 6000 (manufactured by GE Healthcare).

As a result, compared to unaltered 3D8VH-G1m.Avi/hT4VL-KT0, antibodies with the M428R or I253F alteration showed high fluorescence signals immediately after incubation of cells with the antibody, suggesting an increase in the amount of accumulation within the cytosol (Fig. 6). Furthermore, as a result of observing the time course of fluorescence after incubating the cells with the antibodies, the M428R or I253F-introduced antibody showed higher fluorescent signals than the unaltered antibody even after one hour.

The above demonstrated that introduction of an alteration attenuating the TRIM21-binding ability into a cytosol-penetrating antibody can suppress its TRIM21-dependent degradation within the cytosol, and prolong its cytosolic half-life. It was also demonstrated that introduction of the I253F alteration into a cytosol-penetrating antibody can also extend its cytosolic half-life. By using the present disclosure, it is possible to allow antibodies capable of entering the cytosol, such as antibody-CPP complexes and cytosol-penetrating antibodies, to accumulate more within the cytosol and extend their time of action, thereby reinforcing their effects on antigens (cytosolic antigens).

[Example 7]

Induction of degradation of cytosolic antigens by cytosol-penetrating antibodies

Fluorescence microscopic imaging analysis is performed to verify that cytosol-penetrating antibodies with an enhanced TRIM21-binding ability induce proteasomal degradation of antigen within the cytosol by recruiting cytosolic antigens to the proteasome.

Bispecific antibody 3D8//aGFP, which has the cytosol-penetrating domain of the known cytosol-penetrating antibody 3D8 described in Example 5 and the antigen-binding portion of an anti-GFP antibody, is prepared by a method known to those skilled in the art. Antibodies with selected alterations introduced into the heavy chain constant region are also prepared by the method described in Example 2.

GFP-HeLa cells are prepared by allowing Hela cells to transiently express GFP by a method known to those skilled in the art, and cultured in Minimum Essential Medium Eagle containing 10% FBS and Penicillin-Streptomycin. A suspension of GFP-HeLa cells is prepared, and seeded at 50 μL/well (6.0 x $10^5$ cells/mL) in a 96-well plate coated with type I collagen on the bottom. The cells are cultured overnight at 37°C, 5% $CO_2$. On the following day, the culture supernatant is removed, and a medium containing the antibody (final concentration: 3 μM) is added at 30 μL/well and incubated at 37°C for 0.5 hours to 16 hours. The cells are collected at an arbitrary time point, and the fluorescence of GFP is detected using IN Cell Analyzer 6000. Alternatively, after the antibody is added, the cells are cultured at 37°C, 5% $CO_2$, and changes in the fluorescence signal of GFP over time are observed in a time-lapse manner using a fluorescence microscope.

As a result, compared to the cells to which 3D8//aGFP not altered to enhance the TRIM21 binding is added, the cells to which altered 3D8//aGFP is added show lower GFP fluorescence signals at the same time points. Furthermore, in the observation of the GFP fluorescence signal over time, the cells to which altered 3D8//aGFP is added show a decrease in the GFP fluorescence signal in a shorter period of time than the cells to which non-altered 3D8//aGFP is added.

The above shows that TRIM21-dependent degradation of cytosolic antigens within the cytosol can be accelerated by cytosol-penetrating antibodies altered to enhance the TRIM21-binding ability.

[Example 8]

<u>Evaluation of cell growth inhibition ability of cytosol-penetrating antibodies containing an alteration that enhances binding to TRIM21 and having an ability to bind to KRAS</u>

Selected alterations are introduced into the heavy chain constant region of RT11, a known antibody showing a cytosol-penetrating ability and an ability to bind to activated KRAS (Nat. Commun. 2017 May 10;8:15090), by the method of Example 2. The prepared cytosol-penetrating antibodies with an enhanced TRIM21-binding ability are tested for the ability to induce degradation of the KRAS protein, a cytosolic antigen, by recruiting the antigen to the proteasome, and thereby enhance the cell growth inhibition activity.

Hela cells, PAC-1 cells (KRAS G12D mutant), and NIH3T3 cells are cultured overnight in DMEM medium containing 10% FBS and Penicillin-Streptomycin at 37°C, 5% $CO_2$. SW480 cells (KRAS G12V mutant), LoVo cells (KRAS G13D mutant), Colo320DM cells, AsPC-1 cells (KRAS G12D mutant), HT1080 cells (NRAS Q61K mutant), and H1299 cells (NRAS Q61K mutant) are cultured overnight in RPMI1640 medium containing 10% FBS and Penicillin-Streptomycin at 37°C, 5% $CO_2$.

The cell cultures are seeded at 200 μL/well (1 x $10^4$ cells/well) onto a 24-well plate coated with type I collagen on the bottom, and incubated overnight at 37°C, 5% $CO_2$. On the following day, the culture supernatant is removed, and a medium containing the RT11 antibody (final concentration: 1, 5, 10, 15, 20, or 40 μM) is added at 200 μL/well, and incubated at 37°C. The culture supernatant is removed 72 hours later, and a medium containing the RT11 antibody of the same concentration is added again at 200 μL/well. Three days later, the cells are stained with trypan blue, and cell viability is calculated. The ratio of the cell viability in the case of adding antibody RT11 to the viability where PBS is added is calculated as a ratio of cell growth inhibition by the RT11 antibody. Here, antibodies prepared by introducing alterations that attenuate or enhance the binding to TRIM21 into the heavy chain constant region of antibody RT11 by the method described in Example 2 are also used. The RT11 antibody altered to attenuate or enhance the binding to TRIM21 shows a statistically significantly higher cell growth inhibition ratio than the unaltered RT11 antibody.

Accordingly, in cytosol-penetrating antibodies altered to attenuate or enhance the binding to TRIM21, their effects on cytosolic antigens can be enhanced by their facilitated accumulation within the cytosol and extended cytosolic half-life.

[Example 9]

Tm evaluation of altered antibodies by differential scanning fluorometry

Alteration of antibody Fc region is known to have adverse effects on the physical properties of antibodies. For example, an Fc region altered to have enhanced ADCC activity has been reported to show a decrease in thermal denaturation midpoint by approximately 20°C (Biol. Crystallogr. 2008 Jun;64(Pt 6):700-4). It has also been reported that an Fc region altered to have decreased ADCC activity shows a decrease in thermal denaturation midpoint by approximately 5°C, is easily degraded by hydrolases and easily degraded under acidic conditions (Immunol. Lett. 2006 Aug;106(2):144-53; Pharm. Res. 2008 Aug;25(8):1881-90; and Biochem. Biophys. Res. Commun. 2006 Mar;341(3):797-803). Furthermore, an Fc region altered to improve retention in blood has been reported to show decreased thermal stability and storage stability (WO2007092772). Mutation of the TRIM21-binding site in the antibody Fc region might also decrease its thermal stability. Thus, alterations were introduced into the heavy chain constant region of a known cell-penetrating antibody, 3D8VH-G1m /hT4VL-KT0.Avi (heavy chain variable region 3D8VH (SEQ ID NO: 11), heavy chain constant region G1m (SEQ ID NO: 16), light chain variable region hT4VL (SEQ ID NO: 13), light chain constant region KT0.Avi (SEQ ID NO: 17)), according to the method described in Example 2, and the prepared altered antibodies were evaluated for the denaturation temperature (Tm) by differential scanning fluorometry using Rotor-Gene Q (QIAGEN). This technique has been already reported to show good correlation with Tm evaluation using a differential scanning calorimeter, which is a widely known method for evaluating thermal stability of antibody (Journal of Pharmaceutical Science 2010;4:1707-1720).

Measurement samples were prepared by diluting 5000x SYPRO orange (Invitrogen) with PBS (Sigma), and then mixing with the antibody solution. Twenty μL of each sample was set in a measurement tube and the temperature was raised from 30°C to 99°C at a temperature elevation rate of 240°C/hr. Change in fluorescence with increasing temperature was detected at 470 nm (excitation wavelength) / 555 nm (fluorescence wavelength). For the data, the temperature at which fluorescence transition was observed was calculated using Rotor-Gene Q Series Software (QIAGEN), and this value was defined as Tm.

As a result, for the antibodies mutated to attenuate or enhance the TRIM21-binding ability, there were found alterations that resulted in a maintained or increased Tm as compared to the non-mutated antibody (WT), as shown in Table 5 (1) and (2). In each of Table 5 (1) and (2), these alterations were classified into alterations that maintain or increase Tm (Group 1) and other alterations (Group 2).

[Table 5]

| (1) Tm evaluation using differential scanning calorimetry | | |
|---|---|---|
| Amino acid alteration | Tm (°C) | Group |
| WT (3D8VH-G 1m/hT4VL-KT0.Avi) | 70.2 | WT |
| L309D | 72.2 | 1 |
| Q311E | 70.5 | 1 |
| L314K | 64.6 | 2 |
| N315D | 70.3 | 1 |
| M428R | 66.1 | 2 |
| L432R | 61.9 | 2 |
| L432G | 61.5 | 2 |
| H433L | 69.9 | 2 |
| N434G | 69.7 | 2 |
| H435F | 70.6 | 1 |
| Y436D | 65.7 | 2 |
| T437V | 69.4 | 2 |
| T437L | 69.4 | 2 |
| Q438G | 68.6 | 2 |
| S440H | 70.3 | 1 |
| (2) Tm evaluation using differential scanning calorimetry | | |
| Amino acid alteration | Tm (°C) | Group |
| WT (3D8VH-G1m/hT4VL-KT0.Avi) | 69.9 | WT |
| I253F | 70.2 | 1 |
| I253L | 71.1 | 1 |
| I253M | 70.5 | 1 |
| I253Y | 70.1 | 1 |
| D312H | 64.5 | 2 |
| N315M | 70.1 | 1 |
| N315Q | 70.1 | 1 |
| M428F | 72.3 | 1 |
| L432P | 62.9 | 2 |
| Y436T | 68.6 | 2 |

(continued)

| (2) Tm evaluation using differential scanning calorimetry | | |
|---|---|---|
| Amino acid alteration | Tm (°C) | Group |
| T437D | 68.9 | 2 |
| Q438E | 69.9 | 1 |
| I253F.D312H | 64.9 | 2 |
| I253F.N315M | 70.5 | 1 |
| I253F.N315Q | 70.5 | 1 |
| I253F.M428F | 72.6 | 1 |
| I253F.L432P | 63.0 | 2 |
| I253F.Y436T | 68.9 | 2 |
| I253F.T437D | 69.0 | 2 |
| I253F.Q438E | 70.1 | 1 |
| I253L.D312H | 65.9 | 2 |
| I253L.N315M | 71.1 | 1 |
| I253L.N315Q | 71.4 | 1 |
| I253L.M428F | 73.4 | 1 |
| I253L.L432P | 64.2 | 2 |
| I253L.Y436T | 69.7 | 2 |
| I253L.T437D | 69.9 | 1 |
| I253L.Q438E | 71.1 | 1 |
| I253M.D312H | 65.1 | 2 |
| I253M.N315M | 70.9 | 1 |
| I253M.N315Q | 71.0 | 1 |
| I253M.M428F | 73.1 | 1 |
| I253M.L432P | 63.7 | 2 |
| I253M.Y436T | 69.3 | 2 |
| I253M.T437D | 69.5 | 2 |
| I253M.Q438E | 70.7 | 1 |
| I253Y.D312H | 64.6 | 2 |
| I253Y.N315M | 70.2 | 1 |
| I253Y.N315Q | 70.5 | 1 |
| I253Y.M428F | 72.6 | 1 |
| I253Y.L432P | 63.1 | 2 |
| I253Y.Y436T | 68.6 | 2 |
| I253Y.T437D | 68.9 | 2 |
| I253Y.Q438E | 70.1 | 1 |
| D312H.M428F | 67.3 | 2 |
| D312H.L432P | 57.3 | 2 |
| D312H.Y436T | 63.3 | 2 |

(continued)

| (2) Tm evaluation using differential scanning calorimetry | | |
|---|---|---|
| Amino acid alteration | Tm (°C) | Group |
| D312H.T437D | 63.5 | 2 |
| D312H.Q438E | 64.9 | 2 |
| N315M.M428F | 72.6 | 1 |
| N315M.L432P | 62.9 | 2 |
| N315M.Y436T | 68.7 | 2 |
| N315M.T437D | 69.0 | 2 |
| N315M.Q438E | 70.3 | 1 |
| N315Q.M428F | 72.6 | 1 |
| N315Q.L432P | 63.0 | 2 |
| N315Q.Y436T | 68.9 | 2 |
| N315Q.T437D | 68.9 | 2 |
| N315Q.Q438E | 70.2 | 1 |

[Example 10]

Measurement of affinity of antibodies for hTRIM21 by surface plasmon resonance (SPR)

Using Amine Coupling Kit (Cat. # BR-1000-50, GE Healthcare), rProtein L (Cat. # 6530-1, BioVision) prepared at 10 μg/mL in 10 mM sodium acetate buffer pH4.5 (Cat. # BR-1003-50,GE Healthcare) was immobilized on sensor chip Series S CM3 (Cat. # BR-1005-36, GE Healthcare) at approximately 2500 RU per flow cell to prepare rProL-CM3 chip.

To examine the binding of antibodies prepared by the method described in Example 2 to hTRIM21, each antibody prepared at 0.50 μg/mL in HBS-P buffer was allowed to react at 25°C at a flow rate of 10 μL/min for 60 seconds so that the antibody was captured on the rProL-CM3 chip. Furthermore, hTRIM21 prepared at 200 nM, 100 nM, 50 nM, 25 nM, and 12.5 nM in HBS-P buffer as an analyte was serially allowed to act at a flow rate of 30 μL/min for 180 seconds each, then the dissociation phase was monitored for 300 seconds. The binding affinity between the antibody and hTRIM21 and the binding level of hTRIM21 per capture level of the antibody (Binding / Capture) were calculated using Biacore T200 Evaluation Software Version 2.0 (GE Healthcare) (Table 6).

[Table 6]

| Biacore evaluation of the hTRIM21-binding ability of antibodies prepared by altering the heavy chain constant region of cytosol-penetrating antibody 3D8VH-G1m/hT4VL-KT0.Avi | | | | |
|---|---|---|---|---|
| Amino acid alteration | ka (1/Ms) | kd (1/s) | KD (M) | Binding/Capture |
| WT (3D8VH-G1m/hT4VL-KT0.Avi) | 2.15.E+06 | 1.09.E-01 | 5.05.E-08 | 0.255 |
| L309D | 1.23E+05 | 2.88E-03 | 2.34E-08 | 0.052 |
| Q311E | 1.47E+05 | 2.88E-03 | 1.96E-08 | 0.116 |
| L314K | 1.52E+05 | 3.54E-03 | 2.33E-08 | 0.044 |
| N315D | 1.28E+05 | 3.10E-03 | 2.43E-08 | 0.135 |
| E345K | N.D. | N.D. | N.D. | 0.012 |
| M428R | N.D. | N.D. | N.D. | -0.017 |
| L432G | N.D. | N.D. | N.D. | 0.036 |
| L432R | N.D. | N.D. | N.D. | -0.035 |

(continued)

| Biacore evaluation of the hTRIM21-binding ability of antibodies prepared by altering the heavy chain constant region of cytosol-penetrating antibody 3D8VH-G1m/hT4VL-KT0.Avi | | | | |
|---|---|---|---|---|
| Amino acid alteration | ka (1/Ms) | kd (1/s) | KD (M) | Binding/Capture |
| H433A | N.D. | N.D. | N.D. | -0.035 |
| H433L | N.D. | N.D. | N.D. | -0.038 |
| N434G | N.D. | N.D. | N.D. | 0.005 |
| H435A | N.D. | N.D. | N.D. | -0.028 |
| H435F | N.D. | N.D. | N.D. | -0.018 |
| T437L | 1.29E+05 | 3.40E-03 | 2.63E-08 | 0.067 |
| T437V | 1.21E+05 | 3.29E-03 | 2.72E-08 | 0.074 |
| Y436D | 1.51.E+05 | 3.60.E-03 | 2.38.E-08 | 0.056 |
| Q438G | 5.06.E+06 | 4.50.E-01 | 8.90.E-08 | 0.160 |
| S440H | 4.85.E+06 | 2.65.E-01 | 5.46.E-08 | 0.183 |
| I253F | 2.83.E+06 | 7.65.E-03 | 2.70.E-09 | 0.324 |
| I253L | 1.86.E+06 | 1.55.E-03 | 8.34.E-10 | 0.211 |
| I253M | 2.66.E+06 | 1.22.E-02 | 4.56.E-09 | 0.305 |
| I253Y | 2.83.E+06 | 8.06.E-03 | 2.85.E-09 | 0.310 |
| D312H | 2.75.E+06 | 3.89.E-02 | 1.41.E-08 | 0.305 |
| N315M | 2.09.E+06 | 2.94.E-02 | 1.41.E-08 | 0.299 |
| N315Q | 2.03.E+06 | 2.73.E-02 | 1.34.E-08 | 0.308 |
| M428F | 1.90.E+06 | 2.64.E-02 | 1.39.E-08 | 0.283 |
| L432P | 1.72.E+06 | 3.79.E-02 | 2.20.E-08 | 0.419 |
| Y436T | 1.37.E+06 | 2.42.E-02 | 1.77.E-08 | 0.308 |
| T437D | 2.40.E+06 | 3.00.E-02 | 1.25.E-08 | 0.297 |
| Q438E | 2.87.E+06 | 3.19.E-02 | 1.11.E-08 | 0.311 |
| I253F.D312H | 2.06.E+06 | 2.42.E-03 | 1.17.E-09 | 0.388 |
| I253F.N315M | 2.17.E+06 | 1.74.E-03 | 8.03.E-10 | 0.353 |
| I253F.N315Q | 2.17.E+06 | 2.24.E-03 | 1.03.E-09 | 0.400 |
| I253F.M428F | 2.11.E+06 | 1.17.E-03 | 5.53.E-10 | 0.335 |
| I253F.L432P | 2.07.E+06 | 3.86.E-03 | 1.87.E-09 | 0.388 |
| I253F.Y436T | 1.47.E+06 | 2.27.E-03 | 1.55.E-09 | 0.319 |
| I253F.T437D | 2.28.E+06 | 1.92.E-03 | 8.44.E-10 | 0.367 |
| I253F.Q438E | 2.90.E+06 | 1.97.E-03 | 6.82.E-10 | 0.337 |
| I253L.D312H | 1.99.E+06 | 8.39.E-04 | 4.21.E-10 | 0.343 |
| I253L.N315M | 1.78.E+06 | 7.28.E-04 | 4.09.E-10 | 0.370 |
| I253L.N315Q | 2.00.E+06 | 5.61.E-04 | 2.80.E-10 | 0.311 |
| I253L.M428F | 1.77.E+06 | 5.36.E-04 | 3.03.E-10 | 0.327 |
| I253L.L432P | 1.29.E+06 | 1.38.E-03 | 1.07.E-09 | 0.388 |
| I253L.Y436T | 1.10.E+06 | 8.84.E-04 | 8.06.E-10 | 0.318 |

(continued)

| Biacore evaluation of the hTRIM21-binding ability of antibodies prepared by altering the heavy chain constant region of cytosol-penetrating antibody 3D8VH-G1m/hT4VL-KT0.Avi | | | | |
|---|---|---|---|---|
| Amino acid alteration | ka (1/Ms) | kd (1/s) | KD (M) | Binding/Capture |
| I253L.T437D | 1.97.E+06 | 8.86.E-04 | 4.50.E-10 | 0.399 |
| I253L.Q438E | 2.52.E+06 | 6.18.E-04 | 2.45.E-10 | 0.333 |
| I253M.D312H | 2.57.E+06 | 4.19.E-03 | 1.63.E-09 | 0.333 |
| I253M.N315M | 1.81.E+06 | 2.35.E-03 | 1.30.E-09 | 0.384 |
| I253M.N315Q | 1.85.E+06 | 2.49.E-03 | 1.35.E-09 | 0.349 |
| I253M.M428F | 2.25.E+06 | 3.56.E-03 | 1.58.E-09 | 0.336 |
| I253M.L432P | 1.53.E+06 | 2.53.E-03 | 1.66.E-09 | 0.383 |
| I253M.Y436T | 1.10.E+06 | 1.55.E-03 | 1.42.E-09 | 0.391 |
| I253M.T437D | 2.90.E+06 | 3.43.E-03 | 1.18.E-09 | 0.331 |
| I253M.Q438E | 3.31.E+06 | 2.98.E-03 | 9.01.E-10 | 0.330 |
| I253Y.D312H | 2.48.E+06 | 2.44.E-03 | 9.82.E-10 | 0.333 |
| I253Y.N315M | 2.76.E+06 | 2.19.E-03 | 7.91.E-10 | 0.330 |
| I253Y.N315Q | 2.38.E+06 | 2.07.E-03 | 8.70.E-10 | 0.354 |
| I253Y.M428F | 1.87.E+06 | 1.56.E-03 | 8.34.E-10 | 0.385 |
| I253Y.L432P | 1.72.E+06 | 2.99.E-03 | 1.74.E-09 | 0.372 |
| I253Y.Y436T | 1.36.E+06 | 2.50.E-03 | 1.84.E-09 | 0.337 |
| I253Y.T437D | 2.39.E+06 | 2.12.E-03 | 8.89.E-10 | 0.361 |
| I253Y.Q438E | 4.08.E+06 | 2.35.E-03 | 5.76.E-10 | 0.311 |
| D312H.M428F | 2.01.E+06 | 7.47.E-03 | 3.71.E-09 | 0.325 |
| D312H.L432P | 1.83.E+06 | 9.82.E-03 | 5.37.E-09 | 0.673 |
| D312H.Y436T | 1.13.E+06 | 6.98.E-03 | 6.20.E-09 | 0.317 |
| D312H.T437D | 2.27.E+06 | 9.34.E-03 | 4.12.E-09 | 0.327 |
| D312H.Q438E | 2.09.E+06 | 7.41.E-03 | 3.55.E-09 | 0.342 |
| N315M.M428F | 1.66.E+06 | 6.29.E-03 | 3.80.E-09 | 0.321 |
| N315M.L432P | 1.27.E+06 | 6.80.E-03 | 5.33.E-09 | 0.368 |
| N315M.Y436T | 9.97.E+05 | 4.26.E-03 | 4.27.E-09 | 0.290 |
| N315M.T437D | 2.91.E+06 | 7.20.E-03 | 2.48.E-09 | 0.320 |
| N315M.Q438E | 2.90.E+06 | 7.87.E-03 | 2.71.E-09 | 0.314 |
| N315Q.M428F | 1.67.E+06 | 5.99.E-03 | 3.58.E-09 | 0.311 |
| N315Q.L432P | 1.43.E+06 | 7.45.E-03 | 5.20.E-09 | 0.329 |
| N315Q.Y436T | 1.10.E+06 | 6.41.E-03 | 5.82.E-09 | 0.293 |
| N315Q.T437D | 1.95.E+06 | 6.39.E-03 | 3.28.E-09 | 0.355 |
| N315Q.Q438E | 2.93.E+06 | 8.01.E-03 | 2.74.E-09 | 0.313 |
| N.D. means that the binding response was too weak to calculate affinity. | | | | |

[Industrial Applicability]

Antigen-binding molecules comprising an altered TRIM21-binding domain and having an increased cytosolic half-life in the present disclosure can stay within the cytosol for a longer time than conventional antigen-binding molecules, and are useful as antigen-binding molecules for treatment, diagnosis, or detection. Furthermore, antigen-binding molecules comprising an altered TRIM21-binding domain and having a decreased cytosolic half-life can be expected to show an improved ability to remove cytosolic antigens compared to conventional antigen-binding molecules, and are useful as therapeutic antigen-binding molecules.

## Claims

1. A method for producing an antigen-binding molecule comprising an altered TRIM21-binding domain, which comprises introducing one or more amino acid alterations into a TRIM21-binding domain of a parent antigen-binding molecule, wherein the alterations result in a decrease or an increase in the binding affinity of the antigen-binding molecule for TRIM21 compared to that of the parent antigen-binding molecule, and
wherein the amount of the antigen-binding molecule present within the cytosol of a cell after a certain amount of the antigen-binding molecule is contacted with the cell is increased or decreased compared to the amount of the parent anigen-binding molecule present within the cytosol of a cell after the same amount of the parent antigen-binding molecule is contacted with the cell.

2. A method for producing an antigen-binding molecule comprising an altered TRIM21-binding domain, which comprises introducing one or more amino acid alterations into a TRIM21-binding domain of a parent antigen-binding molecule, wherein the alterations result in an increase or a decrease in the binding affinity of the TRIM21-binding domain for TRIM21 compared to that of the parent antigen-binding molecule, and
wherein the antigen-binding molecule has an decreased or increased ability to remove a cytosolic antigen compared to the parent antigen-binding molecule.

3. The method of claim 1 or 2, wherein the alterations further increase or decrease the resistance of the antigen-binding molecule to proteasomal degradation in the cytosol compared to that of the parent antigen-binding molecule.

4. The method of any one of claims 1 to 3, which further comprises:

(a) obtaining an expression vector comprising an appropriate promoter operably linked to a gene encoding the antigen-binding molecule produced by the method of any one of claims 1 to 3;
(b) introducing the vector into a host cell, and culturing the host cell to produce the antigen-binding molecule; and
(c) recovering the antigen-binding molecule from the culture of the host cell.

5. The method of any one of claims 1 to 4, wherein the antigen-binding molecule further comprises an FcRn-binding domain, and the alterations do not substantially change the binding of the FcRn-binding domain to FcRn.

6. The method of any one of claims 1 to 4, wherein the antigen-binding molecule further comprises an FcRn-binding domain, and the alterations increase or decrease the binding of the FcRn-binding domain to FcRn compared to that of the parent antigen-binding molecule.

7. The method of claim 5 or 6, which further comprises introducing one or more amino acid alterations into the FcRn-binding domain, and the alterations result in an increase or a decrease in the binding of the FcRn-binding domain to FcRn compared to that of the parent FcRn-binding domain.

8. The method of any one of claims 1 to 7, wherein the antigen-binding molecule has an ability to penetrate into cytosol.

9. The method of claim 8, wherein the alterations do not remarkably decrease the ability of the antigen-binding molecule to penetrate into cytosol.

10. A method for producing an antigen-binding molecule comprising an altered TRIM21-binding domain, which comprises:

(a) providing a parent antigen-binding molecule having a TRIM21-binding domain;
(b) obtaining a candidate molecule comprising an altered TRIM21-binding domain by introducing one or more amino acid alterations into the TRIM21-binding domain of the parent antigen-binding molecule;
(c) determining the binding affinity of the altered TRIM21-binding domain for TRIM21;
(d) identifying the candidate molecule as a suitable molecule when the altered TRIM21-binding domain binds to TRIM21 with higher or lower binding affinity than the TRIM21-binding domain of the parent antigen-binding molecule;
(e) obtaining an expression vector comprising an appropriate promoter operably linked to a gene encoding the suitable molecule;
(d) introducing the vector into a host cell and culturing the host cell to produce the suitable molecule; and
(e) recovering the suitable molecule from the culture of the host cell;

wherein the suitable molecule has an increased or decreased cytosolic half-life compared to the parent antigen-binding molecule.

11. A method for increasing or decreasing the cytosolic half-life of an antigen-binding molecule compared to that of a parent antigen-binding molecule, which comprises introducing one or more amino acid alterations into a TRIM21-binding domain of the parent antigen-binding molecule, wherein the alterations result in a decrease or an increase in the binding affinity of the TRIM21-binding domain for TRIM21 compared to that of the parent antigen-binding molecule.

12. An antigen-binding molecule comprising an altered TRIM21-binding domain, wherein the altered TRIM21-binding domain comprises one or more amino acid alterations that result in a decrease or an increase in binding affinity for TRIM21 compared to a wildtype TRIM21-binding domain, and wherein the antigen-binding molecule has an increased or decreased cytosolic half-life compared to an antigen-binding molecule comprising a TRIM21-binding domain which does not comprise the alterations.

13. The antigen-binding molecule of claim 12, wherein the altered TRIM21-binding domain comprises an amino acid substitution at one or more positions selected from the group consisting of EU253, EU309, EU311, EU312, EU314, EU315, EU345, EU428, EU432, EU433, EU434, EU435, EU436, EU437, EU438, EU439, and EU440, wherein the numbers indicate the positions of substitution according to EU numbering.

14. The antigen-binding molecule of claim 12 or 13, wherein the altered TRIM21-binding domain comprises one or more amino acid substitutions selected from the group consisting of EU253F, EU314K, EU428R, EU434G, EU436D, and EU437V, wherein the numbers indicate the positions of substitution according to EU numbering.

15. The antigen-binding molecule of any one of claims 12 to 14, which has a cytosol-penetrating ability.

a. Behavior of antibody with attenuated TRIM21-binding ability in cytosol

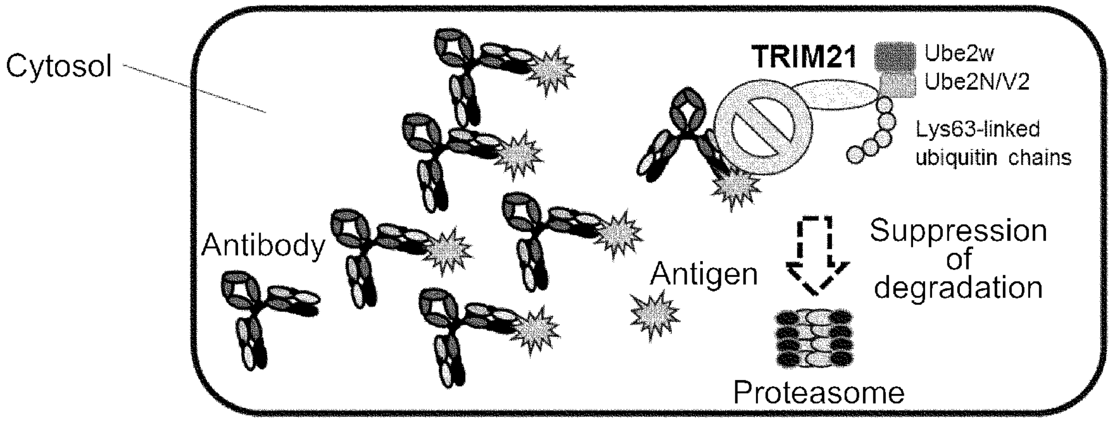


b. Behavior of antibody with enhanced TRIM21-binding ability in cytosol

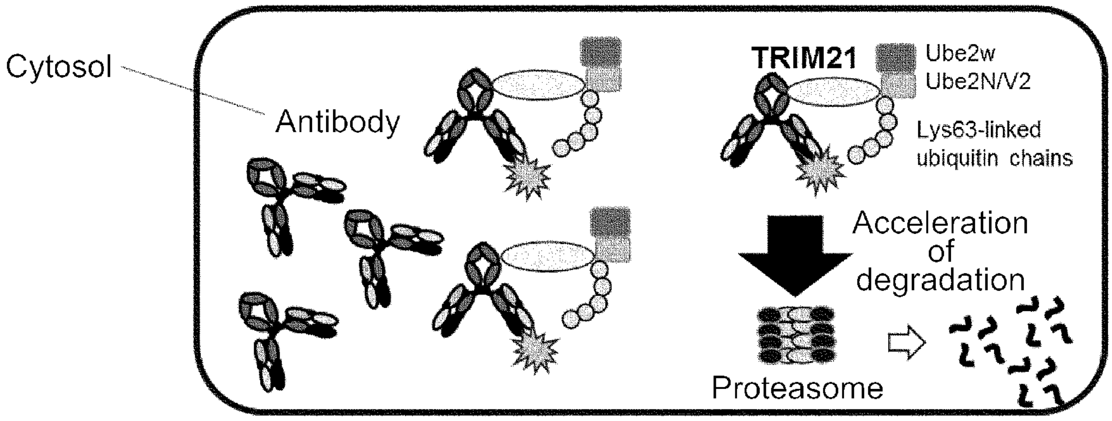


FIG. 1

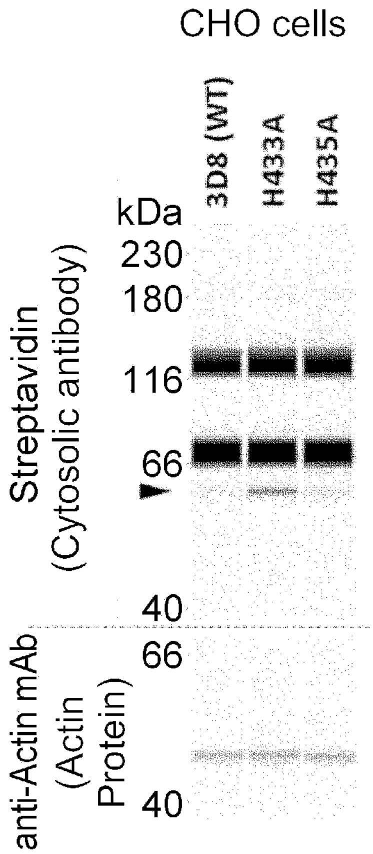
CHO cells
3D8 (WT)
H433A
H435A
kDa
230
180
116
66
40
66
40
Streptavidin
(Cytosolic antibody)
anti-Actin mAb
(Actin
Protein)

FIG. 2

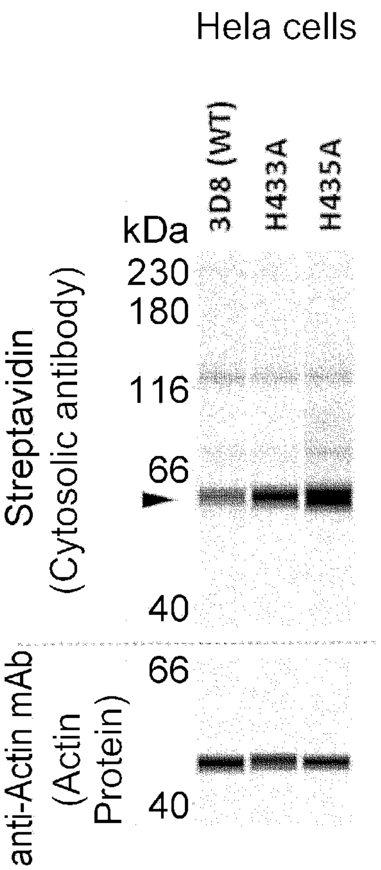
Hela cells
3D8 (WT)
H433A
H435A
kDa
230
180
116
66
40
66
40
Streptavidin
(Cytosolic antibody)
anti-Actin mAb
(Actin
Protein)

FIG. 3

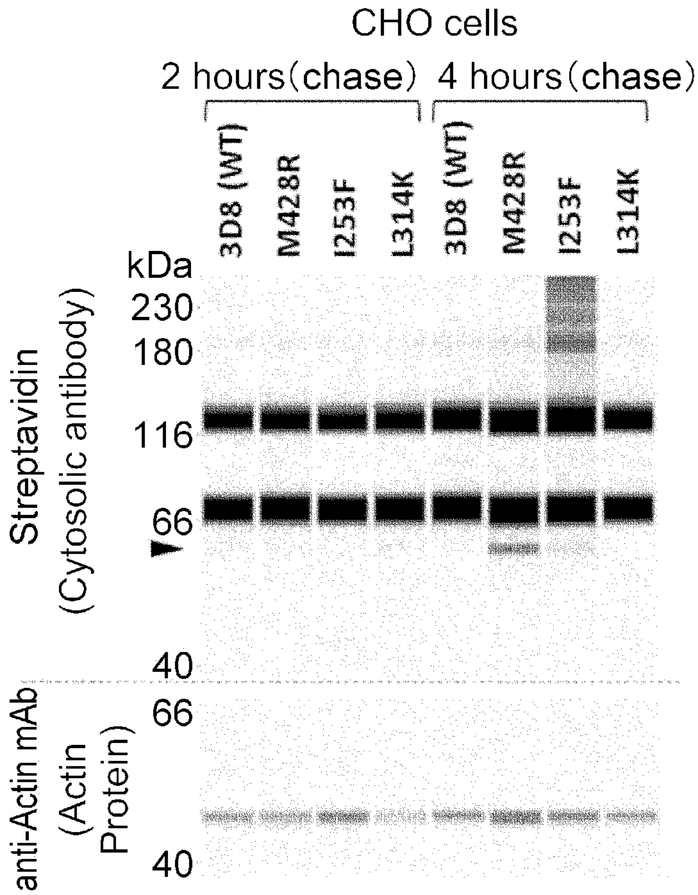
CHO cells
2 hours(chase)
4 hours(chase)
3D8 (WT)
M428R
I253F
L314K
3D8 (WT)
M428R
I253F
L314K
kDa
230
180
116
66
40
Streptavidin (Cytosolic antibody)
anti-Actin mAb (Actin Protein)
66
40

FIG. 4

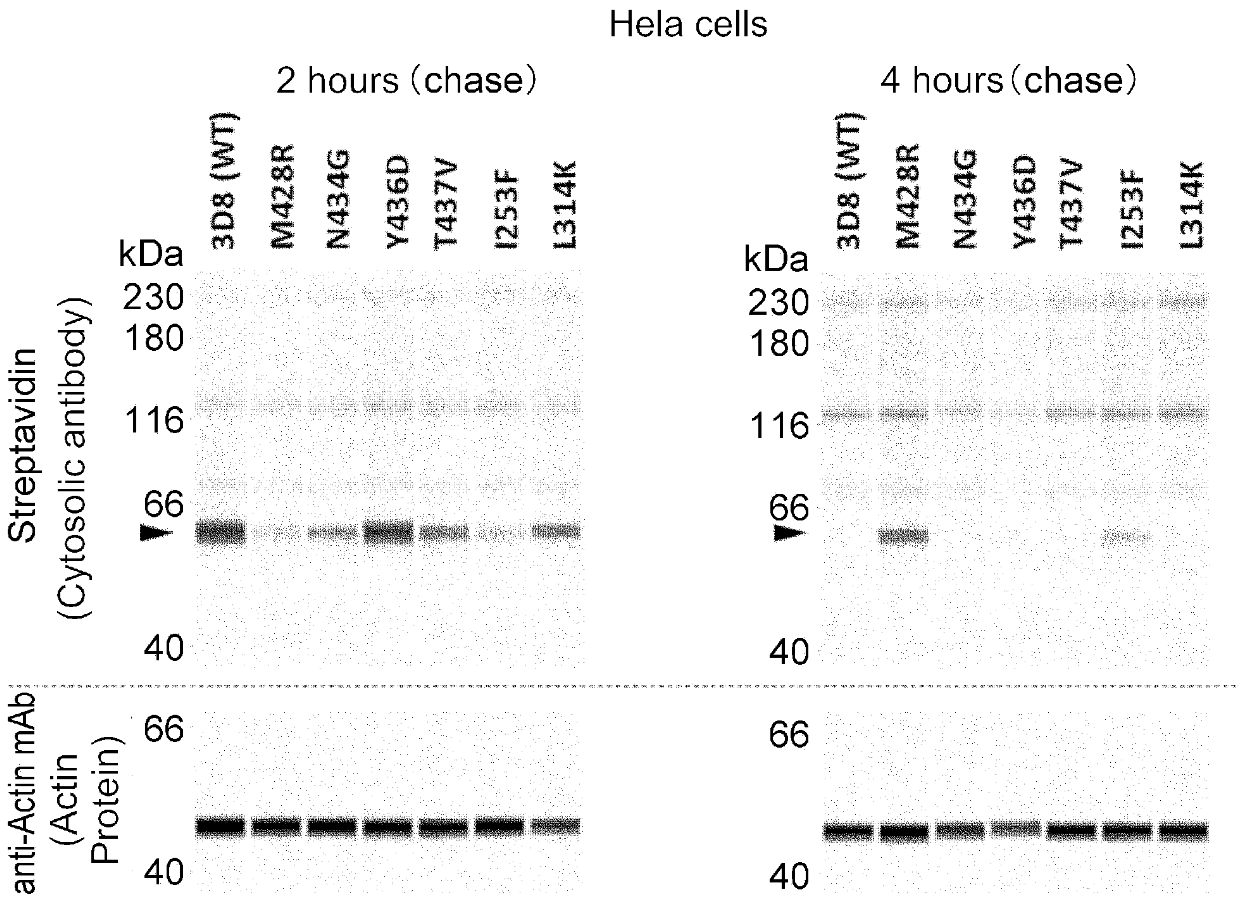
Hela cells
2 hours (chase)
4 hours (chase)
3D8 (WT)
M428R
N434G
Y436D
T437V
I253F
L314K
kDa
230
180
116
66
40
Streptavidin (Cytosolic antibody)
anti-Actin mAb (Actin Protein)

FIG. 5

(1)

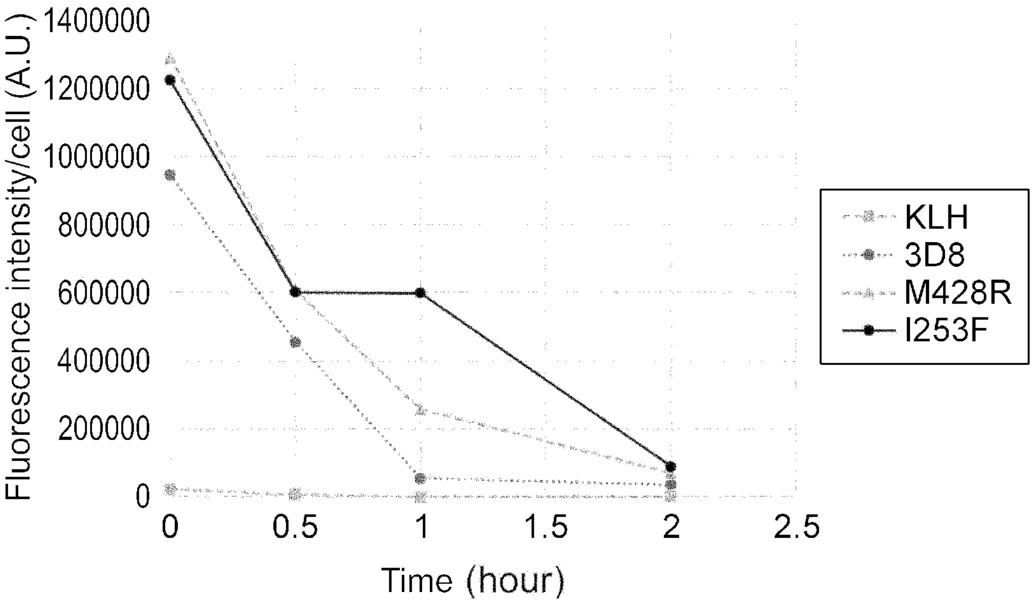
Fluorescence intensity/cell (A.U.)
1400000
1200000
1000000
800000
600000
400000
200000
0
0
0.5
1
1.5
2
2.5
Time (hour)
KLH
3D8
M428R
I253F

(2)

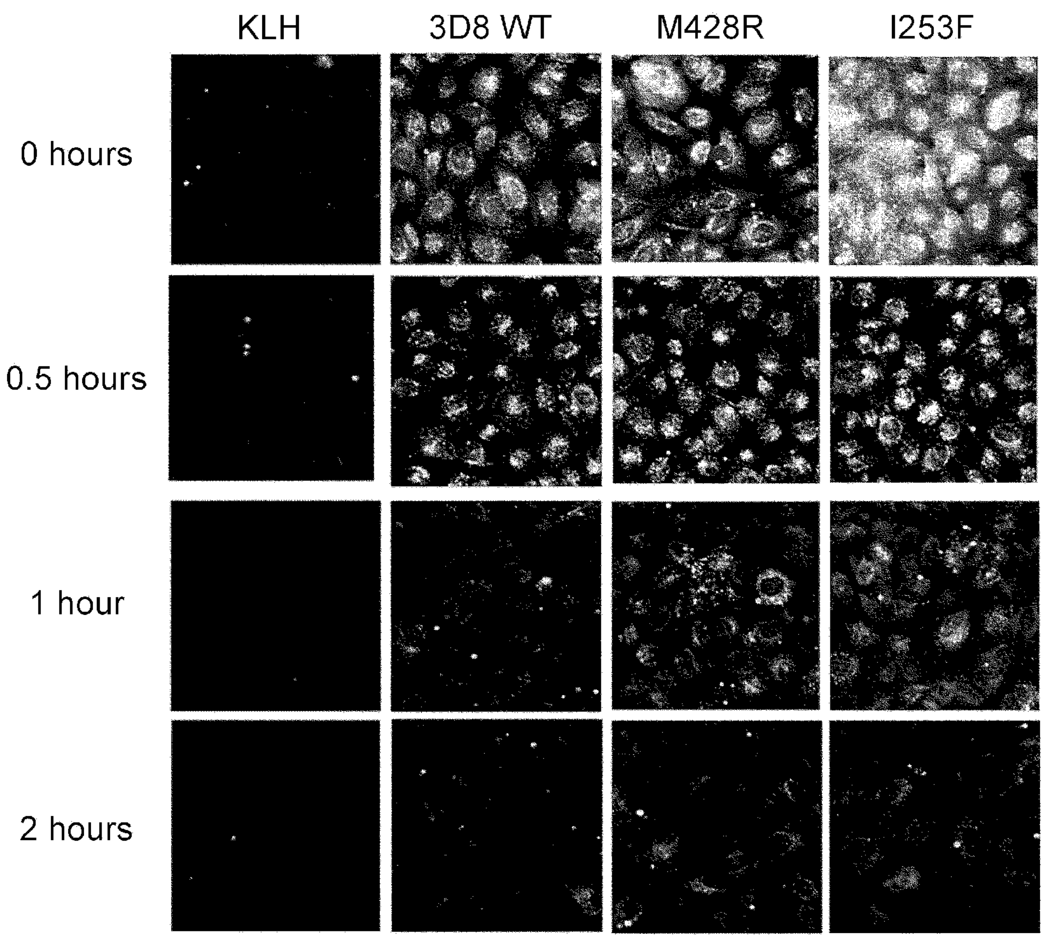
KLH
3D8 WT
M428R
I253F
0 hours
0.5 hours
1 hour
2 hours

FIG. 6

**INTERNATIONAL SEARCH REPORT**

International application No. PCT/JP2019/021984

A. CLASSIFICATION OF SUBJECT MATTER
Int.Cl. C12P21/08(2006.01)i, C07K16/00(2006.01)i, C07K16/28(2006.01)i, C12N15/09(2006.01)i, C12N15/10(2006.01)i

According to International Patent Classification (IPC) or to both national classification and IPC

B. FIELDS SEARCHED

Minimum documentation searched (classification system followed by classification symbols)
Int.Cl. C12P21/08, C07K16/00, C07K16/28, C12N15/09, C12N15/10

Documentation searched other than minimum documentation to the extent that such documents are included in the fields searched

| | |
|---|---|
| Published examined utility model applications of Japan | 1922-1996 |
| Published unexamined utility model applications of Japan | 1971-2019 |
| Registered utility model specifications of Japan | 1996-2019 |
| Published registered utility model applications of Japan | 1994-2019 |

Electronic data base consulted during the international search (name of data base and, where practicable, search terms used)
CAplus/MEDLINE/EMBASE/BIOSIS/WPIDS (STN)

C. DOCUMENTS CONSIDERED TO BE RELEVANT

| Category* | Citation of document, with indication, where appropriate, of the relevant passages | Relevant to claim No. |
|---|---|---|
| X<br>Y | FOSS, Stian et al., "TRIM21 Immune Signaling Is More Sensitive to Antibody Affinity Than Its Neutralization Activity", The Journal of Immunology, 2016, vol. 196, pp. 3452-3459 | 1-10, 12-15<br>11 |
| X<br>Y | US 2018/0037634 A1 (VISWANATHAN, Karthik et al.) 08 February 2018, examples 1, 13, fig. 17A, 26, 27A, etc. & WO 2018/052556 A1 & KR 10-2019-0038607 A | 1-10, 12-15<br>11 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 21 June 2019 (21.06.2019) | 09 July 2019 (09.07.2019) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

INTERNATIONAL SEARCH REPORT

International application No.
PCT/JP2019/021984

C (Continuation). DOCUMENTS CONSIDERED TO BE RELEVANT

| Category* | Citation of document, with indication, where appropriate, of the relevant passages | Relevant to claim No. |
|---|---|---|
| Y | CLIFT, Dean et al., "A Method for the Acute and Rapid Degradation of Endogenous Proteins", Cell, 2017, vol. 172, pp. 1692-1706 | 11 |
| Y | RHODES, A. David et al., "TRIM21 and the Function of Antibodies inside Cells", Trends in Immunology, 2017, vol. 38, no. 12, pp. 916-926 | 11 |
| A | JAMES, C. Leo et al., "Structural basis for PRYSPRY-mediated tripartite motif(TRIM) protein function", PNAS, 2007, vol. 104, no. 15, pp. 6200-6205 | 1-15 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2013102659 A
- WO 2017156630 A
- WO 2015031837 A
- WO 200492219 A
- WO 200042072 A
- WO 2007092772 A
- WO 9316185 A
- US 5571894 A
- US 5587458 A
- US 5869046 A
- EP 404097 A
- WO 199301161 A
- US 6248516 B1
- US 4816567 A
- US 5821337 A
- US 7527791 B
- US 6982321 B
- US 7087409 B
- US 20060025576 A1
- US 20080069820 A
- WO 2008077546 A
- US 20030157108 A
- US 20040093621 A
- WO 200061739 A
- WO 200129246 A
- US 20030115614 A
- US 20020164328 A
- US 20040132140 A
- US 20040110704 A
- US 20040110282 A
- US 20040109865 A
- WO 2003085119 A
- WO 2003084570 A
- WO 2005035586 A
- WO 2005035778 A
- WO 2005053742 A
- WO 2002031140 A
- US 20030157108 A1
- WO 2004056312 A1
- WO 2003085107 A
- WO 2003011878 A, Jean-Mairet
- US 6602684 B, Umana
- US 20050123546 A, Umana
- WO 199730087 A, Patel
- WO 199858964 A, Raju, S.
- WO 199922764 A, Raju, S.
- US 5500362 A
- WO 5821337 A
- WO 2006029879 A
- WO 2005100402 A
- US 6737056 B
- US 7332581 B
- WO 2004056312 A
- US 6194551 B
- WO 9951642 A
- US 20050014934 A1, Hinton
- US 7371826 B
- US 5648260 A
- US 5624821 A
- WO 9429351 A
- US 7521541 B
- US 5648237 A
- US 5789199 A
- US 5840523 A
- US 5959177 A
- US 6040498 A
- US 6420548 B
- US 7125978 B
- US 6417429 B
- WO 2016013870 A
- US 4737456 A
- US 20050260186 A
- US 20060104968 A
- US 6267958 B
- US 6171586 B
- WO 2006044908 A
- WO 2013046704 A
- WO 2016013871 A


### Non-patent literature cited in the description


- *Nat. Rev. Drug Discov.,* March 2018, vol. 17 (3), 197-223
- *MAbs,* January 2011, vol. 3 (1), 3-16
- *Sci. Rep.,* 09 July 2015, vol. 5, 12022
- *Mol. Immunol.,* October 2015, vol. 67 (2), 377-87
- *Trends Immunol.,* December 2017, vol. 38 (12), 916-926
- *J. Immunol.,* 15 April 2016, vol. 196 (8), 3452-3459
- **CARTER PJ ; LAZAR GA.** *Nat Rev Drug Discov.,* March 2018, vol. 17 (3), 197-223

- **MARSCHALL AL ; FRENZEL A ; SCHIRRMANN T ; SCHUNGEL M ; DUBEL S.** *MAbs,* January 2011, vol. 3 (1), 3-16
- **WEISBART RH ; CHAN G ; JORDAAN G ; NOBLE PW ; LIU Y ; GLAZER PM ; NISHIMURA RN ; HANSEN JE.** *Sci Rep.,* 09 July 2015, vol. 5, 12022
- **IM SR ; IM SW ; CHUNG HY ; PRAVINSAGAR P ; JANG YJ.** *Mol Immunol.,* October 2015, vol. 67 (2), 377-87
- **RHODES DA ; ISENBERG DA.** *Trends Immunol.,* December 2017, vol. 38 (12), 916-926
- **FOSS S ; WATKINSON RE ; GREVYS A ; MCADAM MB ; BERN M ; HOYDAHL LS ; DALHUS B ; MICHAELSEN TE ; SANDLIE I ; JAMES LC.** *J Immunol.,* 15 April 2016, vol. 196 (8), 3452-3459
- **KABAT et al.** Sequences of Proteins ofImmunological Interest. National Institutes of Health, 1991
- **KABAT et al.** Sequences of Proteins of Immunological Interest. NIH, 1991, vol. 1-3
- **CHOTHIA ; LESK.** *J. Mol. Biol.,* 1987, vol. 196, 901-917
- **MACCALLUM et al.** *J. Mol. Biol.,* 1996, vol. 262, 732-745
- **FLATMAN et al.** *J. Chromatogr. B,* 2007, vol. 848, 79-87
- **KINDT et al.** Kuby Immunology. W.H. Freeman and Co, 2007, 91
- **PORTOLANO et al.** *J. Immunol.,* 1993, vol. 150, 880-887
- **CLARKSON et al.** *Nature,* 1991, vol. 352, 624-628
- *Nat Immunol.,* April 2013, vol. 14 (4), 327-36
- **DAËRON.** *Annu. Rev. Immunol.,* 1997, vol. 15, 203-234
- **RAVETCH ; KINET.** *Annu. Rev. Immunol,* 1991, vol. 9, 457-92
- **CAPEL et al.** *Immunomethods,* 1994, vol. 4, 25-34
- **DE HAAS et al.** *J. Lab. Clin. Med.,* 1995, vol. 126, 330-41
- **GUYER et al.** *J. Immunol.,* 1976, vol. 117, 587
- **KIM et al.** *J. Immunol.,* 1994, vol. 24, 249
- **GHETIE ; WARD.** *Immunol. Today,* 1997, vol. 18 (12), 592-598
- **GHETIE et al.** *Nature Biotechnology,* 1997, vol. 15 (7), 637-640
- **HINTON et al.** *J. Biol. Chem.,* 2004, vol. 279 (8), 6213-6216
- **SHIELDS et al.** *J. Biol. Chem.,* 2001, vol. 9 (2), 6591-6604
- *Biol. Crystallogr.,* June 2008, vol. 64, 700-4
- *Immunol. Lett.,* 10 August 2006, vol. 6 (2), 144-53
- *Pharm. Res.,* 25 August 2008, vol. 8, 1881-90
- *Biochem. Biophys. Res. Commun.,* March 2006, vol. 341 (3), 797-803
- **HUDSON et al.** *Nat. Med.,* 2003, vol. 9, 129-134
- **PLUCKTHÜN.** The Pharmacology of Monoclonal Antibodies. Springer-Verlag, 1994, vol. 113, 269-315
- **HOLLINGER et al.** *Proc. Natl. Acad. Sci. USA,* 1993, vol. 90, 6444-6448
- **MORRISON et al.** *Proc. Natl. Acad. Sci. USA,* 1984, vol. 81, 6851-6855
- **ALMAGRO ; FRANSSON.** *Front. Biosci.,* 2008, vol. 13, 1619-1633
- **RIECHMANN et al.** *Nature,* 1988, vol. 332, 323-329
- **QUEEN et al.** *Proc. Nat'l Acad. Sci. USA,* 1989, vol. 86, 10029-10033
- **KASHMIRI et al.** *Methods,* 2005, vol. 36, 25-34
- **PADLAN.** *Mol. Immunol.,* 1991, vol. 28, 489-498
- **DALL'ACQUA et al.** *Methods,* 2005, vol. 36, 43-60
- **OSBOURN et al.** *Methods,* 2005, vol. 36, 61-68
- **KLIMKA et al.** *Br. J. Cancer,* 2000, vol. 83, 252-260
- **VAN DIJK ; VAN DE WINKEL.** *Curr. Opin. Pharmacol.,* 2001, vol. 5, 368-74
- **LONBERG.** *Curr. Opin. Immunol.,* 2008, vol. 20, 450-459
- **HOOGENBOOM et al.** Methods in Molecular Biology. Human Press, 2001, vol. 178, 1-37
- **MCCAFFERTY et al.** *Nature,* vol. 348, 552-554
- **CLACKSON et al.** *Nature,* 1991, vol. 352, 624-628
- **MARKS et al.** *J. Mol. Biol.,* 1992, vol. 222, 581-597
- **MARKS ; BRADBURY.** Methods in Molecular Biology. Human Press, 2003, vol. 248, 161-175
- **SIDHU et al.** *J. Mol. Biol.,* 2004, vol. 338 (2), 299-310
- **LEE et al.** *J. Mol. Biol.,* 2004, vol. 340 (5), 1073-1093
- **FELLOUSE.** *Proc. Natl. Acad. Sci. USA,* 2004, vol. 101 (34), 12467-12472
- **LEE et al.** *J. Immunol. Methods,* 2004, vol. 284 (1-2), 119-132
- **CHOWDHURY.** *Methods Mol. Biol.,* 2008, vol. 207, 179-196
- **CUNNINGHAM ; WELLS.** *Science,* 1989, vol. 244, 1081-1085
- **WRIGHT et al.** *TIBTECH,* 1997, vol. 15, 26-32
- **OKAZAKI et al.** *J. Mol. Biol.,* 2004, vol. 336, 1239-1249

- **YAMANE-OHNUKI et al.** *Biotech. Bioeng.,* 2004, vol. 87, 614
- **RIPKA et al.** *Arch. Biochem. Biophys.,* 1986, vol. 249, 533-545
- **KANDA, Y. et al.** *Biotechnol. Bioeng.,* 2006, vol. 94 (4), 680-688
- **RAVETCH ; KINET.** *Annu. Rev. Immunol.,* 1991, vol. 9, 457-492
- **HELLSTROM, I. et al.** *Proc. Nat'l Acad. Sci. USA,* 1986, vol. 83, 7059-7063
- **HELLSTROM, I et al.** *Proc. Nat'l Acad. Sci. USA,* 1985, vol. 82, 1499-1502
- **BRUGGEMANN, M. et al.** *J. Exp. Med.,* 1987, vol. 166, 1351-1361
- **CLYNES et al.** *Proc. Nat'l Acad. Sci. USA,* 1998, vol. 95, 652-656
- **GAZZANO-SANTORO et al.** *J. Immunol. Methods,* 1996, vol. 202, 163
- **CRAGG, M.S. et al.** *Blood,* 2003, vol. 101, 1045-1052
- **CRAGG, M.S. ; M.J. GLENNIE.** *Blood,* 2004, vol. 103, 2738-2743
- **PETKOVA, S.B. et al.** *Int'l. Immunol.,* 2006, vol. 18 (12), 1759-1769
- **IDUSOGIE et al.** *J. Immunol.,* 2000, vol. 164, 4178-4184
- **DUNCAN ; WINTER.** *Nature,* 1988, vol. 322, 738-40
- **KAM et al.** *Proc. Natl. Acad. Sci. USA,* 2005, vol. 102, 11600-11605
- **CHARLTON.** Methods in Molecular Biology. Humana Press, 2003, vol. 248, 245-254
- **GERNGROSS.** *Nat. Biotech.,* 2004, vol. 22, 1409-1414
- **LI et al.** *Nat. Biotech.,* 2006, vol. 24, 210-215
- **GRAHAM et al.** *J. Gen Virol.,* 1977, vol. 36, 59
- **MATHER.** *Biol. Reprod.,* 1980, vol. 23, 243-251
- **MATHER et al.** *Annals N.Y. Acad. Sci.,* 1982, vol. 383, 44-68
- **URLAUB et al.** *Proc. Natl. Acad. Sci. USA,* 1980, vol. 77, 4216
- **YAZAKI ; WU.** Methods in Molecular Biology. Humana Press, 2003, vol. 248, 255-268
- **CHEN et al.** *J. Mol. Biol.,* 1999, vol. 293, 865-881
- **PRESTA et al.** *Cancer Res.,* 1997, vol. 57, 4593-4599
- **W.P.R. VERDURMEN et al.** *Journal of Controlled Release,* 2015, vol. 200, 13-22
- Remington's Pharmaceutical Sciences. 1980
- *Cell,* 14 December 2017, vol. 171 (7), 1692-1706
- *Protein Science,* 1995, vol. 4, 2411-2423
- *Protein Science,* 1999, vol. 8, 921-929
- *Biochemical and Biophysical Research Communications,* 2009, vol. 379, 314-318
- *Nat. Commun.,* 10 May 2017, vol. 8, 15090
- *Immunol. Lett.,* August 2006, vol. 106 (2), 144-53
- *Pharm. Res.,* August 2008, vol. 25 (8), 1881-90
- *Journal of Pharmaceutical Science,* 2010, vol. 4, 1707-1720